# EUROPEAN PATENT APPLICATION

(11) **EP 3 257 525 A2**
(43) Date of publication of application: **20.12.2017**
(21) Application number: 17170910.8
(22) Date of filing: 08.12.2010
(51) Int. Cl.: A61K 39/00, C07K 19/00, C07K 14/705

(54) **VACCINE COMPOSITIONS**

(30) Priority: 22.12.2009 US 289083 P
(62) Divisional of application: 10807742.1
(71) Applicant: Celldex Therapeutics, Inc., Needham, MA 02494-2725 (US)
(72) Inventor: AHMED, Syed Saleem, Chesterfield, MO 63017 (US); BUCKLEY, John Joseph III, Chesterfield, MO 63017 (US); LEWIS, Lavinia Marina, Chesterfield, MO 63017 (US); OSBORNE, Brandi Rae, Chesterfield, MO 63017 (US); SINHA, Sandipan, Chesterfield, MO 63017 (US); THORN, Jennifer Marie, Chesterfield, MO 63017 (US); ZAMAN, Ferhana, Chesterfield, MO 63017 (US)
(74) Representative: J A Kemp

(57) **Abstract**

The present disclosure relates to vaccine compositions, particularly KLH-peptide conjugates, and methods of making such compositions. The present disclosure further relates to methods of reducing abnormal cell growth using the composistions described herein.

SEQUENCE LISTING

<110>  CELLDEX THERAPEUTICS INC

<120>  Vaccine Compositions

<130>  N117144B-EP

<150>  61/289083
<151>  2009-12-22

<160>  3

<170>  PatentIn version 3.5

<210>  1
<211>  13
<212>  PRT
<213>  Human

<400>  1

Leu Glu Glu Lys Lys Gly Asn Tyr Val Val Thr Asp His
1               5                   10


<210>  2
<211>  14
<212>  PRT
<213>  Human

<400>  2

Leu Glu Glu Lys Lys Gly Asn Tyr Val Val Thr Asp His Cys
1               5                   10


<210>  3
<211>  14
<212>  PRT
<213>  Human

<400>  3

Cys Leu Glu Glu Lys Lys Gly Asn Tyr Val Val Thr Asp His
1               5                   10

## Description

This application claims priority to U.S. Provisional Application No. 61/289,083 filed on December 22, 2009, which is incorporated herein by reference in its entirety.

### Field

The present disclosure relates to vaccine compositions, particularly KLH-peptide conjugates, and methods of making such compositions. The present disclosure further relates to methods of reducing abnormal cell growth using the composistions described herein.

### Background

CDX-110 is being developed for the treatment of patients suffering from glioblastoma multiforme, a type of brain cancer. A significant portion of patients with this disease express a variant of the epidermal growth factor receptor (EGFR) on the surface of their cancer cells. This variant, known as EGFRvIII, is formed from a splice mutation leading to a unique peptide sequence (typically of 13 amino acids) on the surface of the cancer cells. Since this variant is not expressed on normal cells, the EGFRvIII sequence is a good target for anti-cancer therapies.

EGFR is a protein that has been well validated as a target for cancer therapy. Unlike EGFR, however, EGFRvIII is not present in normal tissues, suggesting this target will enable the development of a tumor-specific therapy for cancer patients. Furthermore, EGFRvIII is a transforming oncogene that can directly contribute to the cancer cell growth. While originally discovered in glioblastoma multiforme (GBM), the most common and aggressive form of brain cancer, the expression of EGFRvIII has also been observed in various other cancers such as breast, ovarian, metastatic prostate, colorectal, and head & neck cancers.

CDX-110 is a conjugate vaccine formed by covalently linking the EGFRvIII peptide, to which a C-terminal cysteine has been added, to the carrier protein Keyhole Limpet Hemocyanin (KLH) (see, e.g. U.S. Patent Nos. 5,401,828, 6,224,868; and WO 2007/056061). KLH also acts as an immune stimulant to enhance immune responses against the EGFRvIII peptide. Chemical linkage of the EGFRvIII peptide and KLH is achieved through use of the bi-functional linker, Sulfosuccinimidyl 4-[N-maleimidomethyl]cyclohexane-1-carboxylate (Sulfo-SMCC).

Previous processes for production of CDX-110 and formulations thereof had several drawbacks for use in a commercial setting. For example, such drawbacks included lack of complete solubitilty of the linker (SMCC) resulting in a reaction that is difficult to control; unacceptable levels of in-process impurities; process methodology that is not amenable to scale-up; lack of sufficient control of key biophysical characteristics such as the ratio of peptide:KLH carrier, size heterogeneity, particulate and gel formation, and product instability. KLH is a very large carrier protein which has previously proved very difficult to conjugate and formulate to high pharmaceutical standards. Accordingly, there is a need for improved manufacturing methods and formulations that provide compositions of KLH-peptide conjugates that address such drawbacks.

### Summary

Other features and advantages of the instant disclosure will be apparent from the following detailed description and examples which should not be construed as limiting. The contents of all references, figures, Genbank entries, patents and published patent applications cited throughout the disclosure are expressly incorporated herein by reference in their entirety.

In one aspect, the disclosure provides a composition comprising a KLH-peptide conjugate, a potassium phosphate buffer, a disaccharide, and a surfactant. In one embodiment, the potassium phosphate buffer is present in a concentration ranging from 5 mM to 30 mM, for example from 5 mM to 20 mM, from 5 mM to 15 mM, from 7 mM to 13 mM, from 8 mM to 12 mM, from 9 mM to 11 mM, or from 9.5 mM to 10.5 mM. In another embodiment, potassium phosphate buffer is present in a concentration of 10 mM. In a further embodiment, the potassium phosphate buffer is present in a concentration such that after 12 weeks at 40°C, the concentration of the KLH-peptide conjugate in said composition, as measured in mg/mL, has changed by less than 15% when compared to the original concentration.

In a further embodiment, the disaccharide in the composition is trehalose. In one embodiment, trehalose is present in a concentration ranging from 45 mg/mL to 150 mg/mL, from 70 mg/mL to 120 mg/mL, from 80 mg/mL to 100 mg/mL, or from 85 mg/mL to 95 mg/mL. In another embodiment, trehalose is present in a concentration of 90 mg/mL. In another embodiment, trehalose is present in a concentration such that after 12 weeks at 40°C, the concentration of the KLH-peptide conjugate in said composition, as measured in mg/mL, has changed by less than 15% when compared to the original concentration.

In a further embodiment, the surfactant in the composition is polysorbate 80. For example, in one embodiment the polysorbate 80 is present in a concentration ranging from 0.01 mg/mL to 0.3 mg/mL, from 0.05 mg/mL to 0.25 mg/mL, from 0.1 mg/mL to 0.25 mg/mL, or from 0.15 mg/mL to 0.25 mg/mL. In one embodiment, the polysorbate 80 is present in a concentration of 0.2 mg/mL. In a further embodiment, the polysorbate 80 is present in a concentration such that after 12 weeks at 40°C, the concentration of the KLH-peptide conjugate in said composition, as measured in mg/mL, has changed by less than 15% when compared to the original concentration.

In one aspect, the present disclosure provides any of the compositions as described herein, wherein the peptide conjugated to KLH in the KLH-peptide conjugate comprises, consists essentially of, or consists of SEQ ID NO:1. In a further embodiment, the peptide conjugated to KLH in the KLH-peptide conjugate comprises, consists essentially of, or consists of SEQ ID NO:2. In one embodiment, the peptide is conjugated to KLH with a sulfo-SMCC linker.

In one aspect, the present disclosure provides any of the KLH-peptide compositions described herein, wherein the epitope density ranges from 20 to 80. For example, in one embodiment the epitope density ranges from 25 to 75, from 25 to 70, from 25 to 65, from 30 to 60, from 30 to 55, from 35 to 50, from 40 to 50. In a further embodiment, the epitope density is 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60.

In a further aspect, the present disclosure provides any of the compositions described herein, wherein the amount of KLH-peptide conjugate present in dimer form ranges from 45% to 65% by mass of the total mass of the composition, as determined by size exclusion chromatography. For example, in one embodiment the dimer form ranges from 50% to 60%, from 51% to 59%, from 52% to 58%, from 53% to 57%, or from 54% to 56% by mass. In a further embodiment, the amount of KLH-peptide conjugate in dimer form is 50%, 51%, 52%, 53%, 54%, 55%, 56%, 57%, 58%, 59%, or 60% by mass of the total mass of the composition, as determined by size exclusion chromatography. In a further embodiment, the amount of KLH-peptide conjugate in dimer form is greater than 80% by mass of the total mass of the composition, as determined by size exclusion chromatography. For example, in one embodiment, the amount in dimer form is greater than 85%, greater than 90%, greater than 95%, greater than 96%, greater than 97%, greater than 98%, or greater than 99% by mass.

In a further aspect, the present disclosure provides any of the compositions described herein, wherein when subjected to size exclusion chromatography with detection by UV absorption, the peak corresponding to the dimer form of the KLH-peptide conjugate is from 45% to 65% of the total area under the curve. For example, in one embodiment the peak corresponding to the dimer form is from 50% to 60%, from 51% to 59%, from 52% to 58%, from 53% to 57%, or from 54% to 56% of the total area under the curve. In a further embodiment, the peak corresponding to the dimer form is 50%, 51%, 52%, 53%, 54%, 55%, 56%, 57%, 58%, 59%, or 60% of the total area under the curve. In a further embodiment, the peak corresponding to the dimer form is greater than 80% of the total area under the curve. For example, in one embodiment, the peak corresponding to the dimer form is greater than 85%, greater than 90%, greater than 95%, greater than 96%, greater than 97%, greater than 98%, or greater than 99% of the total area under the curve.

In a further aspect, the present disclosure provides any of the compositions described herein, wherein the amount of KLH-peptide conjugate present in monomer form ranges from 15% to 40% by mass of the total mass of the composition, as determined by size exclusion chromatography. For example, in one embodiment the dimer form ranges from 20% to 35%, from 21% to 34%, from 22% to 33%, from 23% to 32%, from 24% to 31% or from 25% to 30% by mass. In a further embodiment, the amount of KLH-peptide conjugate in monomer form is 18%, 19%, 20%, 21%, 22%, 23%, 24%, 25%, 26%, 27%, 28%, 29%, or 30% by mass of the total mass of the composition, as determined by size exclusion chromatography. In a further embodiment, the amount of KLH-peptide conjugate in monomer form is greater than 80% by mass of the total mass of the composition, as determined by size exclusion chromatography. For example, in one embodiment, the amount in monomer form is greater than 85%, greater than 90%, greater than 95%, greater than 96%, greater than 97%, greater than 98%, or greater than 99% by mass.

In a further aspect, the present disclosure provides any of the compositions described herein, wherein when subjected to size exclusion chromatography with detection by UV absorption, the peak corresponding to the monomer form of the KLH-peptide conjugate is from 15% to 40% of the total area under the curve. For example, in one embodiment the peak corresponding to the monomer form is from 20% to 35%, from 21% to 34%, from 22% to 33%, from 23% to 32%, from 24% to 31%, or from 25% to 30% of the total area under the curve. In a further embodiment, the peak corresponding to the monomer form is 18%, 19%, 20%, 21%, 22%, 23%, 24%, 25%, 26%, 27%, 28%, 29%, or 30% of the total area under the curve. In a further embodiment, the peak corresponding to the monomer form is greater than 80% of the total area under the curve. For example, in one embodiment, the peak corresponding to the monomer form is greater than 85%, greater than 90%, greater than 95%, greater than 96%, greater than 97%, greater than 98%, or greater than 99% of the total area under the curve.

The present disclosure further provides any of the KLH-peptide conjugate compositions described herein, wherein the composition is an aqueous pharmaceutical composition. In one embodiment, the pH of said composition ranges from 6 to 8. In a further embodiment, the pH of the composition ranges from 6.5 to 7.5. In a further embodiment, the pH of the composition is 6.5, 6.6, 6.7, 6.8, 6.9, 7.0, 7.1, 7.2, 7.3, 7.4 or 7.5. In a further embodiment, the pH is an amount such that after 12 weeks at 40°C, the concentration of the KLH-peptide conjugate in said composition, as measured in mg/mL, has changed by less than 15% when compared to the original concentration.

In a further aspect, the present disclosure provides a liquid composition comprising a KLH-peptide conjugate, potassium phosphate buffer, trehalose, and polysorbate 80, wherein: the peptide conjugated to KLH comprises, consists essentially of, or consists of SEQ ID NO:1 or SEQ ID NO:2; the KLH-peptide conjugate has an epitope density ranging from 30 to 65; the buffer is present in a concentration ranging from 9 mM to 11 mM; the pH of the composition ranges from 7.3 to 7.5; the trehalose is present in a concentration ranging from 85 mg/mL to 95 mg/mL; the polysorbate 80 is present in a concentration ranging from 0.1 mg/mL to 0.3 mg/mL; and further wherein the amount of KLH-peptide conjugate present in monomer form ranges from 18% to 35% by mass of the total mass of the composition, and the amount of KLH-peptide conjugate present in dimer form ranges from 50% to 65% by mass of the total mass of the composition, as determined by size exclusion chromatography. For example, in one embodiment the buffer is present in a concentration of 10 mM, the pH of the composition is 7.4, the trehalose is present in a concentration of 90 mg/mL, and the polysorbate 80 is present in a concentration of 0.2 mg/mL. Further, for example, said liquid composition was prepared by reconstituting a lyophilized composition with water.

The present disclosure further provides a lyophilized composition comprising, consisting essentially of, or consisting of a KLH-peptide conjugate, a potassium phosphate buffer, a disaccharide, and a surfactant. In one embodiment, said lyophilized composition, when reconstituted with water, has a pH ranging from 6 to 9. In one embodiment, the disaccharide is trehalose and the surfactant is polysorbate 80. In a further embodiment, the trehalose is present in an amount ranging from 80 to 110 mg per mg of KLH-peptide conjugate; and the polysorbate 80 is present in an amount ranging from 0.01 to 0.3 mg per mg of KLH-peptide conjugate. In a further embodiment, the trehalose is present in an amount of 90 mg per mg of KLH-peptide conjugate; and the polysorbate 80 is present in an amount of 0.2 mg per mg of KLH-peptide conjugate.

In one aspect, the disclosure provides a liquid composition comprising a KLH-EGFRvIII conjguate, wherein after 12 weeks at 40°C, the concentration of said conjugate in said liquid composition, as measured in mg/mL, has changed by less than 15%, less than 13%, less than 11 %, less than 10%, less than 8%, or less than 5% when compared to the original concentration.

In a further aspect, the disclosure provides methods of reducing abnormal cell growth in a mammal in need thereof, comprising the step of administering to said mammal any of the compositions disclosed herein. In one embodiment, the abnormal cell growth is cancerous. In a further embodiment, the cancer is glioblastoma.

In a further aspect, the disclosure provides methods of reducing abnormal cell growth in a mammal in need thereof, comprising the steps of a) mixing any of the compositions disclosed herein with an adjuvant; and b) administering to said mammal the resulting mixture. In a further aspect, the disclosure provides methods of reducing abnormal cell growth in a mammal in need thereof, comprising administering any of the compositions disclosed herein in combination with at least one adjuvant. In one embodiment, the adjuvant is granulocyte machrophage colony-stimulating factor.

The present disclosure further provides methods of reducing abnormal cell growth in a mammal in need thereof, comprising: a) providing any of the lyophilized compositions disclosed herein; b) adding water to the lyophilized composition to provide a reconstituted composition; and c) administering the reconstituted composition to said mammal.

The present disclosure further provides methods of reducing abnormal cell growth in a mammal in need thereof, comprising: a) providing any of the lyophilized compositions disclosed herein; b) adding water to the lyophilized composition to provide a reconstituted composition; c) mixing the reconstituted composition with an adjuvant composition; and d) administering the resulting mixture to said mammal. In onen embodiment, said adjuvant is granulocyte machrophage colony-stimulating factor.

The present disclosure further provides the use of any of the compositions disclosed herein in the manufacture of a medicament for the treatment of abnormal cell growth in a mammal.

The present disclosure further provides processes for preparing a lyophilized composition comprising a KLH-peptide conjugate, comprising subjecting any of the liquid compositions disclosed herein to a temperature of -20°C or less, -30°C or less, - 40°C or less, -50°C or less, or -60°C or less, and to vaccum conditions.

The present disclosure further provides processes of preparing liquid compositions comprising adding water to any of the lyophilized compositions disclosed herein.

The present disclosure further provides methods for preparing a KLH-EGFRvIII conjugate comprising: a) combining KLH with a linker and allowing the KLH and linker to interact for a time ranging from 30 to 60 minutes, 35 to 55 minutes, 40 to 50 minutes, or 45 minutes; and b) adding a peptide comprising, consisting essentially of, or consisting of SEQ ID NO: 1 or SEQ ID NO:2 to the activated KLH product resulting from step a) to provide the KLH-EGFRvIII conjugate. In one embodiment step b) is carried out in a tangential flow filtration system. In a further embodiment, the retentate flow rate ranges from 10 to 1000 mL/min, for example 50 to 500 mL/min, for example 100 to 500 mL/min, and further for example, 50 mL/min, 100 mL/min, 150 mL/min, 200 mL/min, 250 mL/min, 300 mL/min, 350 mL/min, 400 mL/min, 450 mL/min, or 500 mL/min.

In one embodiment, the linker is combined with KLH in a linker:KLH molar ratio that ranges from 75:1 to 325:1. For example, in one embodiment, said linker:KLH molar ratio ranges from 100:1 to 300:1, from 150:1 to 250:1, or 175:1 to 225:1. For example, in one embodiment, said linker:KLH molar ratio is 175:1, 180:1, 185:1, 190:1, 195:1, 200:1, 205:1, 210:1, 215:1, 220:1, or 225:1.

The present disclosure further provides a liquid composition comprising a KLH-EGFRvIII conjugate, wherein after 26 weeks at 5°C, the number of particulates greater than or equal to 10 µm, as measured by USP <788>, increased by less than 1000%, less than 750%, less than 500%, less than 400%, less than 300%, less than 200%, or less than 100%.

The present disclosure further provides a liquid composition comprising a KLH-EGFRvIII conjugate, wherein after 26 weeks at 5°C, the number of particulates greater than or equal to 25 µm, as measured by USP <788>, increased by less than 1000%, less than 750%, less than 500%, less than 400%, less than 300%, less than 200%, or less than 100%.

### Brief Description of the Drawings

Figure 1 shows a general process schematic of one example of carrying out a KLH-peptide conjugation reaction.
Figure 2A shows a summary of key operating parameters used during the activation reaction of various cross-flow experiments.
Figure 2B shows a summary of key operating parameters used during the initial diafiltration step in various cross-flow experiments.
Figure 2C shows a summary of key operating parameters used during the conjugation step in various cross-flow experiments.
Figures 3A, 3B, 3C, and 3D show a summary of analytical results for cross-flow filtration development experiments.
Figures 4A, 4B, and 4C show a summary of reaction parameters that were used in various experiments.
Figure 5 shows a representative SEC HPLC chromatogram for the CDX-110 KLH-EGFRvIII conjugate showing the approximate retention times of the five peaks.
Figure 6 shows a plot indicating the effect of sulfo-SMCC ratio on epitope density.
Figure 7 shows a plot indicating the effect of activation time on epitope density.
Figures 8A (dimer) and 8B (monomer) show plots indicating the effect of sulfo-SMCC ratio on SEC heterogeneity.
Figures 9A (dimer) and 9B (monomer) show plots indicating the effect of activation time on SEC heterogeneity.
Figure 10 shows a comparison of epitope density with linker slurry and linker dissolved in DMSO.
Figures 11A (dimer) and 11B (monomer) show plots indicating the effect of DMSO on SEC profile of the activation reaction.
Figures 12A and 12B show the statistical analysis of epitope density results, indicating the interaction of sulfo-SMCC:KLH ratio and protein concentration.
Figures 13A and 13B show the statistical analysis of dimer results, indicating the interaction of sulfo-SMCC:KLH ratio and protein concentration.
Figure 14 shows a summary of the reaction parameters used for the original and optimized process of preparing CDX-110.

### Detailed Description

The present disclosure is based on the discovery of novel formulations for KLH-peptide conjugates that provide beneficial properties, such as decreased particulate and gel formation and increased stability of the conjugate. The formulations described herein can also be lyophilized and reconstituted, providing additional benefits for shipping and long-term storage. Such beneficial properties, and others that will be apparent to those of skill in the art, are described more fully herein. Further, in accordance with the present disclosure, improved processes have been developed for the production of KLH-EGFRvIII peptide conjugate vacccines. The new processess disclosed herein provide optimized reaction conditions that are amenable to large-scale manufacturing protocols, and that result in improvements to the purity profile and control of key biophysical attributes, such as the ratio of peptides/KLH, and size heterogeneity.

In order that the present disclosure may be more readily understood, certain terms are first defined. Additional definitions are set forth throughout the detailed description.

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well known and commonly used in the art.

The methods and techniques of the present disclosure are generally performed according to methods well known in the art and as described in various general and more specific references that are cited and discussed throughout the present specification unless otherwise indicated. Such references include, e.g., Sambrook and Russell, Molecular Cloning, A Laboratory Approach, Cold Spring Harbor Press, Cold Spring Harbor, NY (2001), Ausubel et al., Current Protocols in Molecular Biology, John Wiley & Sons, NY (2002), and Harlow and Lane Antibodies: A Laboratory Manual, Cold Spring Harbor Laboratory Press, Cold Spring Harbor, NY (1990). Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

As used herein, each of the following terms has the meaning associated with it in this section.

The articles "a" and "an" are used herein to refer to one or to more than one (*i.e*., to at least one) of the grammatical object of the article. By way of example, "an element" means one element or more than one element.

As used herein, the twenty conventional amino acids and their abbreviations follow conventional usage. See Immunology--A Synthesis (2nd Edition, E. S. Golub and D. R. Gren, Eds., Sinauer Associates, Sunderland, Mass. (1991)).

As used herein, "KLH" refers to the protein Keyhole Limpet Hemocyanin, a multi-subunit, oxygen-carrying, metalloprotein that is found in the hemolymph of keyhole limpets (*Megathura crenulata*)*,* or a fragment or subunit thereof.

As used herein, the term "KLH-peptide conjugate" refers to a KLH protein, or a fragment or subunit thereof, that is covalentlty bonded to a polypeptide that is unrelated to KLH. Such covalent bonding is typically accomplished through use of an appropriate chemical linker.

As used herein, the term "epitope density", with reference to a KLH-peptide conjugate, refers to the average number of peptides that are coupled to each KLH subunit. Epitope density can be determined using amino acid composition analysis as described herein.

The term "disaccharide", as used herein, refers to a compound which, upon hydrolysis, yields two monosaccharide molecules (e.g. glucose, fructose, mannose, etc.). Suitable disaccharides include, but are not limited to, sucrose, lactose and trehalose.

As used herein, the term "surfactant" refers to an excipient that can alter the surface tension of a liquid KLH-peptide conjugate composition. As discussed further herein, examples of surfactants include, but are not limited to, polysorbate surfactants, poloxamers (e.g., poloxamer 18 and 407), triton surfactants, such as Triton X-100^{®}, and polysorbate surfactants such as Tween 20^{®} and Tween 80^{®}, among many others that are commonly known to those of skill in the art.

The terms "lyophilization", "lyophilized", and "freeze-dried" refer to a process by which the material to be dried is first frozen and then the ice or frozen solvent is removed by sublimation in a vacuum environment.

As used herein, the term "EGFRvIII" refers to a peptide that is representative of the variant mutant III that is present in epidermal growth factor receptor (EGFR), and typically comprises or consists of the amino acid sequence set forth as SEQ ID NO:1 or 2 or at least comprises the amino acid sequence "Lys-Lys-Gly-Asn-Tyr".

"Abnormal cell growth", as used herein, unless otherwise indicated, refers to cell growth that is independent of normal regulatory mechanisms (e.g., loss of contact inhibition), including the abnormal growth of normal cells and the growth of abnormal cells. This includes, but is not limited to, the abnormal growth of: tumor cells (tumors), for example, mesothelioma, hepatobilliary (hepatic and billiary duct), a primary or secondary CNS tumor, a primary or secondary brain tumor, lung cancer (NSCLC and SCLC), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, non-Hodgkin's lymphoma, spinal axis tumors, brain stem glioma, pituitary adenoma, adrenocortical cancer, gall bladder cancer, multiple myeloma, cholangiocarcinoma, fibrosarcoma, neuroblastoma, retinoblastoma, or a combination of one or more of the foregoing cancers.

As used herein, the term "treating" or "treatment" refers to both therapeutic treatment and prophylactic or preventative measures, wherein the object is to prevent or slow down (lessen) the targeted pathologic condition or conditions. Those in need of treatment include those already with the condition as well as those prone to have the condition or those in whom the condition is to be prevented.

A "therapeutically effective amount" of a composition refers to an amount that is effective, at dosages and for periods of time necessary, to achieve the desired therapeutic result, which includes treatment or prophylactic prevention of abnormal cell growth. It is to be noted that dosage values may vary with the severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition. Likewise, a therapeutically effective amount of the composition may vary according to factors such as the disease state, age, sex, and weight of the individual, the ability of the therapeutic composition to elicit a desired response in the individual, and the desired route of administration. A therapeutically effective amount is also one in which any toxic or detrimental effects of the composition are outweighed by the therapeutically beneficial effects.

The term "pharmaceutical composition" refers to preparations which are in such form as to permit the biological activity of the active ingredients to be effective.

"Pharmaceutically acceptable excipients" (vehicles, additives) are those, which can reasonably (i.e., safely) be administered to a subject to provide an effective dose of the active ingredient employed. The term "excipient" or "carrier" as used herein refers to an inert substance, which is commonly used as a diluent, vehicle, preservative, binder or stabilizing agent for drugs. As used herein, the term "diluent" refers to a pharmaceutically acceptable (safe and non-toxic for administration to a human) solvent and is useful for the preparation of the liquid formulations herein. Exemplary diluents include, but are not limited to, sterile water and bacteriostatic water for injection (BWFI).

Unless stated otherwise, the concentrations and pH values of the various components of the compositions disclosed herein are those concentrations at ambient conditions, (i.e., at 25°C and atmospheric pressure). When concentration and pH ranges are listed herein, such ranges that are intermediate to the explicit ranges recited are also intended to be part of the present disclosure. For example, ranges of values using a combination of any of the values as upper and/or lower limits are intended to be included.

In accordance with the present disclosure, it has been discovered that for a KLH-peptide conjugate, the formation of particulates and/or gel can be reduced and the stability of the conjugate can be improved by combining the conjugate with a potassium phosphate buffer, a disaccharide such as trehalose, and a surfactant such as Tween 80. Futhermore, it has been discovered that when these components are used in certain amounts, lyophilization (followed by reconstitution as an aqueous solution) is possible while at the same time minimizing particle and gel formation and maintaining the stability and biophysical characteristics of the conjugate.

While not wishing to be bound by any particular theory, it is believed that compositions of the present disclosure help to improve the stability and reduce particulate and/or gel formation of KLH-peptide conjugates by reducing the incidence of one or more of the following: KLH-peptide aggregation, fragmentation, oxidation, freeze/thaw instability, instability associated with shipping or transporting from one location to another, discoloration, and/or deamidation. Thus, the present disclosure provides KLH-peptide conjugate compositions having improved chemical and/or physical stability as compared to previously disclosed compositions.

Therefore, in certain aspects, the present disclosure provides a composition comprising a KLH-peptide conjugate, a potassium phosphate buffer, a disaccharide, and a surfactant. In still other aspects, the aforementioned compositions can include additional pharmaceutically acceptable excipients, including, but not limited to, buffers, salts, antioxidants, tonicity agents, surfactants, and mixtures thereof. Thus, the present disclosure provides novel formulations for KLH-peptide conjugates.

### KLH-peptide conjugates

KLH is a complex multimeric protein that can be used as a carrier for antigenic peptides. Such peptides can be conjugated to KLH, and the resulting KLH-peptide conjugate can be used as a vaccine in stimulating an immune response directed to the antigenic peptide.

In one aspect of the disclosure, the antigenic peptide is conjugated to KLH by way of chemical cross-linking, typically using a heterobifunctional cross-linker. Several hetero-bifunctional cross-linkers are known in the art. In some embodiments, the hetero-bifunctional crosslinker contains a functional group which can react with first attachment sites, i.e. with the side-chain amino group of lysine residues of KLH, and a further functional group which can react with a preferred second attachment site, i.e. a cysteine residue fused to the antigenic peptide and optionally also made available for reaction by reduction. The first step of the procedure, typically called the derivatization, is the reaction of the KLH with the cross-linker. The product of this reaction is an activated KLH, also called an activated carrier. In the second step, unreacted cross-linker is removed using standard methods such as gel filtration or dialysis. In the third step, the antigenic peptide is reacted with the activated KLH, and this step is typically called the coupling step. Unreacted antigenic peptide may be optionally removed in a fourth step, for example by dialysis. Several hetero-bifunctional crosslinkers are known in the art. These include cross-linkers such as SMPH, Sulfo-MBS, Sulfo-EMCS, Sulfo-GMBS, Sulfo-SIAB, Sulfo-SMPB, Sulfo-SMCC, SVSB, SIA and other cross-linkers available, for example, from the Pierce Chemical Company (Rockford, IL, USA), and having one functional group reactive towards amino groups and one functional group reactive toward cysteine residues. The above mentioned cross-linkers all lead to formation of a thioether linkage.

One example of a KLH-peptide conjugate comprises a peptide known as EGFRvIII (SEQ ID NO:1) that is chemically conjugated to the surface of the KLH protein. This EGFRvIII peptide contains thirteen amino acids of a type III variant form of epidermal growth factor receptor (EGFRvIII) (see, e.g. U.S. Patent No. 5,401,828). The EGFRvIII peptide can be coupled to primary amine groups (e.g. on Lys side chains) on the surface of KLH by including a Cys residue on the N- or C-terminus of the peptide (see, e.g. SEQ ID NO:2 or 3), and then using sulfosuccinimidyl-4-(N-maleimidomethyl)cyclohexane-1-carboxylate (sulfo-SMCC) as a linker. Thus, a typical coupling reaction can be carried out as shown below in Scheme 1.

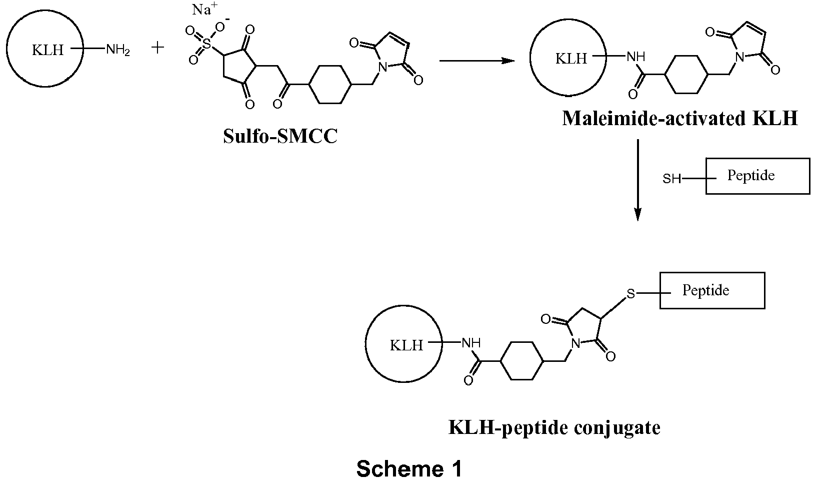

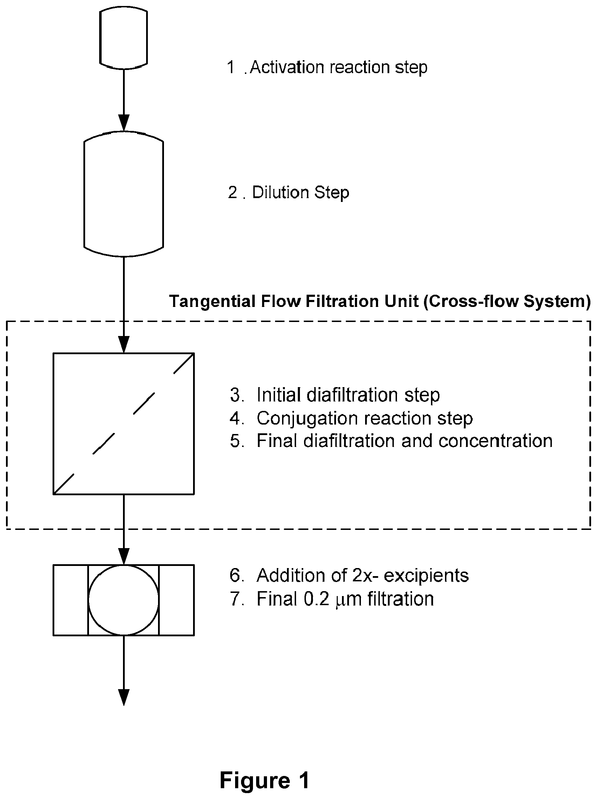

Those of skill in the art will recognize appropriate reaction conditions to achieve conjugation according to Scheme 1. For example, the first step in Scheme 1 above (activation reaction) is where KLH, buffer, water, and linker are mixed together. This activation reaction can then be allowed to proceed for approximately 45 ± 5 minutes. At the end of the reaction time, the activation reaction mix can be diluted and transferred into an appropriate filtration system in order to remove excess linker and linker-related impurities. Peptide pre-dissolved in an appropriate buffer is then introduced and the conjugation reaction is allowed to proceed for 2 to 3 hours. At the end of the conjugation process time, a second filtration operation can be performed in order to remove excess peptide and peptide-related impurities and to buffer exchange into an appropriate final buffer. Further processing may include concentration adjustment by dilution, addition of excipients, final filtering, and packaging. Figure 1 provides a general schematic of one example of how this process can be carried out.

The number of peptides per KLH subunit (also referred to herein as the "epitope density"), will depend on the specific reaction conditions, but typically ranges from about 30 to about 100 peptides per KLH molecule. This epitope density can be measured by comparison of total amino acid composition between conjugated and unconjugated KLH as described herein. The size distribution profile for KLH-peptide conjugates, such as KLH-EGFRvIII conjugates, can include monomers, as well as multi-meric forms such as dimers, trimers, etc. Depending on the reaction conditions, this size profile can vary and can be measured by analytical techniques such as size exclusion chromatography (SEC).

Where the peptide used in the above conjugation scheme is the EGFRvIII peptide (as set forth in SEQ ID NO:2 or 3), the resulting KLH conjugate is referred to as a KLH-EGFRvIII conjugate. An optimized process for producing a KLH-EGFRvIII conjugate was developed in order to address shortcomings of the original process while at the same time maintaining comparibility to drug substance lots which had already been in the clinic. The key analytical attributes that were used for process development were purity by anion-exchange (AEX) and reverse-phase HPLC (RP-HPLC), molecular size distribution as measured by SEC HPLC, and epitope density. Criteria for an optimized process include maintaining or improving drug substance purity and producing a size distribution profile and epitope density within desired ranges.

### Antigenic peptides

Polypeptides that are used for conjugation to KLH can be derived from natural sources and isolated from a mammal, such as, for example, a human, a primate, a cat, a dog, a horse, a mouse, or a rat. Polypeptides of the disclosure can thus be isolated from cells or tissue sources using standard protein purification techniques.

Alternatively, polypeptides can be synthesized chemically or produced using recombinant DNA techniques. For example, a polypeptide of the disclosure (e.g. those shown as SEQ ID NOs: 1 to 3) can be synthesized by solid phase procedures well known in the art. Suitable syntheses may be performed by utilising "T-boc" or "F-moc" procedures. Cyclic peptides can be synthesised by solid phase methods employing the well-known "F-moc" procedure and polyamide resin in a fully automated apparatus. Alternatively, those skilled in the art will know the necessary laboratory procedures to perform the process manually. Techniques and procedures for solid phase synthesis are described in Solid Phase Peptide Synthesis: A Practical Approach by E. Atherton and R. C. Sheppard, published by IRL at Oxford University Press (1989) and Methods in Molecular Biology, Vol. 35: Peptide Synthesis Protocols (ed. M. W. Pennington and B. M. Dunn), chapter 7, pp. 91-171 by D. Andreau et al.

Alternatively, a polynucleotide encoding a polypeptide of the disclosure can be introduced into an expression vector that can be expressed in a suitable expression system using techniques well known in the art, followed by isolation or purification of the expressed polypeptide of interest. A variety of bacterial, yeast, plant, mammalian, and insect expression systems are available in the art and any such expression system can be used. Optionally, a polynucleotide encoding a polypeptide of the disclosure can be translated in a cell-free translation system.

### Keyhole Limpet Hemocyanin

Those skilled in the art recognize KLH as a beneficial carrier of antigenic peptides, due to its ability to increase the immune response because of its large size and distinct epitopes (see, e.g. Harris et al., Micron 30(6):597-623 (1999)). KLH is typically obtained directly by purification from keyhole limpets, and is commercially available from several sources (e.g. Thermo Scientific, Biosyn).

### Preparation of KLH-Peptide Conjugate Compositions

The KLH-peptide conjugates of the present disclosure are typically formulated as pharmaceutical compositions for parenteral administration to a subject. In one embodiment, the composition is a liquid composition. In another embodiment, the composition is a lyophilized composition. The compositions of the present disclosure comprise a KLH-peptide conjugate, such as KLH-EGFRvIII conjugates, combined with a potassium phosphate buffer, a dissacharide, and a surfactant. The amount of KLH-peptide conjugate, such as a KLH-EGFRvIII conjugate, can be present in the compositions of the present disclosure in an amount of from 0.01 to 20.0 mg/mL, for example 0.1 to 10 mg/mL, or 0.5 to 5 mg/mL. For example, in particular embodiments, the KLH-peptide conjugate, such as a KLH-EGFRvIII conjugate, is present in a liquid formulation in an amount of from 0.5 to 1.5 mg/mL, such as 1.0 mg/mL.

Such compositions can be aqueous liquid compositions with a pH range from 7 to 8. In a particular embodiment, the pH is from 7.3 to 7.5, such as 7.4. Those of skill in the art will appreciate that there will be some inter-apparatus variability in the measurment of the pH of any particular solution, typically by as much as 0.1 pH units. Accordingly, where pH numbers are recited throughout the present disclosure, one of skill in the art will recognize that such numbers are intended to encompass such inter-apparatus variability.

The compositions of the present disclosure also include lyophilized compositions that have subsequently been reconsituted by the addition of solvent, such as water.

The compositions of the present disclosure can include a potassium phosphate buffer, which is typically used to adjust the pH to a desired level. However, the present disclosure is not intended to be limited by any specific mechanism by which the potassium phosphate buffer acts in the compositions disclosed herein, and the potassium phosphate buffers used in the compositions dislcosed herein may achieve their properties through mechanisms that are altogether unrelated to their ability to adjust the pH to a desired level. As used herein, the term "buffer" refers to an added composition that allows a liquid composition to resist changes in pH. In certain embodiments, the added buffer allows a liquid KLH-peptide conjugate composition to resist changes in pH by the action of its acid-base conjugate components. When a concentration of a buffer is referred to, it is intended that the recited concentration represent the molar concentration of the free acid or free base form of the buffer.

Although those of skill in the art will appreciate that phosphate buffers are typically not used in parenteral formulations - especially in lyophilized products since phosphate salts have a tendency to crystallize during freezing - the present disclosure surprisingly shows that potassium phosphate buffer is useful in KLH-peptide conjugate formulations without any such crystallization. In particular, potassium phoshate buffer can be used in a concentration that ranges from 5 mM to 15 mM, for example from 7 mM to 12 mM, from 8 to 11 mM, or 9.5 mM to 10.5 mM. Those of skill in the art will recognize that potassium phosphate buffer is typically prepared as a mixture of monobasic dihydrogen phosphate and dibasic monohydrogen phoshate (e.g. KH₂PO₄, K₂HPO₄) in sufficient respective concentrations to arrive at the desired pH. Such buffers are commercially available, or those skilled in the art will recognize how to prepare and use such buffers within the context of the present disclosure.

The compositions of the present disclosure can also comprise a disaccharide, such as trehalose, for use as a cryoprotectant during freezing and as a lyoprotectant during freeze-drying. However, the present disclosure is not intended to be limited by any specific mechanism by which the disaccharide acts in the compositions disclosed herein, and the disaccharides used in the compositions dislcosed herein may achieve their properties through mechanisms that are altogether unrelated to their ability to act as a cryoprotectant during freezing and as a lyoprotectant during freeze-drying. In the present disclosure, such a disaccharide can be used in an amount of from 70 to 110 mg/mL. Those skilled in the art will recognize that disaccharides, such as trehalose, dextran, and sucrose (among others) are readily available through commercial sources.

The composition of the present disclosure can also comprise a surfactant, such as polysorbate 80, to help decrease particle formation by decreasing the interaction of KLH-peptide conjugates with air-water or ice-water interfaces. However, the present disclosure is not intended to be limited by any specific mechanism by which the surfactant acts in the compositions disclosed herein, and the surfactants used in the compositions dislcosed herein may achieve their properties through mechanisms that are altogether unrelated to their ability to decrease the interaction of KLH-peptide conjugates with air-water or ice-water interfaces. As used herein, the term "surfactant" refers to an excipient that can alter the surface tension of a liquid KLH-peptide conjugate composition. In certain embodiments, the surfactant reduces the surface tension of a liquid KLH-peptide conjugate composition. In still other embodiments, the "surfactant" may contribute to an improvement in stability of a KLH-peptide conjugate, such as a KLH-EGFRvIII conjugate. For example, the surfactant may reduce aggregation of the KLH-peptide conjugate and/or minimize the formation of particulates in the composition and/or reduce adsorption. The surfactant may also improve stability of the KLH-peptide conjugate during and after a freeze/thaw cycle.

Suitable surfactants include polysorbate surfactants, poloxamers (e.g., poloxamer 18 and 407), triton surfactants, such as Triton X-100^{®}, polysorbate surfactants such as Tween 20^{®} and Tween 80^{®}, sodium dodecyl sulfate, sodium laurel sulfate, sodium octyl glycoside, lauryl- sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl- betaine, lauroamidopropyl-betaine, cocamidopropyl-betaine, linoleamidopropyl-betaine, myristamidopropyl-betaine, palmidopropyl-betaine, isostearamidopropyl-betaine, myristamidopropyl-dimethylamine, palmidopropyl-dimethylamine, isostearamidopropyl- dimethylamine, sodium methyl cocoyl-taurate, disodium methyl oleyl-taurate, dihydroxypropyl peg 5 linoleammonium chloride, polyethylene glycol, polypropylene glycol, and mixtures thereof.

In one embodiment, the surfactant is a polysorbate surfactant comprising at least one excipient that is selected from the group consisting of polysorbate 20, polysorbate 21, polysorbate 40, polysorbate 60, polysorbate 61, polysorbate 65, polysorbate 80, polysorbate 81, polysorbate 85, and mixtures thereof.

The concentration of the surfactant when present in the composition generally ranges from 0.01 mg/mL to 10 mg/mL, from 0.05 mg/mL to 5.0 mg/mL, from 0.1 mg/mL to 1.0 mg/mL, or from 0.2 mg/mL to 0.7 mg/mL. In another embodiment, the surfactant is present in an amount that is 0.2 mg/mL. In another embodiment, the surfactant is present in an amount that is 0.5 mg/mL. In another embodiment the surfactant is polysorbate 80 (e.g. Tween 80^{®}), and is present in an amount from 0.1 to 0.3 mg/mL, for example 0.2 mg/mL. Ranges intermediate to the above-recited surfactant concentrations are also intended to be part of this disclosure. For example, ranges of values using a combination of any of the above- recited values as upper and/or lower limits are intended to be included. Those skilled in the art will recognize that surfactants, such as Tween 80^{®}, are readily available through commercial sources.

Once a KLH-peptide conjugate, such as a KLH-EGFRvIII conjugate, is made, it can be formulated with the components as described above using methods that are well known to those of skill in the art. For example, a KLH-EGFRvIII conjugate can be introduced into a suitable compounding vessel and stirred gently to achieve homongeniety. The conjugate can then be diluted to about 1.0 mg/mL with the formulation buffer (e.g. potassium phosphate). The solution can then be sampled to confirm the appropriate pH and concentration. The solution can then be passed through two sterilizing filters (0.22 micron) in series.

### Routes of Administration and Dosages

The compositions of the present disclosure may be in liquid solutions (e.g., injectable and infusible solutions). The preferred form may depend on the intended mode of administration and therapeutic application. Typical preferred compositions are in the form of injectable or infusible solutions, such as compositions similar to those used for passive immunization of humans. The preferred mode of administration is parenteral (e.g., intravenous, intradermal, intra-arterial, subcutaneous, intraosseous, intraperitoneal, intramuscular, and intrasternally) or by infusion techniques, in the form of sterile injectable liquid or olagenous suspensions. As will be appreciated by the skilled artisan, the route and/or mode of administration will vary depending upon the desired results.

In one embodiment, the KLH-peptide conjugate composition is administered by intravenous infusion or injection. In another embodiment, the composition is administered by intramuscular or subcutaneous injection. Therapeutic compositions typically are sterile and stable under the conditions of manufacture and storage. The composition can be formulated as a solution, microemulsion, dispersion, or liposome. Sterile injectable solutions can be prepared by incorporating the KLH-peptide conjugate composition of the required amount in an appropriate diluent with one or a combination of ingredients enumerated above, as required, followed by sterilization (e.g., filter sterilization). Generally, dispersions are prepared by incorporating the active compound into a sterile vehicle that contains a basic dispersion medium and the required other ingredients from those enumerated above.

The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed, including synthetic mono- or diglycerides. In addition, n-3 polyunsaturated fatty acids may find use in the preparation of injectables. In the case of sterile powders for the preparation of sterile injectable solutions, the preferred methods of preparation are vacuum drying, spray-drying, and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants.

Prolonged absorption of injectable compositions can be brought about by including in the composition an agent that delays absorption, for example, monostearate salts and gelatin or by formulating the composition into prolonged absorption forms such as, depots, liposomes, polymeric microspheres, polymeric gels, and implants.

Other methods for administration of the compositions described herein include dermal patches that release the medications directly into a subject's skin. Such patches can contain the compositions of the present disclosure in an optionally buffered, liquid solution, dissolved and/or dispersed in an adhesive, or dispersed in a polymer. Still other methods for administration of the compositions described herein include liquid ophthalmological drops for the eyes.

The KLH-peptide conjugate composition may be administered once, or multiple times. For example, the composition may be administered from once daily to once every six months or longer. The administering may be on a schedule such as three times daily, twice daily, once daily, once every two days, once every three days, once weekly, once every two weeks, once every month, once every two months, once every three months and once every six months.

The composition may also be administered continuously via a minipump. The composition may be administered at the site of the tumor or inflamed body part, into the tumor or inflamed body part or at a site distant from the site of the tumor or inflamed body part. The composition may be administered once, at least twice or for at least the period of time until the condition is treated, palliated or cured. The KLH-peptide conjugate composition generally may be administered for as long as the tumor is present provided that the composition results in the desired therapeutic effect. The composition typically would be administered as part of a pharmaceutical composition as described herein. The compositions of the disclosure may include a therapeutically effective amount of a KLH-peptide conjugate as disclosed herein. In preparing the formulation, the therapeutically effective amount of the KLH-peptide conjugate present in the formulation can be determined, for example, by taking into account the desired dose volumes and mode(s) of administration, the nature and severity of the condition to be treated, and the age and size of the subject.

Exemplary, non-limiting dose ranges for administration of the compositions of the present disclosure to a subject are from 0.01 mg/kg to 200 mg/kg (expressed in terms of milligrams (mg) of KLH-peptide conjugate administered per kilogram (kg) of subject weight), from 0.1 mg/kg to 100 mg/kg, from 1.0 mg/kg to 50 mg/kg, from 5.0 mg/kg to 20 mg/kg, or 15 mg/kg. For purposes of the present disclosure, an average human subject weighs about 70 kg. Ranges intermediate to any of the dosages cited herein, e.g., 0.01 mg/kg - 199 mg/kg, are also intended to be part of this disclosure. For example, ranges of values using a combination of any of the recited values as upper and/or lower limits are intended to be included.

Dosage regimens can also be adjusted to provide the optimum desired response (e.g., a therapeutic or prophylactic response) by administering several divided doses to a subject over time or the dose can be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the KLH-peptide conjugate and the particular therapeutic or prophylactic effect to be achieved, and (b) the limitations inherent in the art of compounding such KLH-peptide conjugate for the treatment of sensitivity in individuals.

The liquid formulations of the present disclosure can be prepared as unit dosage forms. For example, a unit dosage per vial may contain from 1 to 1000 milliliters (mL) of different concentrations of a KLH-peptide conjugate. In other embodiments, a unit dosage per vial may contain 0.2 mL, 0.4 mL, 0.6 mL, 0.8 mL, 1 mL, 2 mL, 3 mL, 4 mL, 5 mL, 6 mL, 7 mL, 8 mL, 9 mL, 10 mL, 15 mL, 20 mL, 30 mL, 40 mL, 50 mL or 100 mL of different concentrations of a KLH-peptide conjugate. If necessary, these preparations can be adjusted to a desired concentration by adding a sterile diluent to each vial. The liquid formulations of the present disclosure can also be prepared as unit dosage forms in sterile bags, containers, pre-filled syringes, pens, or micro-needles which can be used for direct administration and/or are suitable for connection to an intravenous administration line or catheter.

KLH-peptide conjugates, such as KLH-EGFRvIII conjugates, can be combined with other immunostimulatory agents, such as granulocyte macrophage colony-stimulating factor (GM-CSF), e.g. sargramostim or molgramostim. For example, GM-CSF can be combined directly with a KLH-EGFRvIII conjugate, and then administered to a subject. In one example, 0.35 mL of GM-CSF that is concentrated at 0.5 mg/mL can be combined with 0.6 mL of a KLH-EGFRvIII conjugate composition that is concentrated at 1 mg/mL to provide 0.95 mL of combined solution containing 0.5 mg of a KLH-EGFRvIII conjugate and 0.15 mg of GM-CSF. This combined composition can then be administered directly to a subject. Those skilled in the art will recognize that a variety of amounts and concentrations can be used to provide the apporiate dosage amount to a subject in need thereof.

### Stability Assessment

The present disclosure provides stable liquid and lyophilized compositions comprising a KLH-peptide conjugate as described herein. A stable composition is desirable to maintain or resist changes in, for example, product appearance and integrity (including physical or chemical degradation potentially leading to a reduction in biological activity). Various analytical techniques and indicators for measuring protein stability are reported in the literature and a number of these techniques and indicators are reviewed in Peptide and Protein Drug Delivery, 247-301, Vincent Lee Ed., Marcel Dekker, Inc., New York, N.Y., Pubs. (1991) and Jones, A., Adv. Drug Delivery Rev. 10: 29-90 (1993).

In general, the compositions of the present disclosure exhibit improved stability when subjected to low storage temperatures over a period of time, and/or when subjected to one or more freeze/thaw cycles. The stability of KLH-peptide conjugates can be measured using a variety of analytical techniques that are well known to those of skill in the art. For example, analytical assays such as appearance (as measured by visual inspection and/or by UV light scattering), microscopy, light obscuration, size exclusion chromatography, anion exchange (AEX) chromatography, and reverse-phase high-pressure liquid chromatography. Such assays can provide data on stability for multiple degradation pathways, such as change in content of monomer/dimer ratio, formation of peptide-linker impurities, change in A280/A340 ratio, and AEX retention time of a KLH-peptide conjugate peak due to formation of acidic species. Such analytical assays can be used to monitor the stability of a conjugate as a function of time and temperature.

Protein fragmentation in a liquid pharmaceutical composition can be measured by various methods known in the art. Such methods include, for example, size exclusion chromatography, ultraviolet detection (e.g., at 214 nanometers), SDS-PAGE and/or matrix- assisted laser desorption ionization/time-of-flight mass spectrometry (MALDI/TOF MS). Protein fragmentation resulting in a charge alteration (e.g., occurring as a result of deamidation) can be evaluated, for example, by ion-exchange chromatography or isoelectric focusing (IEF). Composition discoloration generally can be measured by visual observation of the composition itself. The compositions disclosed herein generally reduce composition discoloration (e.g., pink or yellow) and/or maintain composition clarity (e.g., turbidity, cloudiness and/or particulate formation). For purposes of the present disclosure, the term "discoloration" refers to both changes in color (e.g., from clear and colorless to pink or yellow) and to changes in clarity (e.g., from clear and colorless to turbid, cloudy and/or having particulates). Composition discoloration generally can be measured using additional techniques such as by ultraviolet detection at 214 nanometers and/or by visual comparison against a standard color scale of the compositions being compared.

As used herein, the term "a freeze/thaw cycle" refers to techniques for using a liquid sample after frozen storage, wherein the temperature of the sample is lowered to a temperature of 0°C or lower in order to freeze the liquid sample, and then-subjecting the sample to a temperature which will restore its liquid state for a sufficient period of time to permit use of the sample, followed by and return to frozen storage, preferably at a temperature of 0°C or lower. As used herein, the term "frozen storage" refers to freezing and maintaining a previously liquid sample at a temperature of 0°C or below, and preferably -20°C or lower.

### Methods of Treatment

Any of the KLH-peptide compositions described herein may be used therapeutically. In one embodiment, the KLH-peptide conjugate is a KLH-EGFRvIII conjugate, as described herein, and is used to treat a human subject. Alternatively, the subject may be a mammal that expresses the EGFRvIII variant. Thus, the KLH-EGFRvIII conjugate compositions disclosed herein may be administered to a non-human mammal expressing the EGFRvIII variant for veterinary purposes or as an animal model of human disease. Such animal models may be useful for evaluating the therapeutic efficacy of compositions of the present disclosure.

The present disclosure provides methods of reducing abnormal cell growth in a mammal in need thereof, comprising the step of administering to said mammal any of the compositions disclosed herein, particularly those wherein the KLH-peptide conjugate is a KLH-EGFRvIII conjugate. In one embodiment, the abnormal cell growth is cancerous. In a further embodiment, the cancer is glioblastoma. The present disclosure further provides methods of reducing abnormal cell growth in a mammal in need thereof, comprising the steps of a) mixing any of the compositions as decribed herein with an adjuvant; and b) administering to said mammal the resulting mixture. In one embodiment, the KLH-peptide conjugate is a KLH-EGFRvIII conjugate, and the adjuvant is granulocyte macrophage colony-stimulating factor (GM-CSF), e.g. sargramostim or molgramostim.

The present disclosure further provides methods of reducing abnormal cell growth in a mammal in need thereof, comprising: a) providing any of the lyophilized compositions disclosed herein, adding water to the lyophilized composition to provide a reconstituted composition, and administering the reconstituted composition to said mammal. Also provided herein are methods of reducing abnormal cell growth in a mammal in need thereof, comprising providing any of the lyophilized compositions as described herein, adding water to the lyophilized composition to provide a reconstituted composition, mixing the reconstituted composition with an adjuvant composition, and administering the resulting mixture to said mammal. In particular embodiments, the mammal is human, and the KLH-peptide conjugate is a KLH-EGFRvIII conjugate.

Therapeutic methods involve administering to a subject in need of treatment a therapeutically effective amount, or "effective amount", of a composition comprising a KLH-peptide conjugate, such as a KLH-EGFRvIII conjugate, as contemplated by the present disclosure. As used herein, a "therapeutically effective", or "effective", amount refers to an amount of a KLH-peptide conjugate thereof that is of sufficient quantity to result in a decrease in severity of disease symptoms, an increase in frequency and duration of disease symptom-free periods, or a prevention of impairment or disability due to the disease affliction - either as a single dose or according to a multiple dose regimen, alone or in combination with other agents. One of ordinary skill in the art would be able to determine such amounts based on such factors as the subject's size, the severity of the subject's symptoms, and the particular composition or route of administration selected. The subject may be a human or non-human animal (e.g., rabbit, rat, mouse, monkey or other lower-order primate). A composition of the present disclosure might also be co-administered with known medicaments.

The KLH-EGFRvIII conjugate compositions disclosed herein can be used as a therapeutic product in a variety of situations where the EGFRvIII variant is undesirably expressed or found. Given the expression of the EGFRvIII variant in various cancers, such as glioblastoma, disorders and conditions particularly suitable for treatment with a KLH-EGFRvIII conjugate compositions of the present disclosure include abnormal cell growth, for example, mesothelioma, hepatobilliary (hepatic and billiary duct), a primary or secondary CNS tumor, a primary or secondary brain tumor, lung cancer (NSCLC and SCLC), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, non-Hodgkin's lymphoma, spinal axis tumors, brain stem glioma, pituitary adenoma, adrenocortical cancer, gall bladder cancer, multiple myeloma, cholangiocarcinoma, fibrosarcoma, neuroblastoma, retinoblastoma, or a combination of one or more of the foregoing cancers.

The compositions described herein can be administered by any suitable means, which can vary, depending on the type of disorder being treated. Possible administration routes include parenteral (e.g., intravenous, intradermal, intra-arterial, subcutaneous, intraosseous, intraperitoneal, intramuscular, and intrasternally), intrapulmonary and intranasal, and, if desired for local immunosuppressive treatment, or intralesional administration. In addition, compositions of the disclosure might be administered by pulse infusion, with, e.g., declining doses of the KLH-peptide conjugate. Preferably, the dosing is given by injections, most preferably intradermal or intramuscular injections, depending in part on whether the administration is brief or chronic.

The amount to be administered will depend on a variety of factors such as the clinical symptoms, weight of the individual, whether other drugs are administered. The skilled artisan will recognize that the route of administration will vary depending on the disorder or condition to be treated. For example, determining a therapeutically effective amount of a composition comprising a KLH-peptide conjugate (e.g. a KLH-EGFRvIII conjugate) according to the present disclosure will largely depend on particular patient characteristics, route of administration, and the nature of the disorder being treated. General guidance can be found, for example, in the publications of the International Conference on Harmonization and in Remington's Pharmaceutical Sciences, chapters 27 and 28, pp. 484-528 (18th ed., Alfonso R. Gennaro, Ed., Easton, Pa.: Mack Pub. Co., 1990). More specifically, determining a therapeutically effective amount will depend on such factors as toxicity and efficacy of the medicament. Toxicity may be determined using methods well known in the art and found in the foregoing references. Efficacy may be determined utilizing the same guidance.

### Adjuvants

In some embodiments, a KLH-peptide conjugate composition of the present disclosure (e.g. a KLH-EGFRvIII conjugate) can be combined with or administered concurrently with, at least one adjuvant. Suitable adjuvants include those suitable for use in mammals, preferably in humans. Examples of known suitable adjuvants that can be used in humans include, but are not necessarily limited to, alum, aluminum phosphate, aluminum hydroxide, MF59™ (4.3% w/v squalene, 0.5% w/v polysorbate 80 (Tween 80), 0.5% w/v sorbitan trioleate (Span 85)), CpG-containing nucleic acids (where the cytosine is unmethylated), QS21 (saponin adjuvant), MPL (Monophosphoryl Lipid A), 3DMPL (3-O-deacylated MPL), extracts from Aquilla, ISCOMS (see, e.g., Sjölander et al., J. Leukocyte Biol. 64:713 (1998); PCT Publication Nos. WO 90/03184, WO 96/11711, WO 00/48630, WO 98/36772, WO 00/41720, WO 06/134423 and WO 07/026190), LT/CT mutants, poly(D,L-lactide-co-glycolide) (PLG) microparticles, Quil A, interleukins, and the like. For veterinary applications including but not limited to animal experimentation, one can use Freund's, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion.

Further exemplary adjuvants to enhance effectiveness of the composition include, but are not limited to: (1) oil-in-water emulsion formulations (with or without other specific immunostimulating agents such as muramyl peptides (see below) or bacterial cell wall components), such as for example (a) MF59™ (PCT Publication No. WO 90/14837; Chapter 10 in Vaccine design: the subunit and adjuvant approach, eds. Powell & Newman, Plenum Press 1995), containing 5% Squalene, 0.5% Tween 80 (polyoxyethylene sorbitan mono-oleate), and 0.5% Span 85 (sorbitan trioleate) (optionally containing muramyl tri-peptide covalently linked to dipalmitoyl phosphatidylethanolamine (MTP-PE)) formulated into submicron particles using a microfluidizer, (b) SAF, containing 10% Squalene, 0.4% Tween 80, 5% pluronic-blocked polymer L121, and thr-MDP either microfluidized into a submicron emulsion or vortexed to generate a larger particle size emulsion, and (c) RIBI™ adjuvant system (RAS), (Ribi Immunochem, Hamilton, MT) containing 2% Squalene, 0.2% Tween 80, and one or more bacterial cell wall components such as monophosphorylipid A (MPL), trehalose dimycolate (TDM), and cell wall skeleton (CWS), preferably MPL + CWS (DETOX™); (2) saponin adjuvants, such as QS21, STIMULON™ (Cambridge Bioscience, Worcester, MA), Abisco® (Isconova, Sweden), or Iscomatrix® (Commonwealth Serum Laboratories, Australia), may be used or particles generated therefrom such as ISCOMs (immunostimulating complexes), which ISCOMS may be devoid of additional detergent e.g. PCT Publication No. WO 00/07621; (3) Complete Freund's Adjuvant (CFA) and Incomplete Freund's Adjuvant (IFA); (4) cytokines, such as interleukins (e.g. IL-1, IL-2, IL-4, IL-5, IL-6, IL-7, IL-12 (PCT Publication No. WO 99/44636), *etc.),* interferons (e.g. gamma interferon), granulocyte-macrophage colony stimulating factor (GM-CSF, such as molgramostim or sargramostim), tumor necrosis factor (TNF), *etc.;* (5) monophosphoryl lipid A (MPL) or 3-O-deacylated MPL (3dMPL) e.g. Great Britain Patent No. GB-2220221, and European Patent No. EP-A-0689454, optionally in the substantial absence of alum when used with pneumococcal saccharides e.g. PCT Publication No. WO 00/56358; (6) combinations of 3dMPL with, for example, QS21 and/or oil-in-water emulsions e.g. EP-A-0835318, EP-A-0735898, EP-A-0761231; (7) oligonucleotides comprising CpG motifs [Krieg, Vaccine (2000) 19:618-622; Krieg, Curr Opin Mol Ther (2001) 3:15-24; Roman et al., Nat. Med. (1997) 3:849-854; Weiner et al., PNAS USA (1997) 94:10833-10837; Davis et al, J. Immunol (1998) 160:870-876; Chu et al., J. Exp.Med (1997) 186:1623-1631; Lipford et al, Ear. J. Immunol. (1997) 27:2340-2344; Moldoveami e/ al., Vaccine (1988) 16:1216-1224, Krieg et al., Nature (1995) 374:546-549; Klinman et al., PNAS USA (1996) 93:2879-2883; Ballas et al, J. Immunol, (1996) 157:1840-1845; Cowdery et al, J. Immunol (1996) 156:4570-4575; Halpern et al, Cell Immunol. (1996) 167:72-78; Yamamoto et al, Jpn. J. Cancer Res., (1988) 79:866-873; Stacey et al, J. Immunol., (1996) 157:2116-2122; Messina et al, J. Immunol, (1991) 147:1759-1764; Yi et al, J. Immunol (1996) 157:4918-4925; Yi et al, J. Immunol (1996) 157:5394-5402; Yi et al, J. Immunol, (1998) 160:4755-4761; and Yi et al, J. Immunol, (1998) 160:5898-5906; PCT Publication Nos. WO 96/02555, WO 98/16247, WO 98/18810, WO 98/40100, WO 98/55495, WO 98/37919 and WO 98/52581] *i.e.* containing at least one CG dinucleotide, where the cytosine is unmethylated; (8) a polyoxyethylene ether or a polyoxyethylene ester e.g. PCT Publication No. WO 99/52549; (9) a polyoxyethylene sorbitan ester surfactant in combination with an octoxynol (PCT Publication No. WO 01/21207) or a polyoxyethylene alkyl ether or ester surfactant in combination with at least one additional non-ionic surfactant such as an octoxynol (PCT Publication No. WO 01/21152); (10) a saponin and an immunostimulatory oligonucleotide (e.g. a CpG oligonucleotide) (PCT Publication No. WO 00/62800); (11) an immunostimulant and a particle of metal salt e.g. PCT Publication No. WO 00/23105; (12) a saponin and an oil-in-water emulsion e.g. PCT Publication No. WO 99/11241; (13) a saponin (e.g. QS21) + 3dMPL + IM2 (optionally + a sterol) e.g. PCT Publication No. WO 98/57659; (14) other substances that act as immunostimulating agents to enhance the efficacy of the composition, such as Muramyl peptides including N-acetyl-muramyl-L-threonyl-D-isoglutamine (thr-MDP), N-25 acetyl-normuramyl-L-alanyl-D-isoglutamine (nor-MDP), N-acetylmuramyl-L-alanyl-D-isoglutarninyl-L-alanine-2-(1'-2'-dipalmitoyl-sn-glycero-3-hydroxyphosphoryloxy)-ethylamine MTP-PE), (15) ligands for toll-like receptors (TLR), natural or synthesized (e.g. as described in Kanzler et al., Nature Med. 13:1552-1559 (2007)), including TLR3 ligands such as polyl:C and similar compounds such as Hiltonol and Ampligen.

In one embodiment, a composition of the present disclosure is administered concurrently with, or is combined with prior to administration, at least one adjuvant. In a particular embodiment, said adjuvant is a cytokine, in particular GM-CSF (e.g. molgramostim or sargramostim).

### Articles of Manufacture

In another embodiment of the disclosure, an article of manufacture is provided comprising a container, which holds the liquid composition comprising a KLH-peptide conjugate as described herein, and optionally provides instructions for its use. Suitable containers include, for example, bottles, vials, bags and syringes. The container may be formed from a variety of materials such as glass or plastic. An exemplary container is a 3-20 cc single use glass vial. Alternatively, for a multidose formulation, the container may be a 3-100 cc glass vial. The container holds the composition and the label on, or associated with, the container may indicate directions for use. The article of manufacture may further include other materials desirable from a commercial and user standpoint, including other buffers, diluents, filters, needles, syringes, and package inserts with instructions for use, contraindications, and/or lists of potential side-effects.

The present disclosure also provides a kit for preparing a liquid composition of a KLH-peptide conjugate comprising a first container, comprising a KLH-peptide conjugate, such as a KLH-EGFRvIII conjugate, in solution, and a second container comprising a sufficient amount of an adjuvant, such as GM-CSF, alone or in combination with other excipients.

The present disclosure is further illustrated by the following examples which should not be construed as further limiting. The contents of all figures and all references, patents and published patent applications cited throughout this disclosure are expressly incorporated herein by reference in their entirety.

### EXAMPLES

In the examples that follow, "epitope density" was determined using amino acid analysis, as follows. The coupling of the EGFRvIII peptide to KLH via a bifunctional linker leads to a distribution of peptides conjugated to the KLH carrier protein. The number of peptides conjugated to KLH was measured using amino acid composition analysis. There are several amino acids whose contribution comes exclusively from the KLH carrier protein and not the peptide. By comparing those amino acids to those that originate from both the peptide and KLH, the molar excess can be calculated. The molar excess of these amino acids can be attributed to the EGFRvIII peptide and is used to calculate the number of peptides per KLH subunit. Prior to amino acid analysis, the samples are buffer exchanged to remove any residual process impurities such as peptide dimer or peptide:linker conjugate. These impurities have the potential to contribute to the amino acid levels and skew the results. The samples are then hydrolyzed using 6N HCl, labeled with a fluorescent tag, and separated and quantified using HPLC.

### Example 1: Effect of pH/buffer

The objective of the study was to investigate the effect of pH and buffer species on the storage stability of CDX-110 at different temperatures (5°C, 25°C and 40°C) for both liquid and lyophilized formulations (Table 1A). The pH range explored was 4.5 to 7.4, and the different buffering species were Potassium Phosphate, Sodium Phosphate, Succinate and Histidine. The data reported here is at the end of 12 weeks for all formulations except for succinate (J) that was stopped at the end of 8 weeks. Protein concentration, pH and polysorbate content were kept constant as shown in Table 1A. "liq" indicates a liquid formlation, whereas "lyo" indicates a lyophilized formulation. The pH ranges were chosen for intradermal administration. All comparisons are made against the current formulation F (both liquid and lyo in PBS; composition in Table 1 B).

**Table 1A: Formulation composition for pH/buffer study**

| **Formulation** | **A (liq)** | **A (lyo)** | **B (liq)** | **B (lyo)** | **C (liq)** | **C (lyo)** | **D (liq)** | **D (lyo)** | **E (liq)** | **E (lyo)** | **F (liq)** | **F (lyo)** | **J (8 wk, liq)** |
|---|---|---|---|---|---|---|---|---|---|---|---|---|---|
| **CDX-110 mg/mL** | 1.0 | 1.0 | 1.0 | 1.0 | 1.0 | 1.0 | 1.0 | 1.0 | 1.0 | 1.0 | 1.0 | 1.0 | 1.0 |
| **K Phosphate mM** | 10 | 10 | | | 10 | 10 | 10 | 10 | | | | | |
| **Na Phosphate mM** | | | 10 | 10 | | | | | | | | | |
| **Histidine mM** | | | | | | | | | 15 | 15 | | | |
| **Succinate mM** | | | | | | | | | | | | | 10 |
| **pH** | 7.4 | 7.4 | 7.4 | 7.4 | 7.4 | 7.4 | 7.4 | 7.4 | 6.5 | 6.5 | | | 4.5 |
| **Sucrose, mg/ml** | 80 | 80 | 80 | 80 | | | | | | | | | |
| **Trehalose mg/mL** | | | | | 84 | 84 | 90 | 90 | 84 | 84 | | | |
| **Tween 80 mg/mL** | 0.2 | 0.2 | 0.2 | 0.2 | 0.2 | 0.2 | 0.2 | 0.2 | 0.2 | 0.2 | | | |
| **PBS** | | | | | | | | | | | * | * | |
| **Fill volume (ml)** | 0.6 | 0.6 | 0.6 | 0.6 | 0.6 | 0.6 | 0.6 | 0.6 | 0.6 | 0.6 | 0.6 | 0.6 | 0.6 |

| | | | | | | | | | | | | | |
|---|---|---|---|---|---|---|---|---|---|---|---|---|---|
| * PBS composition given in Table 1 B. Formulation J was studied for 8 weeks only. Formulations C and D differ only in trehalose content by 6 mg/mL. All lyophilized formulations were reconstituted with WFI such that final CDX-110 concentration was -1.0 mg/mL. | | | | | | | | | | | | | |

**Table 1B: Current CDX-110 formulation composition in PBS (F)**

| **Component** | **Concentration** | **Amt per 0.5 mL dose** |
|---|---|---|
| CDX-110 DS | 1.0 mg/mL | 0.5 mg |
| Phosphate buffer (Na₂HPO₄/KH₂PO₄) | 10 mM | 0.72 mg Na2HPO4 |
| | | 0.12 mg KH2PO4 |
| Sodium chloride | 137 mM | 4 mg |
| Potassium Chloride | 2.7 mM | 0.1 mg |

### Preparation of Buffer Solutions

Five buffer solutions were prepared as described in Table 1C below. Each solution was prepared by first dissolving an amount of the buffer species (listed in Table 1C) in water for injection (WFI). The pH of each buffer solution was measured. The buffer solution was then filtered through a sterilization filter (0.22 micron pore size) into a sterilized receptacle for subsequent use.

**Table 1C: Buffer Solutions**

| **Buffer Type** | **pH** | **Buffer Species** | **Buffer Strength (mM)** |
|---|---|---|---|
| Histidine | 6.5 | L-Histidine | 15 |
| | | L-Histidine HCl monohydrate | |
| Succinate | 4.5 | Succinic acid | 10 |
| | | Sodium Succinate | |
| PBS (phosphate buffer saline) | 7.4 | Sodium phosphate dibasic, anhydrous | 10 |
| | | Potassium phosphate monobasic | |
| | | Sodium chloride | |
| | | Potassium chloride | |
| Potassium Phosphate | 7.4 | Potassium phosphate dibasic anhydrous | 10 |
| | | Potassium phosphate monobasic | |
| Sodium | 7.4 | Sodium Phosphate, Monobasic | 10 |
| phosphate | | monohydrate | |
| | | Sodium Phosphate, Dibasic, Anhydrous | |

### Preparation of CDX-110 Formulation

The formulations that were evaluated are listed in Table 1A. To prepare each formulation, CDX-110 material was obtained at 1.0 mg/mL in 10 mM PBS pH 7.4. Buffer exchanges of the CDX-110 material into the above identified formulation solutions were carried out with Amicon Ultra15 MWCO 50 centrifugal concentration on a Beckman Coulter Allegra 21 R centrifuge run at 4500 RPM at 5°C. Approximately eight volume exchanges were made and the solution concentrated between 1.4 to 1.8 mg/mL. Approximately 70 to 99 mL of all formulations were prepared. CDX-110 concentration was determined by Ultraviolet-Visible spectrometry (UV-Vis) method using an extinction coefficient of 1.38 (mg/mL)⁻¹ cm⁻¹ at 280nm.

A 400 mg/mL disaccharide solution was prepared by dilution and dissolution of trehalose/sucrose by the appropriate formulation buffer as described in Table 1A. In addition, a 10 mg/mL polysorbate 80 (PS80) solution was prepared by dissolution of PS80 in the appropriate formulation buffer. Appropriate volumes of the disacchraride stock and PS80 stock solution were then added to the CDX-110 stock solution to obtain a final concentration of 1.0 mg/mL in the formulation compositions listed in Table 1A.

The formulations were then filtered through 0.2 µm sterilizing filters and filled into vials. A fill volume of 0.6 mL was filled in 2 mL type I glass vials. Liquid formulation vials were stoppered with Daikyo 777-1 Flurotec® coated serum stoppers crimped and sealed. Lyophilized formulation vials were partially stoppered with Daikyo 777-1 Flurotec® B2-TR coated, then lyophilized according to the freeze drying cycle parameters outlined in Table 1D, stoppered, crimped and sealed. Both liquid and lyophilized formulations were placed in stability chambers stored at 5, 25 and 40°C for 2, 4, 8, 13, 26, 52 and 104 weeks. The vials were washed and autoclaved, as were the 13 mm Daikyo 777-1 serum and lyo stoppers. The lyo stoppers were dried at 100°C for 6 hours. The samples were analyzed by appearance, pH, and protein concentration by UV-Vis absorbance, size exclusion HPLC, Anion Exchange HPLC, Reverse Phase-HPLC. The study is ongoing and values reported are from intermediate time points or 12 weeks.

**Table 1D: Freeze Drying Cycle Parameters**

| **Step** | **Temperature/Pressure** |
|---|---|
| Freezing | -40°C |
| Annealing | -21°C |
| Freezing | -40°C |
| Primary Drying | -25°C |
| Chamber pressure | 100 mT |
| Secondary Drying | 40°C |
| Stoppering | Under nitrogen |

### Assays

### Appearance

Each formulation was visually evaluated after 0 (initial), and defined time points for particulate formation, color change and clarity. The sample was held against a light and dark background with proper illumination. The sample cannot be more intensely colored than level 6 of the color range and not more opalescent than reference suspension III. Visible particulates were also reported.

### pH

Samples were subjected to a potentiometic pH measurement. pH meter calibration was done using commercial standard buffers ranging from pH 4 to 10.

### Protein Concentration by UV-Vis Absorbance

Protein concentration measurement was carried out using a suitable spectrophotometer. The sample was scanned at 280 nm and the absorbance at 280 nm was used to determine the concentration of the protein. An experimentally derived extinction coefficient of 1.19 (mg/mL)⁻¹ cm⁻¹ and 1.38 (mg/mL)⁻¹ cm⁻¹ (for D liq and D lyo) at 280 nm was used to determine the protein concentration.

### Size Exclusion HPLC

The presence of monomers, dimers and high molecular mass species (HMMS) is monitored using size exclusion chromatography (SEC). The size exclusion chromatography was carried out using Waters Biosuite 450 SEC column, mobile phase 50 mM sodium phosphate, 75 mM NaCl, 0.01% sodium azide, pH 7.4, a flow rate of 0.3 mL/min, and UV detection at 214 nm. The levels of monomers, dimers and HMMS were calculated by integrating the area under the chromatogram peaks for each formulation and reporting the integrated areas of monomer, dimer and high molecular mass species peaks as a percentage of total peak area.

### Anion Exchange HPLC

The anion exchange chromatography was carried out using TSKgel DEAE-5PW column, with mobile phase 10 mM Tris, pH 7.3, and 10 mM Tris, 0.5M NaCl, pH 7.3 gradient elution, a flow rate of 1 mL/min, and UV detection at 280 nm. Anion exchange chromatography separates CDX-110 from KLH, peptide, linker, and related compounds based on their partitioning between a charged stationary phase and a mobile phase of varying ionic strength. The species of interest are detected by ultraviolet absorbance and quantitated by relative area.

### Reverse Phase HPLC

Reverse phase chromatography was carried out using Waters symmetry 300 C4 column, with mobile phase 0.1% TFA water, and 0.1% TFA in acetronitrile gradient elution, a flow rate of 1 mL/min, and UV detection at 215 nm. Reversed phase chromatography separates CDX-110 from peptide and linker-related impurities on their partitioning between a nonpolar stationary phase and a polar mobile phase. Peaks are detected using an ultraviolet light detector at a fixed wavelength where the peptide and linker based impurities have absorption.

### Results

### Appearance

From the appearance analysis, it could be concluded that all formulations in sodium phosphate (B), potassium phosphate (A, C & D) and histidine (E) behaved similarly in both the liquid and lyophilized formulations. Formulation J (liq, Succinate) and F (lyo, PBS) behaved the worst. For details, see liquid and lyophilized formulations outlined in Tables 1A, 1 B, and 1C.

### Liquid formulations (Table 1E)

All T0 (time zero) formulations were reported colorless and NMO RSI. In terms of particulates, 1 white flake particle was observed for formulation D (liq, KPi, Tre) whereas formulation C (liq, KPi, Tre), which only differs in trehalose content by 6 mg/mL, is EFVP essentially free of visible particles (EFVP). Current formulation F (liq, PBS), is also colorless and EFVP.

At 5°C (T=12 weeks), all formulations were colorless and NMO RSI. 1 particle was observed for the B (liq) and C (liq) formulation whereas current formulation F (in PBS) remained EFVP and NMO RSI.

At 25°C (T=12weeks), J (liq, succinate - NMO RSII) and D (liq, KPi, Tre - NMO RSI) formulations were reported to be very slightly brown. F (liq, PBS) formulation was reported to have a number of different types of particles while D (liq, KPi, Tre) had 1 particle.

At 40°C (T=12 weeks), all formulations changed color to yellow or brown (except PBS i.e., F) with J (liq, succinate) being the worst in terms of opalescence (NMO RS IV). Potassium phosphate liquid formulations C (liq, KPi, Tre) and D (liq, KPi, Tre) were reported to be very slightly brown with one or no particles. Formulation F (liq, PBS) was colorless with two white flake-like particles. Formulation F (liq, PBS) formulation was the only one without PS 80.

### Lyophilized formulations (Table 1E)

All formulations at T=0 were colorless except for F (lyo, PBS) formulation (slightly brown on reconstitution) which also had many brown/tan particles resembling cardboard packaging. A (lyo, KPi, Suc) also had one particle on reconstitution.

At 5°C (T=12 weeks) all formulations were colorless and EFVP except for F (lyo, PBS) which had many particles and opalescent. At 25°C (T=12 weeks) all formulations except F (lyo, PBS) were colorless and NMO RSI. Two small white particles were also reported for C (lyo, KPi, Tre). At 40°C (T=12 weeks), all formulations were colorless and NMO RSI except for F (lyo, PBS) on reconstitution. One and two particles were observed in B (lyo, NaPi, Suc) and D (lyo, KPi, Tre), respectively, while F (lyo, PBS) had multiple particles.

### Concentration

### Liquid formulations (Table 1F)

At 5°C (T=12 weeks) no significant change was observed. At 25°C (T=12weeks), an increase in concentration was observed for E (liq, His, Tre) formulation. All other formulations did not show any significant change. At 40°C, formulations C (liq, KPi, Tre), D (liq, KPi, Tre) and F (liq, PBS) did not show any significant change while A (liq, KPi, Suc), B (liq, NaPi, Suc) and E (liq, His, Tre) showed a marked increase in concentration.

### Lyophilized formulations (Table 1F)

At 5°C (T=12 weeks) no significant changes were observed except for F (lyo, PBS) which showed a slight drop in concentration. At 25°C (T=12 weeks), no significant changes except for a slight decrease for E (lyo, His, Tre) and F (lyo, PBS). At 40°C (T=12weeks) no significant changes except for F (lyo, PBS). A slight increase was observed for A (lyo, KPi, Suc).

### A280/A340 Ratio

The ratio of absorbance at 280 nm to absorbance at 340 assumes importance due to the presence of bound copper in CDX-110 which gives it a characteristic blue color. The 340 nm absorbance is due to the presence of copper and therefore a reduction of absorbance at this wavelength indicates a loss of copper or change in copper oxidation state.

### Liquid formulations (Table 1G)

At 5°C (T=12 weeks) there were no significant changes in the ratio except for formulation A (liq, KPi, Suc) where an increase is observed. At 25°C (T=12 weeks) all liquid formulations show an increase in the ratio indicating loss/changes to the copper molecule except for formulation J (liq, succinate). Formulation J (liq, succinate) showed a decrease in the ratio unlike the other liquid formulations. Some of the formulations showed a greater change in ratio compared to others e.g., formulations C (liq, KPi, Tre), D (liq, KPi, Tre) and F (liq, PBS) had a more significant increase than A (liq, KPi, Suc) and B (liq, NaPi, Suc). E (liq, His, Tre) had a moderate increase in the ratio.

At 40°C, formulation J (liq, succinate) shows a significant decrease in the ratio (Table 1 B). Formulations C, D and E show a significant increase with F showing the highest increase. The ratio for sucrose containing formulations A (liq, KPi, Suc) and B (liq, NaPi, Suc) appear to be constant. A closer look at the data revealed that the sucrose containing formulations show an increase in absorbance at 280 nm as well as an increase in the absorbance at 340 nm with time and temperature, thus allowing the A280 to A340 ratio to appear to be constant. For trehalose and PBS containing formulations, an increase in this ratio is observed due to a decrease in the absorbance at 340 nm.

### Lyophilized formulations (Table 1G)

At 5°C (T=12 weeks) no/minimal change observed except for F (lyo, PBS). At 25°C no/minimal change observed except for F (lyo, PBS). At 40°C, no significant change in the ratio is observed for all formulations except for F (lyo, PBS).

### Dimer/Monomer/HMMS

### Liquid formulations (Tables 1H, 1I and 1J)

Dimer content (Table 1H) decreased for all formulations with increasing temperature. The smallest decrease was observed for C (liq, KPi, Tre) and D (liq, KPi, Tre) formulations. Monomer content increased for all sugar containing formulations whereas the histidine (E) and PBS (F) formulations showed a decrease. The histidine formulation showed a marked decrease in monomer content (Table 1 H) at 25 and 40°C with a simultaneous increase in the HMMS content (Table 11). An exact opposite trend was observed for the sodium phosphate, potassium phosphate formulations where an increased formation of monomer from HMMS occurred at higher temperatures.

### Lyophilized formulations (Table 1H, 1I and 1J)

All lyophilized formulations showed no/minimal changes at 5, 25 and 40°C at the end of 12 weeks except for the lyophilized PBS formulation (F lyo, PBS).

### Percent Impurity and CDX-110 retention time changes by AEX

### Liquid formulations (Table 1K and 1L)

At 5°C (T=12 weeks) percent impurity for all formulations was less than or equal to 1% except for the succinate formulation (I). Retention time for the CDX-110 peak moved approximately one minute for all formulations except succinate. The shift in retention time indicated formation of acidic species which can be attributed to a combination of linker hydrolysis and deamidation. At 25°C and 40°C (T=12 weeks), percent impurity increased significantly for all formulations. Change in retention time also followed a similar trend indicating increased formation of acidic species.

### Lyophilized formulations (Table 1K and 1L)

For all lyophilized formulations, the percent impurity change and the shift in retention times at all temperatures were not significant except for the F (lyo, PBS) formulation.

### Percent Impurity by RPHPLC

### Liquid formulation (Table 1M)

An increased formation of peptide and linker related impurities were observed for formulations with increasing temperature, with histidine formulation (E) showing the least formation of impurities.

### Lyophilized formulations (Table 1M)

No significant change was observed for any of the formulations except for the lyophilized PBS (F) formulation.

### Mouse potency

Drug product specification for potency is set at ≥75% seroconversion in mice. Formulations tested were potassium phosphate (A, C, D), histidine (E) and PBS (F). Samples tested were stored at 5°C for 8 and 12 weeks. All samples demonstrated >75% seroconversion (Table 1 K) and therefore met the drug product specifications.

**Table 1E: Appearance Data for pH/buffer Study**

| **Formulation** | **Appearance at T=0 wk** | **Appearance at 5C T=12 wk** | **Appearance at 25C T=12 wk** | **Appearance at 40C T=12 wk** |
|---|---|---|---|---|
| J Succinate, PS80, pH 4.5, **liq*** | NMO RSI Colorless (<B9) EFVP | NMO RSI Colorless (<B9) EFVP | NMO RSII V SI brown (<B7) EFVP | NMO RS IV Brown (<B6) 2 floc (white globular particles) |
| A (liq) KPO₄, Suc-80 mg/ml, PS80, pH 7.4, **liq** | NMO RSI Colorless (<B9) EFVP | NMO RSI Colorless (<B9) EFVP | NMO RSI Colorless (<B9) EFVP | NMO RSI SI brown yellow (<BY6) 1 small white round particle |
| A (lyo) KPO₄, Suc-80 mg/ml, PS80, pH 7.4, **lyo** | NMO RSI Colorless (<B9) 1 short white silver-like particle | NMO RSI Colorless (<B9) EFVP | NMO RSI Colorless (<B9) EFVP | NMO RSI Colorless (<B9) EFVP |
| B (liq) NaPO₄, Suc-80 mg/ml, PS80, pH 7.4, **liq** | NMO RSI Colorless (<B9) EFVP | NMO RSI Colorless (<B9) 1 white, 1 tan short fiberlike Particle | NMO RSI Colorless (<B9) EFVP | NMO RSI SI Brown Yellow (≤BY6) EFVP |
| B (lyo) NaPO₄, Suc-80 mg/ml, PS80, pH 7.4, **lyo** | NMO RSI Colorless (< B9) EFVP | NMO RSI Colorless (<B9) EFVP | NMO RSI Colorless (<B9) EFVP | NMO RSI Colorless (<B9) 1 short white fiberlike particle |
| C (liq) KPO4, Tre-84 mg/ml, PS80, pH | NMO RSI Colorless (<B9) | NMO RSI Colorless (<B9) 1 shortwhite | NMO RSI Colorless (<B9) EFVP | NMO RSI SI brown yellow (<BY7) |
| 7.4, **liq** | EFVP | fiberlike particle, and 3 small white round particles | | 1 small white round particle |
| C (lyo) KPO4, Tre-84 mg/ml, PS80, pH 7.4, **lyo** | NMO RSI Colorless (<B9) EFVP | NMO RSI Colorless (<B9) EFVP | NMO RSI Colorless (<B9) 2 small white round particle | NMO RSI Colorless (<B9) EFVP |
| D (liq) KPO4, Tre-90 mg/ml, PS80, pH 7.4, **liq** | NMO RSI Colorless (<B9) 1 white flake like particle | NMO RSI Colorless (<B9) EFVP | NMO RSI v slight brown (<B7) 1small white round particle | NMO RSI v slight brown (<B7) EFVP |
| D **(lyo)** KPO4, Tre-90 mg/ml, PS80, pH 7.4, **lyo** | NMO RSI Colorless (<B9) EFVP | NMO RSI Colorless (<B9) EFVP | NMO RSI Colorless (<B9) EFVP | NMO RSI Colorless (<B9) 2 small white round particles |
| E (liq) His, Tre-84 mg/ml, PS80, pH 6.5, **liq** | NMO RSI Colorless (<B9) EFVP | NMO RSI Colorless (<B9) EFVP | NMO RSI Colorless (<B9) EFVP | NMO RS II SI Brown Yellow (<B6) EFVP |
| E (lyo) His, Tre-84 mg/ml, PS80, pH 6.5, **lyo** | NMO RSI Colorless (<B9) EFVP | NMO RSI Colorless (<B9) EFVP | NMO RSI Colorless (<B9) EFVP | NMO RSI Colorless (<B9) EFVP |
| F (liq) PBS, pH 7.4, **liq** | NMO RSI Colorless (<B9) EFVP | NMO RSI Colorless (<B9) EFVP | NMO RSI Colorless (<B9) ∼5 short white fiber like particles; ∼5 small white globular particles; ∼6 small white flake-like particles | NMO RSI Colorless (<B9) 2 white flake like particles |
| F (lyo) PBS, pH 7.4, **lyo** | NMO RSI Slightly brown (<B6) Many (∼15) short brown /tan fibers. Typical of cardboard packaging. 2 short white silverlike particles | NMO RSII Very Slightly Brown (≤B7) ∼10 tan short fiberlike and 3 white globular particles (floc) | NMO RSIII Very Slightly Brown (≤B7) 2 short white fiberlike and 4 white globular particles (floc) | NMO RSIII Very Slightly Brown (≤B7) 3 tan short fiberlike, 2 small white round, 5 white globular particles (flocs) |

| | | | | |
|---|---|---|---|---|
| EFVP: Essentially free of visible particles; NMO RS I/II/III: Not more opalescent than reference suspension I/II/III. * Formulation J was discontinued after 8 weeks. Data reported is at the end of 8 weeks | | | | |

**Table 1F: Change in Concentration**

| **Formulation** | **Change in concentration (mg/mL) at 5C in 12 wks** | **Change in concentration (mg/mL) at 25C in 12 wks** | **Change in concentration (mg/mL) at 40C in 12 wks** |
|---|---|---|---|
| J Succinate, PS80, pH 4.5, **liq*** | -0.05 | 0.01 | 0.40 |
| A (liq) KPO₄, Suc-80 mg/ml, PS80, pH 7.4, **liq** | 0.14 | 0.04 | 0.35 |
| A (lyo) KPO₄, Suc-80 mg/ml, PS80, pH 7.4, **lyo** | 0.08 | 0.03 | 0.13 |
| B (liq) NaPO₄, Suc-80 mg/ml, PS80, pH 7.4, **liq** | -0.04 | 0.05 | 0.31 |
| B (lyo) NaPO₄, Suc-80 mg/ml, PS80, pH 7.4, **lyo** | 0.08 | -0.01 | -0.03 |
| C (liq) KPO4, Tre-84 mg/ml, PS80, pH 7.4, **liq** | 0.01 | 0.03 | 0.07 |
| C (lyo) KPO4, Tre-84 mg/ml, PS80, pH 7.4, **lyo** | 0.03 | 0.02 | 0.09 |
| D (liq) KPO4, Tre-90 mg/ml, PS80, pH 7.4, **liq** | -0.01 | 0.00 | 0.02 |
| D (lyo) KPO4, Tre-90 mg/ml, PS80, pH 7.4, **lyo** | 0.02 | 0.04 | 0.02 |
| E (liq) His, Tre-84 mg/ml, PS80, pH 6.5, **liq** | 0.05 | 0.18 | 0.47 |
| E (lyo) His, Tre-84 mg/ml, PS80, pH 6.5, **lyo** | -0.07 | -0.11 | 0.09 |
| F (liq) PBS, pH 7.4, **liq** | 0.02 | 0.03 | 0.05 |
| F (lyo) PBS, pH 7.4, **lyo** | -0.09 | -0.12 | -0.45 |

| | | | |
|---|---|---|---|
| * Formulation J was discontinued after 8 weeks. Data reported is at the end of 8 weeks. | | | |

**Table 1G: Change in A280/A340 Ratio**

| **Formulation** | **Change in A280/A340 at 5C in 12 wks** | **Change in A280/A340 at 25C in 12 wks** | **Change in A280/A340 at 40C in 12 wks** |
|---|---|---|---|
| J Succinate, PS80, pH 4.5, **liq*** | 0 | -0.54 | -4.12 |
| A (liq) KPO₄, Suc-80 mg/ml, PS80, pH 7.4, **liq** | 0 | 0.45 | 0 |
| A (lyo) KPO₄, Suc-80 mg/ml, PS80, pH 7.4, **lyo** | -0.02 | 0.01 | 0.12 |
| B (liq) NaPO₄, Suc-80 mg/ml, PS80, pH 7.4, **liq** | 0.21 | 0.45 | 0.21 |
| B (lyo) NaPO₄, Suc-80 mg/ml, PS80, pH 7.4, **lyo** | -0.02 | -0.06 | 0.06 |
| C (liq) KPO4, Tre-84 mg/ml, PS80, pH 7.4, **liq** | 0.01 | 2.79 | 5.65 |
| C (lyo) KPO4, Tre-84 mg/ml, PS80, pH 7.4, **lyo** | 0.09 | 0.03 | 0.98 |
| D (liq) KPO4, Tre-90 mg/ml, PS80, pH 7.4, **liq** | -0.01 | 2.59 | 5.45 |
| D (lyo) KPO4, Tre-90 mg/ml, PS80, pH 7.4, **lyo** | 0.13 | 0 | 0.20 |
| E (liq) His, Tre-84 mg/ml, PS80, pH 6.5, **liq** | 0.01 | 1.53 | 3.98 |
| E (lyo) His, Tre-84 mg/ml, PS80, pH 6.5, **lyo** | 0.02 | 0.24 | 0.20 |
| F (liq) PBS, pH 7.4, **liq** | 0.02 | 2.59 | 7.95 |
| F (lyo) PBS, pH 7.4, **lyo** | 0.34 | -0.03 | 0.98 |

| | | | |
|---|---|---|---|
| * Formulation J was discontinued after 8 weeks. Data reported is at the end of 8 weeks. | | | |

**Table 1H: Change in Percent Dimer**

| **Formulation** | **Change in dimer % at 5C in 12 wks** | **Change in dimer % at 25C in 12 wks** | **Change in dimer % at 40C in 12 wks** |
|---|---|---|---|
| J Succinate, PS80, pH 4.5, **liq*** | NM | NM | NM |
| A (liq) KPO₄, Suc-80 mg/ml, PS80, pH 7.4, **liq** | -0.12 | -4.08 | -41.98 |
| A (lyo) KPO₄, Suc-80 mg/ml, PS80, pH 7.4, **lyo** | 1.50 | 1.50 | 0.70 |
| B (liq) NaPO₄, Suc-80 mg/ml, PS80, pH 7.4, **liq** | -0.13 | -4.93 | -45.73 |
| B (lyo) NaPO₄, Suc-80 mg/ml, PS80, pH 7.4, **lyo** | 1.20 | -0.10 | -0.80 |
| C (liq) KPO4, Tre-84 mg/ml, PS80, pH 7.4, **liq** | 0.19 | -4.41 | -14.61 |
| C (lyo) KPO4, Tre-84 mg/ml, PS80, pH 7.4, **lyo** | 1.70 | 1.10 | -0.30 |
| D (liq) KPO4, Tre-90 mg/ml, PS80, pH 7.4, **liq** | -1.20 | -4.20 | -18.40 |
| D (lyo) KPO4, Tre-90 mg/ml, PS80, pH 7.4, **lyo** | -0.90 | -0.70 | -0.80 |
| E (liq) His, Tre-84 mg/ml, PS80, pH 6.5, **liq** | -0.29 | -3.41 | -46.71 |
| E (lyo) His, Tre-84 mg/ml, PS80, pH 6.5, **lyo** | 0.60 | -1.10 | -0.30 |
| F (liq) PBS, pH 7.4, **liq** | -0.35 | -2.35 | -23.65 |
| F (lyo) PBS, pH 7.4, **lyo** | -0.10 | -15.60 | -30.60 |

| | | | |
|---|---|---|---|
| * Formulation J was discontinued after 8 weeks. Data reported is at the end of 8 weeks; NM-not measured since assay methods were different when the study was performed. | | | |

**Table 1I: Change in Percent Monomer**

| **Formulation** | **Change in monomer % at 5C in 12 wks** | **Change in monomer % at 25C in 12 wks** | **Change in monomer % at 40C in 12 wks** |
|---|---|---|---|
| J Succinate, PS80, pH 4.5, **liq*** | NM | NM | NM |
| A (liq) KPO₄, Suc-80 mg/ml, PS80, pH 7.4, **liq** | 0.24 | 4.64 | 26.94 |
| A (lyo) KPO₄, Suc-80 mg/ml, PS80, pH 7.4, **lyo** | -0.20 | -0.70 | -0.20 |
| B (liq) NaPO₄, Suc-80 mg/ml, PS80, pH 7.4, **liq** | -0.92 | 5.52 | 35.32 |
| B (lyo) NaPO₄, Suc-80 mg/ml, PS80, pH 7.4, **lyo** | -0.30 | -0.20 | -0.30 |
| C (liq) KPO4, Tre-84 mg/ml, PS80, pH 7.4, **liq** | 0.2 | 4.20 | 8.30 |
| C (lyo) KPO4, Tre-84 mg/ml, PS80, pH 7.4, **lyo** | -0.80 | -0.60 | -0.20 |
| D (liq) KPO4, Tre-90 mg/ml, PS80, pH 7.4, **liq** | 0.70 | 4.10 | 11.30 |
| D (lyo) KPO4, Tre-90 mg/ml, PS80, pH 7.4, **lyo** | -0.10 | 0.00 | 0.30 |
| E (liq) His, Tre-84 mg/ml, PS80, pH 6.5, **liq** | -0.94 | 2.96 | -19.34 |
| E (lyo) His, Tre-84 mg/ml, PS80, pH 6.5, **lyo** | -0.40 | -0.20 | -0.40 |
| F (liq) PBS, pH 7.4, **liq** | 0.78 | 2.28 | -3.02 |
| F (lyo) PBS, pH 7.4, **lyo** | 0.80 | -2.00 | -2.70 |

| | | | |
|---|---|---|---|
| * Formulation J was discontinued after 8 weeks. Data reported is at the end of 8 weeks; NM-not measured since assay methods were different when the study was performed. | | | |

**Table 1J: Change in Percent HMMS**

| **Formulation** | **Change in HMMS % at 5C in 12 wks** | **Change in HMMS % at 25C in 12 wks** | **Change in HMMS % at 40C in 12 wks** |
|---|---|---|---|
| J Succinate, PS80, pH 4.5, **liq*** | NM | NM | NM |
| A (liq) KPO₄, Suc-80 mg/ml, PS80, pH 7.4, **liq** | -0.33 | -2.63 | -12.53 |
| A (lyo) KPO₄, Suc-80 mg/ml, PS80, pH 7.4, **lyo** | -0.30 | -0.50 | -0.40 |
| B (liq) NaPO₄, Suc-80 mg/ml, PS80, pH 7.4, **liq** | -1.27 | -2.97 | -12.97 |
| B (lyo) NaPO₄, Suc-80 mg/ml, PS80, pH 7.4, **lyo** | 0.10 | 0.80 | 1.10 |
| C (liq) KPO4, Tre-84 mg/ml, PS80, pH 7.4, **liq** | -0.16 | -2.36 | -6.66 |
| C (lyo) KPO4, Tre-84 mg/ml, PS80, pH 7.4, **lyo** | -0.6 | -0.40 | 0.60 |
| D (liq) KPO4, Tre-90 mg/ml, PS80, pH 7.4, **liq** | -0.10 | -1.70 | -4.30 |
| D (lyo) KPO4, Tre-90 mg/ml, PS80, pH 7.4, **lyo** | 0.20 | -0.10 | -0.20 |
| E (liq) | -0.80 | -2.20 | 68.40 |
| His, Tre-84 mg/ml, PS80, pH 6.5, **liq** | | | |
| E (lyo) His, Tre-84 mg/ml, PS80, pH 6.5, **lyo** | 0.10 | 1.30 | 0.90 |
| F (liq) PBS, pH 7.4, **liq** | -0.11 | -1.21 | 21.39 |
| F (lyo) PBS, pH 7.4, **lyo** | -0.30 | 18.20 | 33.30 |

| | | | |
|---|---|---|---|
| * Formulation J was discontinued after 8 weeks. Data reported is at the end of 8 weeks; NM-not measured since assay methods were different when the study was performed. | | | |

**Table 1K: Change in Percent Impurities by AEX**

| **Formulation** | **Change in AEX % impurities at 5C in 12 wks** | **Change in AEX % impurities at 25C in 12 wks** | **Change in AEX % impurities at 40C in 12 wks** |
|---|---|---|---|
| J Succinate, PS80, pH 4.5, **liq*** | 3.45 | 5.80 | 17.70 |
| A (liq) KPO₄, Suc-80 mg/ml, PS80, pH 7.4, **liq** | 0.30 | 1.20 | 1.60 |
| A (lyo) KPO₄, Suc-80 mg/ml, PS80, pH 7.4, **lyo** | 0.20 | 0.20 | 0.10 |
| B (liq) NaPO₄, Suc-80 mg/ml, PS80, pH 7.4, **liq** | 0.10 | 1.00 | 1.30 |
| B (lyo) NaPO₄, Suc-80 mg/ml, PS80, pH 7.4, **lyo** | 0.10 | 0.20 | 0.10 |
| C (liq) KPO4, Tre-84 mg/ml, PS80, pH 7.4, **liq** | 0.10 | 0.9 | 2.60 |
| C(lyo) KPO4, Tre-84 mg/ml, PS80, pH 7.4, **lyo** | 0.20 | 0.10 | 0 |
| D (liq) KPO4, Tre-90 mg/ml, PS80, pH 7.4, **liq** | 1.00 | 2.70 | 3.50 |
| D (lyo) KPO4, Tre-90 mg/ml, PS80, pH 7.4, **lyo** | 1.10 | 0.50 | 0.40 |
| E (liq) His, Tre-84 mg/ml, PS80, pH 6.5, **liq** | -0.80 | 2.70 | 6.70 |
| E (lyo) His, Tre-84 mg/ml, PS80, pH 6.5, **lyo** | -1.40 | -1.20 | -0.90 |
| F (liq) PBS, pH 7.4, **liq** | 0.90 | 1.90 | 3.30 |
| F (lyo) PBS, pH 7.4, **lyo** | 0.40 | 0.50 | 3.30 |

| | | | |
|---|---|---|---|
| * Formulation J was discontinued after 8 weeks. Data reported is at the end of 8 weeks. | | | |

**Table 1 L: Change in AEX Retention Time**

| **Formulation** | **Change in AEX RT (mins) at 5C in 12 wks** | **Change in AEX RT (mins0 at 25C in 12 wks** | **Change in AEX RT (mins) at 40C in 12 wks** |
|---|---|---|---|
| J Succinate, PS80, pH 4.5, **liq*** | 0.01 | 1.21 | 3.02 |
| A (liq) KPO₄, Suc-80 mg/ml, PS80, pH 7.4, **liq** | 1.05 | 2.93 | 8.91 |
| A (lyo) KPO₄, Suc-80 mg/ml, PS80, pH 7.4, **lyo** | 0.80 | 0.99 | 0.97 |
| B (liq) NaPO₄, Suc-80 mg/ml, PS80, pH 7.4, **liq** | 0.93 | 2.97 | 8.65 |
| B (lyo) NaPO₄, Suc-80 mg/ml, PS80, pH 7.4, **lyo** | 0.80 | 0.92 | 0.85 |
| C (liq) KPO4, Tre-84 mg/ml, PS80, pH 7.4, **liq** | 0.84 | 2.70 | 6.27 |
| C (lyo) KPO4, Tre-84 mg/ml, PS80, pH 7.4, **lyo** | 0.74 | 0.90 | 0.81 |
| D (liq) KPO4, Tre-90 mg/ml, PS80, pH 7.4, **liq** | 0.76 | 1.98 | 5.28 |
| D (lyo) KPO4, Tre-90 mg/ml, PS80, pH 7.4, **lyo** | -0.42 | -0.44 | 0.55 |
| E (liq) His, Tre-84 mg/ml, PS80, pH 6.5, **liq** | 0.93 | 2.19 | 4.98 |
| E (lyo) His, Tre-84 mg/ml, PS80, pH 6.5, **lyo** | 0.80 | 0.90 | 0.88 |
| F (liq) PBS, pH 7.4, **liq** | 1.05 | 3.35 | 6.90 |
| F (lyo) PBS, pH 7.4, **lyo** | 0.98 | 1.60 | 2.78 |

| | | | |
|---|---|---|---|
| * Formulation J was discontinued after 8 weeks. Data reported is at the end of 8 weeks. | | | |

**Table 1M: Change in Percent Impurities by Reverse Phase**

| **Formulation** | **Change in RP impurities % at 5C in 12 wks** | **Change in RP impurities % at 25C in 12 wks** | **Change in RP impurities % at 40C in 12 wks** |
|---|---|---|---|
| J Succinate, PS80, pH 4.5, **liq*** | NM | NM | NM |
| A (liq) KPO₄, Suc-80 mg/ml, PS80, pH 7.4, **liq** | 1.90 | 3.10 | 11.00 |
| A (lyo) KPO₄, Suc-80 mg/ml, PS80, pH 7.4, **lyo** | 1.10 | 0.90 | 0.90 |
| B (liq) NaPO₄, Suc-80 mg/ml, PS80, pH 7.4, **liq** | 2.00 | 3.40 | 14.80 |
| B (lyo) NaPO₄, Suc-80 mg/ml, PS80, pH 7.4, **lyo** | 0.90 | 0.70 | 0.70 |
| C (liq) KPO4, Tre-84 mg/ml, PS80, pH 7.4, **liq** | 1.90 | 3.0 | 10.00 |
| C (lyo) KPO4, Tre-84 mg/ml, PS80, pH 7.4, **lyo** | 1.0 | 1.10 | 0.80 |
| D (liq) KPO4, Tre-90 mg/ml, PS80, pH 7.4, **liq** | 0.40 | 1.90 | 8.00 |
| D (lyo) KPO4, Tre-90 mg/ml, PS80, pH 7.4, **lyo** | -0.10 | -0.20 | -0.40 |
| E (liq) His, Tre-84 mg/ml, PS80, pH 6.5, **liq** | 2.0 | 3.60 | 3.10 |
| E (lyo) His, Tre-84 mg/ml, PS80, pH 6.5, **lyo** | 0.90 | 0.80 | 0.80 |
| F (liq) PBS, pH 7.4, **liq** | 1.50 | 3.30 | 5.70 |
| F (lyo) PBS, pH 7.4, **lyo** | 0.40 | -0.10 | 2.70 |

| | | | |
|---|---|---|---|
| * Formulation J was discontinued after 8 weeks. Data reported is at the end of 8 weeks; NM-not measured since assay methods were different when the study was performed. | | | |

**Table 1N: Mouse Potency Results**

| **Formulation** | **% Seroconversion*** | | |
|---|---|---|---|
| | T=0 | T=8 weeks | T=012 weeks |
| **A (lyo)** | 83 | NM | NM |
| **C (liq)** | 100 | NM | 100 |
| **C (lyo)** | 100 | NM | 100 |
| **D (liq)** | 92 | NM | NM |
| **D (lyo)** | 100 | 100 | NM |
| **F (liq)** | 100 | NM | 92 |
| **G (liq)** | 100 | NM | NM |
| **E (liq)** | 100 | NM | NM |
| **E (lyo)** | 100 | NM | NM |

| | | | |
|---|---|---|---|
| * DP Spec ≥ 75%; NM: Not measured. | | | |

### Example 2: Effect of Disaccharides

A study was carried out in which various liquid and lyophilized formulations were prepared to study the effect of disaccharides at pH 7.4. Trehalose and sucrose were used for the study. The C and D formulations have two different concentrations of trehalose with the rest of the components the same. The protein concentration and pH was kept constant as shown in Table 2A. Potassium phosphate and sodium phosphate buffering species were used for these formulations. All comparisons are made against the current formulation (formulation F - both liquid and lyo) in PBS (see Table 1B).

**Table 2A: Formulation Compositions for Effect of Disaccharide**

| **Formulation** | **A (liq)** | **A (lyo)** | **B (liq)** | **B (lyo)** | **C (liq)** | **C (lyo)** | **D (liq)** | **D (lyo)** | **F (liq)** | **F (lyo)** |
|---|---|---|---|---|---|---|---|---|---|---|
| **CDX-110 mg/ml** | 1.0 | 1.0 | 1.0 | 1.0 | 1.0 | 1.0 | 1.0 | 1.0 | 1.0 | 1.0 |
| **Na Phosphate mM** | | | 10 | 10 | | | | | | |
| **K Phosphate mM** | 10 | 10 | | | 10 | 10 | 10 | 10 | | |
| **Histidine mM** | | | | | | | | | | |
| **Succinate mM** | | | | | | | | | | |
| **pH** | 7.4 | 7.4 | 7.4 | 7.4 | 7.4 | 7.4 | 7.4 | 7.4 | 7.4 | 7.4 |
| **Sucrose, mg/ml** | 80 | 80 | 80 | 80 | | | | | | |
| **Trehalose mg/ml** | | | | | 84 | 84 | 90 | 90 | | |
| **Tween 80 mg/ml** | 0.2 | 0.2 | 0.2 | 0.2 | 0.2 | 0.2 | 0.2 | 0.2 | * | * |
| **Fill volume (ml)** | 0.6 | 0.6 | 0.6 | 0.6 | 0.6 | 0.6 | 0.6 | 0.6 | 0.6 | 0.6 |

| | | | | | | | | | | |
|---|---|---|---|---|---|---|---|---|---|---|
| * PBS composition as given in Table 1B. Formulations C and D differ only in trehalose content by 6 mg/mL. All lyophilized formulations were reconstituted with WFI such that final CDX-110 concentration was -1.0 mg/mL. | | | | | | | | | | |

The analytical assays performed for stability were Appearance, UV, pH, SEC, AEX and RPHLC. Tables 2B - 2K show data on stability for multiple degradation pathways including change in monomer/dimer/high molecular weight species content, formation of peptide-linker related impurities, change in A280/A340 ratio, AEX retention time of the CDX-110 peak due to formation of acidic species and mouse potency studies.

### Preparation of Buffer Solutions

Five buffer solutions were prepared as described in Table 2B below. Each solution was prepared by first dissolving an amount of the buffer species (listed in Table 2B) in water for injection (WFI). The pH of each buffer solution was measured. The buffer solution was then filtered through a sterilization filter (0.22 micron pore size) into a sterilized receptacle for subsequent use.

**Table 2B: Buffer Solutions**

| **Buffer Type** | **pH** | **Buffer Species** | **Buffer Strength (mM)** |
|---|---|---|---|
| Histidine | 6.5 | L-Histidine L-Histidine HCl monohydrate | 15 |
| Succinate | 4.5 | Succinic acid Sodium Succinate | 10 |
| PBS (phosphate buffer saline) | 7.4 | Sodium phosphate dibasic, anhydrous Potassium phosphate monobasic Sodium chloride Potassium chloride | 10 |
| Potassium Phosphate | 7.4 | Potassium phosphate dibasic anhydrous Potassium phosphate monobasic | 10 |
| Sodium phosphate | 7.4 | Sodium Phosphate, Monobasic monohydrate Sodium Phosphate, Dibasic, Anhydrous | 10 |

### Preparation of CDX-110 Formulations

The formulations that were evaluated are listed in Table 2A. To prepare each formulation, CDX-110 material was obtained at 1.0 mg/mL in 10 mM PBS pH 7.4. Buffer exchanges of the CDX-110 material into the above identified formulation solutions were carried out with Amicon Ultra15 MWCO 50 centrifugal concentration on a Beckman Coulter Allegra 21 R centrifuge run at 4500 RPM at 5°C. Approximately eight volume exchanges were made and the solution concentrated between 1.4 to 1.8 mg/mL. Approximately 70 to 99 mL of all formulations were prepared. CDX-110 concentration was determined by Ultraviolet-Visible spectrometry (UV-Vis) methods using an extinction coefficient of 1.38 (mg/mL)⁻¹ cm⁻¹ at 280nm.

A 400 mg/mL disaccharide solution was prepared by dilution and dissolution of trehalose/sucrose by the appropriate formulation buffer as described in Table 2A. In addition, a 10 mg/mL polysorbate 80 (PS80) solution was prepared by dissolution of PS80 in the appropriate formulation buffer as described above. Appropriate volumes of the disacchraride stock and PS80 stock solution were then added to the CDX-110 stock solution to obtain a final concentration of 1.0 mg/mL in the formulation compositions listed in Table 2A.

The formulations were then filtered through 0.2 µm sterilizing filters and filled into vials. A fill volume of 0.6 mL was filled in 2 mL type I glass vials. Liquid formulation vials were stoppered with Daikyo 777-1 Flurotec® coated serum stoppers crimped and sealed. Lyophilized formulation vials were partially stoppered with Daikyo 777-1 Flurotec® B2-TR coated, then lyophilized according to the freeze drying cycle parameters outlined in Table 2C, stoppered, crimped and sealed. Both liquid and lyophilized formulations were placed in stability chambers stored at 5, 25 and 40°C for 2, 4, 8, 13, 26, 52 and 104 weeks. The vials were washed and autoclaved, as were the 13 mm Daikyo 777-1 serum and lyo stoppers. The lyo stoppers were dried at 100°C for six hours. The samples were analyzed by appearance, pH, protein concentration by UV-Vis absorbance, Size Exclusion HPLC, Anion Exchange HPLC, and Reverse Phase-HPLC. The study is ongoing and values reported are from intermediate time points or 12 weeks.

**Table 2C: Freeze Drying Cycle Parameters**

| **Step** | **Temp/Pressure** |
|---|---|
| Freezing | -40°C |
| Annealing | -21°C |
| Freezing | -40°C |
| Primary Drying | -25°C |
| Chamber Pressure | 100mT |
| Secondary Drying | 40°C |
| Stoppering | Under nitrogen |

### Assays

### Appearance

Each formulation was visually evaluated after 0 (initial) and defined time points for particulate formation, color change and clarity. The sample was held against a light and dark background with proper illumination. The sample cannot be more intensely colored than level 6 of the color range and not more opalescent than reference suspension III. Visible particulates were also reported.

### pH

Samples were subjected to a potentiometic pH measurement. pH meter calibration was done using commercial standard buffers ranging from pH 4 to 10.

### Protein Concentration by UV-Vis Absorbance

Protein concentration measurement was carried out using a suitable spectrophotometer. The sample was scanned at 280 nm and the absorbance at 280 nm was used to determine the concentration of the protein. An experimentally derived extinction coefficient of 1.19 (mg/mL)⁻¹ cm⁻¹ and 1.38 (mg/mL)⁻¹ cm⁻¹ (for D liq and D lyo) at 280 nm was used to determine the protein concentration.

### Size Exclusion HPLC

The presence of monomers, dimers and high molecular mass species (HMMS) is monitored using size exclusion chromatography (SEC). The size exclusion chromatography was carried out using Waters Biosuite 450 SEC column, mobile phase 50 mM sodium phosphate, 75 mM NaCl, 0.01% sodium azide, pH 7.4, a flow rate of 0.3 mL/min, and UV detection at 214 nm. The levels of monomers, dimers and HMMS were calculated by integrating the area under the chromatogram peaks for each formulation and reporting the integrated areas of monomer, dimer and high molecular mass species peaks as a percentage of total peak area.

### Anion Exchange HPLC

The anion exchange chromatography was carried out using a TSKgel DEAE-5PW column, with mobile phase 10 mM Tris, pH 7.3, and 10 mM Tris, 0.5 M NaCl, pH 7.3 gradient elution, a flow rate of 1 mL/min, and UV detection at 280 nm. Anion exchange chromatography separates CDX-110 from KLH, peptide, linker, and related compounds based on their partitioning between a charged stationary phase and a mobile phase of varying ionic strength. The species of interest are detected by ultraviolet absorbance and quantitated by relative area.

### Reverse Phase HPLC

Reverse phase chromatography was carried out using a Waters symmetry 300 C4 column, with mobile phase 0.1% TFA water, and 0.1% TFA in acetronitrile gradient elution, a flow rate of 1 mL/min, and UV detection at 215 nm. Reversed phase chromatography separates CDX-110 from peptide and linker-related impurities on their partitioning between a nonpolar stationary phase and a polar mobile phase. Peaks are detected using an ultraviolet light detector at a fixed wavelength where the peptide and linker based impurities have absorption.

### Results

### Appearance

From appearance analysis, it could be concluded that sucrose and trehalose formulations behaved similarly in both the liquid and lyophilized formulations. Formulation F (lyo, PBS) behaved the worst. Further details are noted below.

### Liquid formulations (Table 2D)

All TO formulations were reported to be colorless. In terms of particulates, 1 white flake particle was observed for formulation D (liq, KPi, Tre) whereas formulation C (liq, KPi, Tre), which essentially only differs in trehalose content by 6 mg/mL, is EFVP. Current formulation F (liq, PBS) is also colorless and EFVP. At 5°C (T=12 weeks), all formulations were colorless and NMO RSI. One particle was observed for the B (liq, NaPi, Suc) and C (liq, KPi, Tre) formulations whereas formulation F (liq, PBS) remained EFVP.

At 25°C (T=12 weeks), formulation D (liq, KPi, Tre - NMO RSI) was reported to be very slightly brown. Formulation F (liq, PBS) was reported to have a number of different types of particles while D (liq, KPi, Tre) had one particle. At 40°C (T=12 weeks), all formulations changed color to yellow or brown except formulation F (liq, PBS). The trehalose formulations C and D were reported to be very slightly brown with one or no particles. Formulation F (liq, PBS) was colorless with two white flake-like particles. Formulation F (liq, PBS) was the only formualtion without PS 80.

### Lyophilized formulations (Table 2D)

All formulations at T=0 were colorless. A (lyo, KPi, Suc) was also reported to have one particle. At 5°C (T=12 weeks) all formulations were colorless and EFVP. At 25°C (T=12 weeks) all formulations were colorless and NMO RSI. Two small white particles were reported for C (lyo, KPi, Tre). At 40°C (T=12 weeks), all formulations were colorless and NMO RSI. One and two particles were observed in B (lyo, NaPi, Suc) and D (lyo, KPi, Tre) respectively.

### Concentration

### Liquid formulations (Table 2E)

At 5°C (T=12 weeks) no significant change was observed. At 25°C (T=12 weeks), all formulations did not show any significant change. At 40°C, formulations C (liq, KPi, Tre), D (liq, KPi, Tre) and F (liq, PBS) did not show any significant change while A (liq, KPi, Suc) and B (liq, NaPi, Suc) showed a marked increase in concentration. This demonstrated that the trehalose formulation behaved better than the sucrose formulation.

### Lyophilized formulations (Table 2E):

At 5°C (T=12 weeks) no significant changes were observed. At 25°C (T=12 weeks) no significant changes except for formulation F (lyo, PBS). At 40°C (T=12 weeks) no significant changes except for formulation F (lyo, PBS). A slight increase was observed for A (lyo, KPi, Suc).

### A280/A340 Ratio

### Liquid formulations (Table 2F)

At 5°C (T=12 weeks) there are no significant changes in the ratio except for sucrose formulations (A and B) where an increase is observed. At 25°C (T=12 weeks) all liquid formulations show an increase in the ratio indicating loss/changes to the copper molecule. Some of the formulations showed a greater change in ratio compared to others e.g., formulations C (liq, KPi, Tre), D (liq, KPi, Tre) and F (liq, PBS) had a more significant effect than A (liq, KPi, Suc) and B (liq, NaPi, Tre). At 40°C, the ratio of the sucrose containing formulations A (liq, KPi, Suc) and B (liq, NaPi, Suc) appear to be least affected (Table 2F) than the other formulations. The concentration data (Table 2E) shows that formulations A (liq, KPi, Suc) and B (liq, NaPi, Suc) had significantly high concentration (or high absorption values at 280 nm) values at 40°C. In addition, the absorbance at 340 nm also increases with time for the sucrose containing formulations, thus resulting in little change in the A280/A340 ratio. For the trehalose containing formulations C (liq, KPi, Tre), D (liq, KPi, Tre) and the current PBS formulation F (liq, PBS), this phenomenon (i.e. no increase in A280 with time) is not observed, leading to an increase in the A280/A340 ratio, largely due to the decrease in absorbance at 340 nm.

### Lyophilized formulations (Table 2F)

No significant changes were observed in any of the lyophilized formulations except for formulation F (lyo, PBS).

### Dimer/Monomer/HMMS Content

### Liquid formulations (Table 2G, 2H, and 2I)

Dimer content (Table 2G) decreases for all formulations with increasing temperature. The smallest decrease was observed for the trehalose containing formulations C (liq, KPi, Tre) and D (liq, KPi, Tre). Monomer content increased for all disaccharide containing formulations whereas the current formulation F (liq, PBS) showed a decrease. The PBS formulation showed a marked decrease in monomer content (Table 2H) at 25 and 40°C with a simultaneous increase in the HMMS content (Table 21). An exact opposite trend was observed for the sucrose (A and B) and trehalose (C and D) containing formulations where an increased formation of monomer with simultaneous decrease in HMMS and dimer content was observed at higher temperatures. At 25°C, both sucrose (A and B) and trehalose (C and D) formulations behaved similarly. At 40°C the trehalose formulations (C and D) showed significantly smaller changes in dimer, monomer and HMMS content compared to the sucrose (A and B) formulations at 40°C. This demonstrated that at 40°C trehalose formulations are relatively more stable than the sucrose containing formulations.

### Lyophilized formulations (Table 2G, 2H and 2I)

All lyophilized formulations showed no/minimal changes at 5, 25 and 40°C at the end of 12 weeks except for lyophilized PBS (F) formulation.

### Percent Impurity and Shift in Retention Time by AEX

### Liquid formulations (Table 2 J and 2K)

At 5°C (T=12 weeks) the percent impurity for both sucrose and trehalose formulations was less than or equal to 1 %. Retention time for the CDX-110 peak moved by approximately one minute for all formulations. The movement in retention time indicated formation of acidic species which has been attributed to a combination of linker hydrolysis and deamidation. At 25°C and 40°C (T=12 weeks), percent impurity increased significantly for all formulations. Change in retention time also followed a similar trend indicating increased formation of acidic species. Retention time shifts for trehalose containing formulations was less compared to the sucrose formulations indicating less formation of acidic species in the trehalose formulations.

### Lyophilized formulations (Table 2J and K)

For all lyophilized formulations, the percent impurity change at all temperatures was not significant. A similar trend was observed for retention time (<1 minutes) for all formulations.

### Percent Impurity by RPHPLC

### Liquid formulation (Table 2L)

An increased formation of peptide and linker related impurities were observed with increasing temperature for both sucrose and trehalose formulations. The trehalose formulation showed less formation of impurities than the sucrose formulations.

### Lyophilized formulations (Table 2L)

No significant change was observed for any of the formulations except for the lyophilized PBS (F) formulation.

### Mouse potency

All liquid and lyophilized formulations (5°C samples) tested met the drug product specification of ≥ 75% seroconversion in mice (Table 2M).

**Table 2D: Appearance Data**

| **Formulation** | **Appearance at T=0 wk** | **Appearance at 5C T=12 wk** | **Appearance at 25C T=12 wk** | **Appearance at 40C T=12 wk** |
|---|---|---|---|---|
| A (liq) KPO₄, Suc-80 mg/ml, PS80, pH 7.4, **liq** | NMO RSI Colorless (<B9) EFVP | NMO RSI Colorless (<B9) EFVP | NMO RSI Colorless (<B9) EFVP | NMO RSI SI brown yellow (<BY6) 1 small white round particle |
| A (lyo) KPO₄, Suc-80 mg/ml, PS80, pH 7.4, **lyo** | NMO RSI Colorless (<B9) 1 short white silverlike particle | NMO RSI Colorless (<B9) EFVP | NMO RSI Colorless (<B9) EFVP | NMO RSI Colorless (<B9) EFVP |
| B (liq) NaPO₄, Suc-80 mg/ml, PS80, pH 7.4, **liq** | NMO RSI Colorless (<B9) EFVP | NMO RSI Colorless (<B9) 1 white, 1 tan short fiberlike Particle | NMO RSI Colorless (<B9) EFVP | NMO RSI SI Brown Yellow (≤BY6) EFVP |
| B (lyo) NaPO₄, Suc-80 mg/ml, PS80, pH 7.4, **lyo** | NMO RSI Colorless (< B9) EFVP | NMO RSI Colorless (<B9) EFVP | NMO RSI Colorless (<B9) EFVP | NMO RSI Colorless (<B9) 1 short white fiberlike particle |
| C (liq) KPO₄, Tre-84 mg/ml, PS80, pH 7.4, **liq** | NMO RSI Colorless (<B9) EFVP | NMO RSI Colorless (<B9) 1 shortwhite fiberlike particle, and 3 small white round particles | NMO RSI Colorless (<B9) EFVP | NMO RSI SI brown yellow (<BY7) 1 small white round particle |
| C (lyo) KPO₄, Tre-84 mg/ml, PS80, pH 7.4, **lyo** | NMO RSI Colorless (<B9) EFVP | NMO RSI Colorless (<B9) EFVP | NMO RSI Colorless (<B9) 2 small white round particle | NMO RSI Colorless (<B9) EFVP |
| D (liq) KPO₄, Tre-90 mg/ml, PS80, pH 7.4, **liq** | NMO RSI Colorless (<B9) 1 white flake like particle | NMO RSI Colorless (<B9) EFVP | NMO RSI v slight brown (<B7) 1small white round particle | NMO RSI v slight brown (<B7) EFVP |
| D (lyo) KPO₄, Tre-90 mg/ml, PS80, pH 7.4, **lyo** | NMO RSI Colorless (<B9) EFVP | NMO RSI Colorless (<B9) EFVP | NMO RSI Colorless (<B9) EFVP | NMO RSI Colorless (<B9) 2 small white round particles |
| F (liq) PBS, pH 7.4, **liq** | NMO RSI Colorless (<B9) EFVP | NMO RSI Colorless (<B9) EFVP | NMO RSI Colorless (<B9) ∼5 short white fiber like particles; ∼5small white globular particles; ∼6 small white flake like particles | NMO RSI Colorless (<B9) 2 white flake like particles |
| F (lyo) PBS, pH 7.4, **lyo** | NMO RSI Slightly brown (<B6) Many (∼15) short brown /tan fibers. Typical of cardboard packaging. 2 short white silverlike particles | NMO RSII Very Slightly Brown (≤B7) ∼10 tan short fiberlike and 3 white globular particles (floc) | NMO RSIII Very Slightly Brown (≤B7) 2 short white fiberlike and 4 white globular particles (floc) | NMO RSIII Very Slightly Brown (≤B7) 3 tan short fiberlike, 2 small white round, 5 white globular particles (flocs) |

| | | | | |
|---|---|---|---|---|
| EFVP: Essentially free of visible particles; NMO RS I/II/III: Not more opalescent than reference suspension I/II/III. | | | | |

**Table 2E: Change in Concentration Data**

| **Formulation** | **Change in concentration (mg/mL) at 5C in 12 wks** | **Change in concentration (mg/mL) at 25C in 12 wks** | **Change in concentration (mg/mL) at 40C in 12 wks** |
|---|---|---|---|
| A (liq) KPO₄, Suc-80 mg/ml, PS80, pH 7.4, **liq** | 0.14 | 0.04 | 0.35 |
| A (lyo) KPO₄, Suc-80 mg/ml, PS80, pH 7.4, **lyo** | 0.08 | 0.03 | 0.13 |
| B (liq) NaPO₄, Suc-80 mg/ml, PS80, pH 7.4, **liq** | -0.04 | 0.05 | 0.31 |
| B (lyo) NaPO₄, Suc-80 mg/ml, PS80, pH 7.4, **lyo** | 0.08 | -0.01 | -0.03 |
| C (liq) KPO₄, Tre-84 mg/ml, PS80, pH 7.4, **liq** | 0.01 | 0.03 | 0.07 |
| C (lyo) KPO₄, Tre-84 mg/ml, PS80, pH 7.4, **lyo** | 0.03 | 0.02 | 0.09 |
| D (liq) KPO₄, Tre-90 mg/ml, PS80, pH 7.4, **liq** | -0.01 | 0.00 | 0.02 |
| D (lyo) KPO₄, Tre-90 mg/ml, PS80, pH 7.4, **lyo** | 0.02 | 0.04 | 0.02 |
| F (liq) PBS, pH 7.4, **liq** | 0.02 | 0.03 | 0.05 |
| F (lyo) PBS, pH 7.4, **lyo** | -0.09 | -0.12 | -0.45 |

**Table 2F: Change in A280/A340 Ratio**

| **Formulation** | **Change in A280/A340 at 5C in 12 wks** | **Change in A280/A340 at 25C in 12 wks** | **Change in A280/A340 at 40C in 12 wks** |
|---|---|---|---|
| A (liq) KPO₄, Suc-80 mg/ml, PS80, pH 7.4, **liq** | 0.00 | 0.45 | 0.00 |
| A (lyo) KPO₄, Suc-80 mg/ml, PS80, pH 7.4, **lyo** | -0.02 | 0.01 | 0.12 |
| B (liq) NaPO₄, Suc-80 mg/ml, PS80, pH 7.4, **liq** | 0.21 | 0.45 | 0.21 |
| B (lyo) NaPO₄, Suc-80 mg/ml, PS80, pH 7.4, **lyo** | -0.02 | -0.06 | 0.06 |
| C (liq0 | 0.01 | 2.79 | 5.65 |
| KPO₄, Tre-84 mg/ml, PS80, pH 7.4, **liq** | | | |
| C (lyo) KPO₄, Tre-84 mg/ml, PS80, pH 7.4, **lyo** | 0.09 | -0.03 | 0.33 |
| D (liq) KPO₄, Tre-90 mg/ml, PS80, pH 7.4, **liq** | -0.01 | 2.59 | 5.45 |
| D (lyo) KPO₄, Tre-90 mg/ml, PS80, pH 7.4, **lyo** | 0.13 | 0.00 | 0.20 |
| F (liq) PBS, pH 7.4, **liq** | 0.02 | 2.59 | 7.95 |
| F (lyo) PBS, pH 7.4, **lyo** | 0.34 | -0.03 | 0.98 |

**Table 2G: Change in Percent Dimer Data**

| **Formulation** | **Change in dimer % at 5C in 12 wks** | **Change in dimer % at 25C in 12 wks** | **Change in dimer % at 40C in 12 wks** |
|---|---|---|---|
| A (liq) KPO₄, Suc-80 mg/ml, PS80, pH 7.4, **liq** | -0.12 | -4.08 | -41.98 |
| A (lyo) KPO₄, Suc-80 mg/ml, PS80, pH 7.4, **lyo** | 1.50 | 1.50 | 0.70 |
| B (Iiq) NaPO₄, Suc-80 mg/ml, PS80, pH 7.4, **liq** | -0.13 | -4.93 | -45.73 |
| B (lyo) NaPO₄, Suc-80 mg/ml, PS80, pH 7.4, **lyo** | 1.20 | -0.10 | -0.80 |
| C (liq) KPO₄, Tre-84 mg/ml, PS80, pH 7.4, **liq** | 0.19 | -4.41 | -14.61 |
| C (lyo) KPO₄, Tre-84 mg/ml, PS80, pH 7.4, **lyo** | 1.70 | 1.10 | -0.30 |
| D (liq) KPO₄, Tre-90 mg/ml, PS80, pH 7.4, **liq** | -1.20 | -4.20 | -18.40 |
| D (lyo) KPO₄, Tre-90 mg/ml, PS80, pH 7.4, **lyo** | -0.90 | -0.70 | -0.80 |
| F (liq) PBS, pH 7.4, **liq** | -0.35 | -2.35 | -23.65 |
| F (lyo) PBS, pH 7.4, **lyo** | -0.10 | -15.60 | -30.60 |

**Table 2H: Change in Percent Monomer Data**

| **Formulation** | **Change in % monomer at 5C in 12 wks** | **Change in % monomer at 25C in 12 wks** | **Change in % monomer at 40C in 12 wks** |
|---|---|---|---|
| A (liq) KPO₄, Suc-80 mg/ml, PS80, pH 7.4, **liq** | 0.24 | 4.64 | 26.94 |
| A (lyo) KPO₄, Suc-80 mg/ml, PS80, pH 7.4, **lyo** | -0.20 | -0.70 | -0.20 |
| B (liq) NaPO₄, Suc-80 mg/ml, PS80, pH 7.4, **liq** | -0.92 | 5.52 | 35.32 |
| B (lyo) NaPO₄, Suc-80 mg/ml, PS80, pH 7.4, **lyo** | -0.30 | -0.20 | -0.30 |
| C (liq) KPO₄, Tre-84 mg/ml, PS80, pH 7.4, **liq** | 0.2 | 4.20 | 8.30 |
| C (lyo) KPO₄, Tre-84 mg/ml, PS80, pH 7.4, **lyo** | -0.80 | -0.60 | -0.20 |
| D (liq) KPO₄, Tre-90 mg/ml, PS80, pH 7.4, **liq** | 0.70 | 4.10 | 11.30 |
| D (lyo) KPO₄, Tre-90 mg/ml, PS80, pH 7.4, **lyo** | -0.10 | 0.00 | 0.30 |
| F (liq) PBS, pH 7.4, **liq** | 0.78 | 2.28 | -3.02 |
| F (lyo) PBS, pH 7.4, **lyo** | 0.80 | -2.00 | -2.70 |

**Table 2I: Change in Percent HMMS Data**

| **Formulation** | **Change in HMMS % at 5C in 12 wks** | **Change in HMMS % at 25C in 12 wks** | **Change in HMMS % at 40C in 12 wks** |
|---|---|---|---|
| A (liq) KPO₄, Suc-80 mg/ml, PS80, pH 7.4, **liq** | -0.33 | -2.63 | -12.53 |
| A (lyo) KPO₄, Suc-80 mg/ml, PS80, pH 7.4, **lyo** | -0.30 | -0.50 | -0.40 |
| B (liq) NaPO₄, Suc-80 mg/ml, PS80, pH 7.4, **liq** | -1.27 | -2.97 | -12.97 |
| B (lyo) NaPO₄, Suc-80 mg/ml, PS80, pH 7.4, **lyo** | 0.10 | 0.80 | 1.10 |
| C (liq) KPO₄, Tre-84 mg/ml, PS80, pH 7.4, **liq** | -0.16 | -2.36 | -6.66 |
| C (lyo) KPO₄, Tre-84 mg/ml, PS80, pH 7.4, **lyo** | -0.6 | -0.40 | 0.60 |
| D (liq) | -0.10 | -1.70 | -4.30 |
| KPO₄, Tre-90 mg/ml, PS80, pH 7.4, **liq** | | | |
| D (lyo) KPO₄, Tre-90 mg/ml, PS80, pH 7.4, **lyo** | 0.20 | -0.10 | -0.20 |
| F (liq) PBS, pH 7.4, **liq** | -0.11 | -1.21 | 21.39 |
| F (lyo) PBS, pH 7.4, **lyo** | -0.30 | 18.20 | 33.30 |

**Table 2J: Change in Percent Impurities by AEX Data**

| **Formulation** | **Change in AEX % impurities at 5C in 12 wks** | **Change in AEX % impurities at 25C in 12 wks** | **Change in AEX % impurities at 40C in 12 wks** |
|---|---|---|---|
| A (liq) KPO₄, Suc-80 mg/ml, PS80, pH 7.4, **liq** | 0.30 | 1.20 | 1.60 |
| A (lyo) KPO₄, Suc-80 mg/ml, PS80, pH 7.4, **lyo** | 0.20 | 0.20 | 0.10 |
| B (liq) NaPO₄, Suc-80 mg/ml, PS80, pH 7.4, **liq** | 0.10 | 1.00 | 1.30 |
| B (lyo) NaPO₄, Suc-80 mg/ml, PS80, pH 7.4, **lyo** | 0.10 | 0.20 | 0.10 |
| C (liq) KPO₄, Tre-84 mg/ml, PS80, pH 7.4, **liq** | 0.10 | 0.9 | 2.60 |
| C (lyo) KPO₄, Tre-84 mg/ml, PS80, pH 7.4, **lyo** | 0.20 | 0.10 | 0 |
| D (liq) KPO₄, Tre-90 mg/ml, PS80, pH 7.4, **liq** | 1.00 | 2.70 | 3.50 |
| D (lyo) KPO₄, Tre-90 mg/ml, PS80, pH 7.4, **lyo** | 1.10 | 0.50 | 0.40 |
| F (liq) PBS, pH 7.4, **liq** | 0.90 | 1.90 | 3.30 |
| F (lyo) PBS, pH 7.4, **lyo** | 0.40 | 0.50 | 3.30 |

### Example 3: Filtration Process Development

### 3.1 Materials & Equipment

The following process materials and equipment were used during this study: AKTA Cross flow tangential flow filtration unit from GE Healthcare; 30K and 100K Hydrosart cassettes with 200 cm² membrane area (Sartorius # 308144590 2E-SG; 308144680 2E-SG); 30K Hydrosart cassettes with 0.1 m² membrane (Sartorius # 308144590 2E-SG); Keyhole limpet hemocyanin (KLH) purchased as Vacmune^{®} (Biosyn Corporation lots 739690V1, R625258, 817873V1, and 817873V2); EGFRvIII peptide (Ambiopharm Inc. lot APi 080616); EGFRvIII peptide (Chinese Peptide Corporation (CPC) lot # CH-0600453); Sulfosuccinimidyl 4-[N-maleimidomethyl]cyclohexane-1-carboxylate (Sulfo-SMCC) (Thermo, various lots, catalog # 22322); Dimethyl sulfoxide (DMSO), anhydrous 99.9% (Sigma Catalog # 276855); NaPh buffer = 100mM sodium phosphate pH 7.2; PBS = OmniPur 10x phosphate buffered saline diluted to 1x (VWR Catalog # EM6506); KPh buffer = 10mM potassium phosphate pH 7.4; PETG (polyethylene terephthalate copolyester) bottles (various sizes from Nalgene).

### 3.2 Methods

The overall process included: (1) an activation reaction between KLH and linker; (2) an initial TFF step to remove excess linker and linker-related impurities; (3) a conjugation reaction step between activated KLH and peptide; followed by (4) a second TFF step to remove excess peptide and peptide-related impurities and to change the buffer. An extinction coefficient of 1.38 for CDX-110 was used throughout this study. A more detailed description for each step follows below.

### 3.2.1 Activation Reaction Step

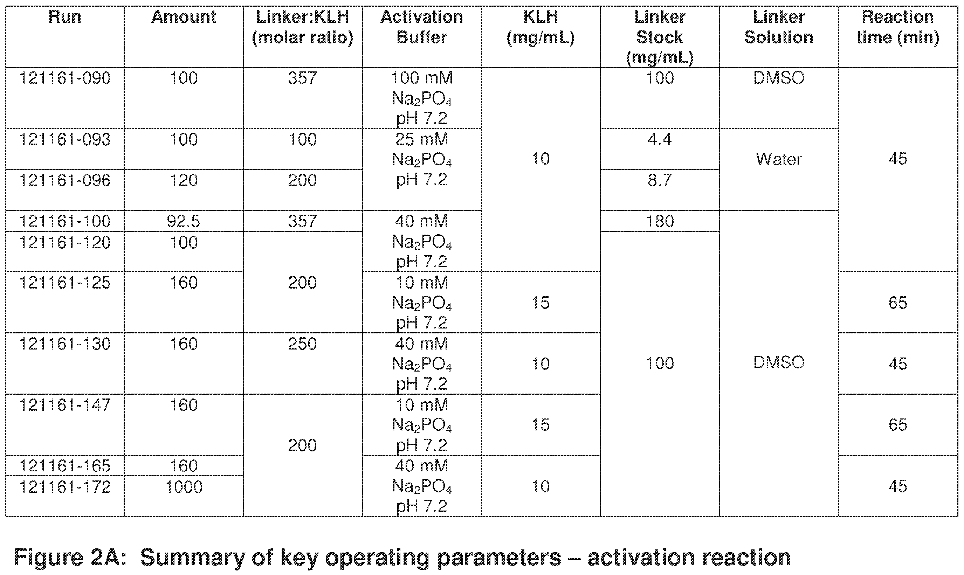

For most of the TFF runs, KLH (100-160 mg) was aliquoted into a 15 mL or 50 mL plastic tube, diluted with NaPh buffer and water for injection (WFI), and mixed by inversion of the tube several times. The linker solution (pre-dissolved either in water or DMSO) was added to the KLH mixture, gently mixed by inverting the tube several times, and then held stationary for the remainder of the 45 ± 5 minutes. For runs 121161-165 and 121161-172, PETG bottles were used as reaction vessels and the solution was vigorously mixed with a stir bar during linker addition and more gently mixed for the remainder of the reaction. Different activation reaction parameters such as linker:KLH molar ratio, buffer concentration, linker stock solution concentration, and reaction times were investigated as different process inputs into the TFF system. Complete details are provided in Figure 2A.

### 3.2.2 First TFF Operation

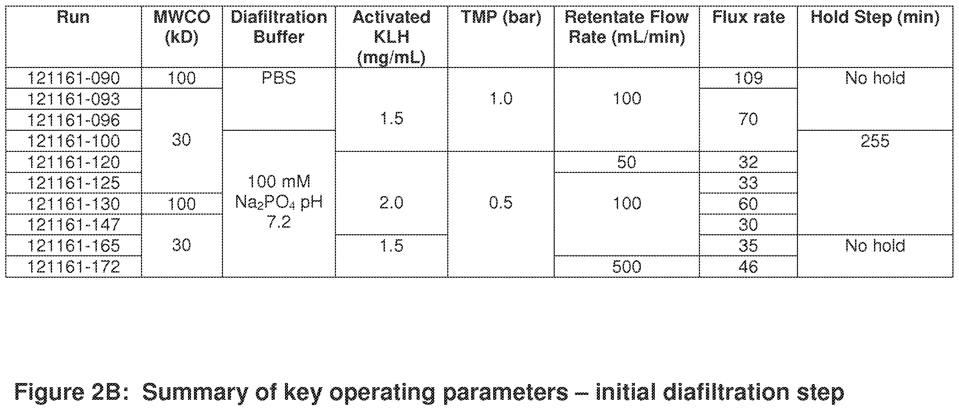

At the end of the activation reaction, the activated KLH solution was diluted with NaPh buffer to 1.5-2.0 mg/mL protein concentration prior to being transferred through a transfer pump into the cross-flow system. Membranes were pre-sanitized before use. During the development and optimization of the TFF operation, different parameters were investigated such as protein concentration during diafiltration, diafiltration buffer, membrane molecular weight cutoff (MWCO), transmembrane pressure (TMP), retentate flow rate, and the number of turn over diafiltration volumes (TOVs). Complete details are provided in Figure 2B.

### 3.2.3 Conjugation Step

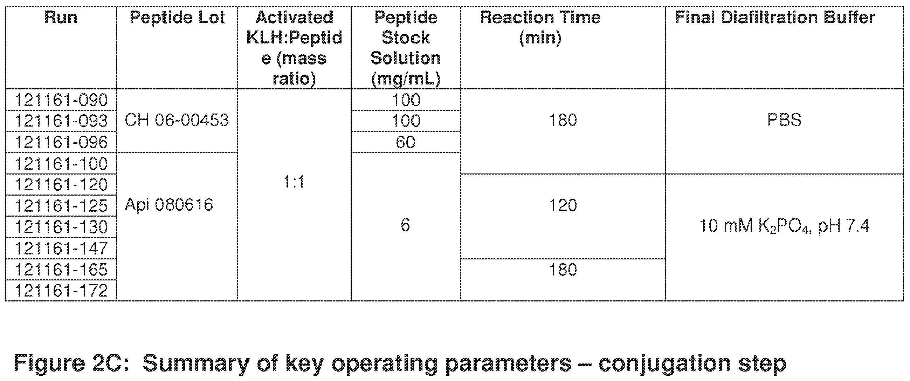

The diafiltered activated KLH solution processed in the TFF system was recirculated at 10 mL/min and either held for a total of 255 minutes (from start of activation reaction to the addition of peptide) or conjugated immediately with the peptide solution. Prior to the conjugation step, the protein solution in the reservoir was concentrated to 2 mg/mL, in order to reach the target concentration for the conjugation reaction step. For all conjugation reactions, the target concentration for both KLH and peptide was 1.5 mg/mL. The peptide stock solution was pre-dissolved in NaPh buffer and added in a single bolus directly into the TFF reservoir with a pipet. The KLH:peptide ratio was held constant at 1:1 (by mass ratio). Parameters investigated were peptide stock solution concentration and conjugation reaction time. During the reaction, the TFF system was set to recirculate at 0.5 L/min/m² with the permeate line closed and the retentate line wide open. Complete details are provided in Figure 2C.

### 3.2.4 Second TFF Operation

After completion of the conjugation reaction, a second diafiltration was carried out to buffer exchange the conjugated KLH and to remove excess peptide and peptide-related impurities. For this second diafiltration step, the same membrane and the same operating conditions as the first diafiltration were used. Upon completion of the second diafiltration, the retentate was removed from the TFF system and filtered using a 0.2 µm filter. A buffer rinse was performed across the membrane and collected separately.

### 3.2.5 Summary of Cross-Flow Experiments

Figures 2A-2C summarize the key operating parameters for the ten development TFF runs completed to develop the optimized process. Runs 121161-090, 121161-093, 121161-096, and 121161-100 were a set of initial runs to evaluate linker:KLH ratio, linker stock concentration and dissolution solvent, and peptide stock concentration. Additionally, the MWCO of the membrane was evaluated (30 kDa vs. 100 kDa).

The main objective of runs 121161-120, 121161-125, and 121161-130 was to determine TFF operating parameters such as TMP, retentate flow rate, and flux. Run 121161-147 was a repeat of 121161-125. The reasons for repeating 121161-125 are discussed further below. Runs 121161-165 and 121161-172 were demonstration runs of the process which was to be transferred to a pilot plant. Run 121161-165 was done using a 160 mg charge of KLH and run 121161-172 was done using a 1 g charge of KLH to assess the scalability of the optimized process.

### Example 4: Results and Discussion of Filtration Process Development

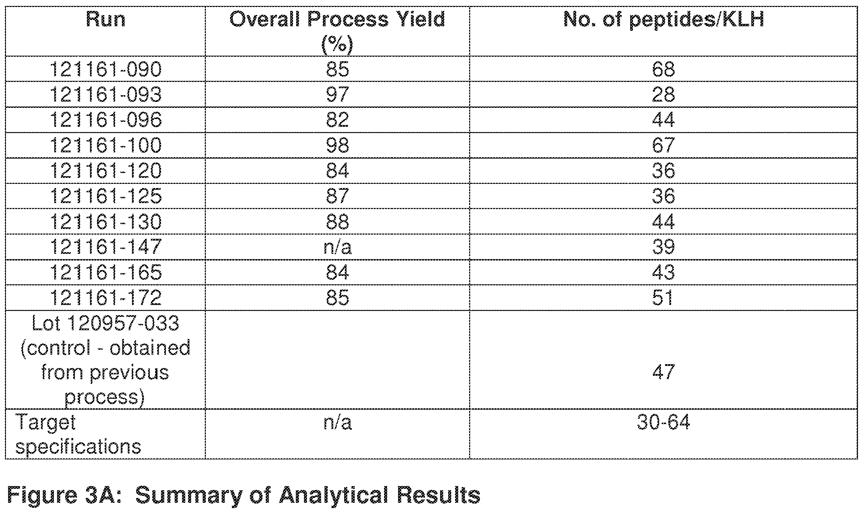

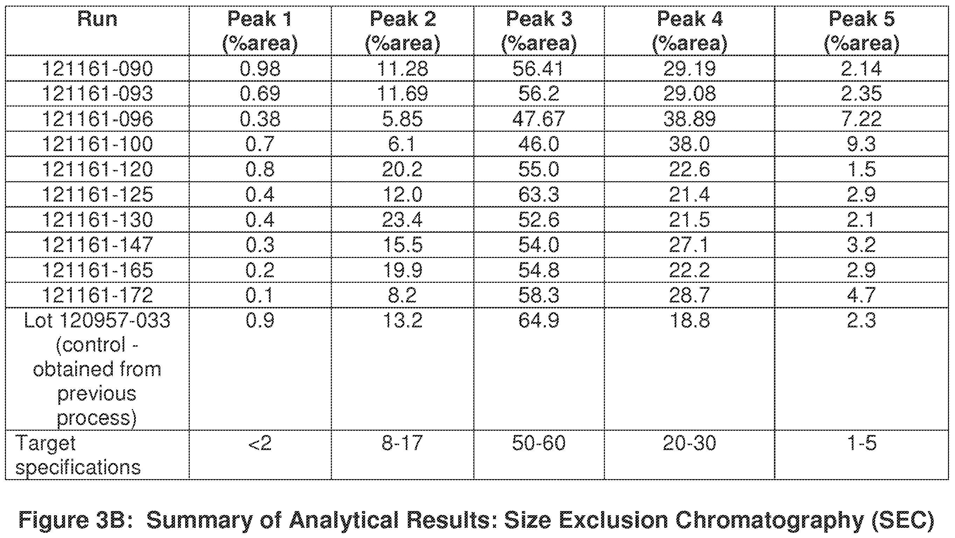

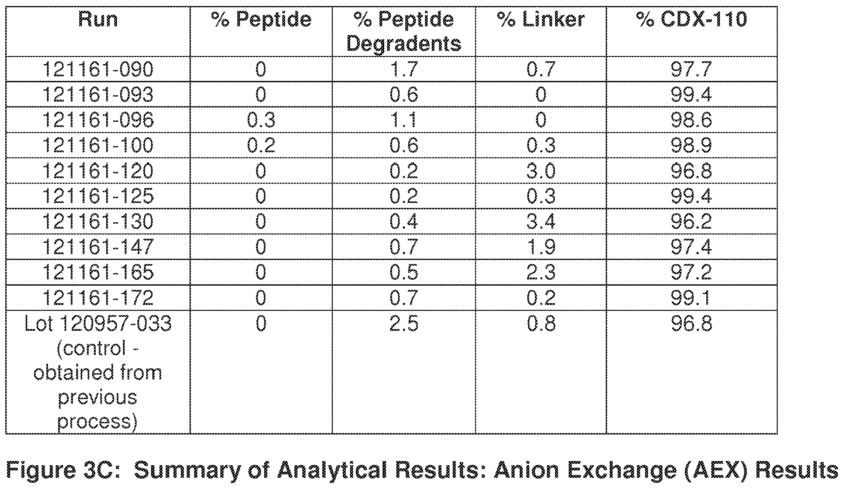

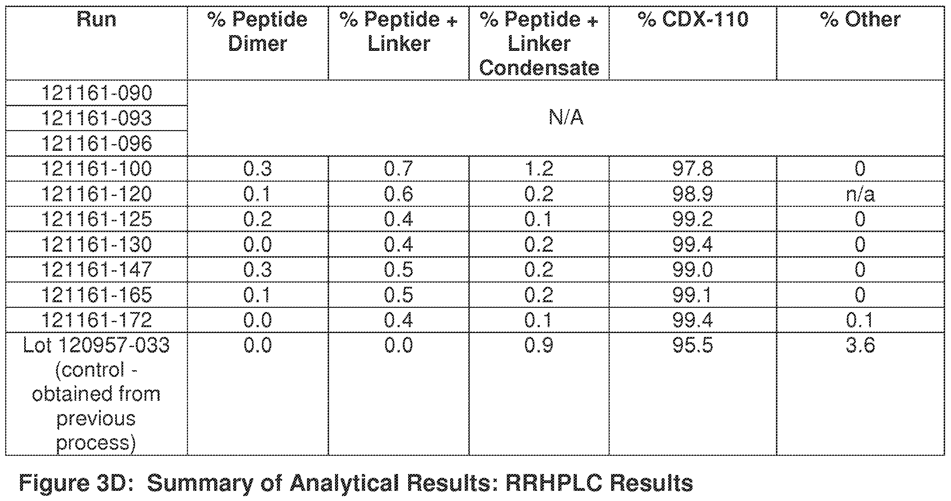

Key analytical results for all the process development runs are summarized in Figures 3A-3D. Final drug substance was used for all assays, except for epitope density which used the retentate recovered from the TFF system before addition of excipients. It was unclear whether PS80, which is one of the excipients added, would interfere with the epitope density assay. Target ranges were based on results from previous clinical lots (i.e. Lots 06-044-001 and 07-044-001, as shown below in Table 3A) taking into account the normal variations in the assays. Nearly all the runs produced material which was on target for all assays or were at least close to targeted ranges. The only exception was % linker as determined by the AEX assay which was significantly higher in some lots compared to others.

**Table 3A**

| **Lot** | **SEC Peaks** | | | | | **AEX purity** | **RP-HPLC purity** | **Epitope Density** |
|---|---|---|---|---|---|---|---|---|
| | 1 | 2 | 3 (dimer) | 4 (monomer) | 5 | | | |
| 06-044-001 | 6.51 | 15.86 | 58.86 | 16.85 | 1.93 | 94.9 | 93.1 | 50 |
| 07-044-001 | 1.93 | 10.42 | 54.3 | 30.44 | 2.9 | 94.2 | 92 | 36 |

### Runs 121161-090, -093, -096, and -100

From runs 121161-090, -093, -096, and -100, several decisions could be made regarding the activation reaction. First, a decision was made after 121161-090 to switch from a 100 kD to a 30 kD membrane. This decision was based on possible loss of activated KLH through the membrane. RP-HPLC analysis of the permeate seemed to show some leakage of activated KLH; however, later work determined that the assay was prone to carry-over under certain conditions. The activated KLH seen in the permeate was most likely due to carry-over from previous runs on the column and not due to leakage through the membrane.

Second, the decision was made to incorporate DMSO into the process to provide complete dissolution of the linker, thereby having more control over linker:KLH ratios. From these runs, it was determined that DMSO did not have a detrimental effect on product qualtiy. The appropriate SEC profile was maintained and epitope density was close to the target range; however, the original 357:1 linker:KLH ratio (121161-090 & 121161-100) resulted in an epitope density which was just above the desired range and 100:1 resulted in too low of an epitope density (121161-093). Data from these runs and additional data from small-scale activation experiments led to the decision to set the linker:KLH ratio at 200:1.

A decision was also made to set the linker stock solution at 100 mg/mL. The desire was to keep the concentration high to minimize the amount of DMSO added to the reaction, but at a concentration where linker dissolved quickly and easily. Run 121161-100 used a linker stock of 180 mg/mL, but dissolution of the linker was not as quick as with 100 mg/mL.

The concentration of the peptide stock solution was set at 6 mg/mL. This stock concentration provided the appropriate volume of peptide solution to be added to the conjugation reaction which results in the desired peptide and activated KLH concentrations of 1.5-2.0 mg/mL without the need for further buffer addition. The final decision based upon these four runs was to set the NaPh buffer at 40 mM in the activation reaction.

### Runs 121161-120, -125, -130, and -147

The purpose of the next set of runs was to determine the optimal values for TFF parameters such as TMP, retentate flow rate, and flux. By this time, formulation nomination had occurred and as a result, the second diafiltration buffer was changed for all runs going forward to KPh buffer. Linker:KLH ratio was set at 200:1 and linker was dissolved at 100 mg/mL in DMSO. Run 121161-120 was set up to explore the lower range of TMP and retentate flow rate. Assay results indicated that the material produced fulfilled all target specifications, except the % linker as measured by AEX was significantly higher than seen previously. This increase in linker was orginally thought to be due to a decrease in retentate flow rate (50 vs. 100 mL/min) between the initial set of four runs and 121161-120.

Run 121161-125 was set up to address the hypothesis that a decrease in retentate flow rate was the cause of the decrease in linker clearance. The SEC profile showed that % dimer was slightly out of the target range and epitope density, at 36 peptides/KLH, was close to the low end of the target range (30-64 peptides/KLH); however, linker clearance improved significantly (0.3% vs. 3.0% from 121161-120.

For run 121161-130, a decision was made to try higher MWCO membrane (100 kD) to try to improve impurity clearance. At the same time, linker:KLH ratio was increased to 250:1 to increase the epitope density resulting in a peptide/KLH number more towards the middle of the targeted range. The resulting material fulfilled all analytical targets, except for % linker which had increased to 3.4%. Since two parameters had been changed for this run (membrane MWCO and linker:KLH ratio) it was unclear what had caused the % linker to increase compared to run 121161-125. A decision was made to return to using a 30 kD MWCO membrane since run 121161-130 had shown no advantage to using a 100 kD membrane.

To address questions regarding the normal level of lot-to-lot variation in % linker, a decision was made to repeat run 121161-125 as closely as possible to determine if the low level of % linker could be achieved again. The results from this run showed that although the level of linker had decreased to 1.9%, it still wasn't as low as earlier runs (< 1%). To assess the true impact of these levels of linker impurities, calculations were performed to determine a correlation between % by area and actual ppm. What was determined was that the levels of % linker by area seen in the process development runs corresponded to < 3 ppm; thus, the actual amount of the linker impurities by mass is actually quite small and at an acceptable level.

### Runs 121161-165 and -172

Run 121161-165 was a lab-scale demonstration run using a 160 mg charge of KLH to test the acceptability of material made by the optimized parameters determined during process development. These parameters are summarized in Figures 2A-2C and the analytical results shown in Figures 3A-3D. To build further confidence in the optimized process, a larger-scale lab run was carried out. This run (121161-172) used a 1 g charge of KLH. The TFF parameters are summarized in Figures 2A-2C and the analytical results in Figures 3A-3D. Parameters for scaling up the process to 1 g were based on keeping the flux constant (see Table 3B below). Also, in Table 3B are the scale-up parameters which can be used for running the process at a 10 g scale.

**Table 3B**

| **Parameter** | **Units** | **Bench Scale** | **1 gram scale-up run** | **JJ Clinical Run** |
|---|---|---|---|---|
| Mass of KLH | g | 0.16 | 1.0 | 10 |
| Volume of KLH | Liter | 0.107 | 0.67 | 6.7 |
| Membrane Area | m² | 0.02 | 0.1 | 1.2 |
| Volume/Area | Liter/m² | 5.35 | 6.7 | 5.6 |
| Mass/Area | g/m² | 8.0 | 10.0 | 8.3 |
| Cross flowrate | L/min | 0.1 | 0.5 | 6 |
| Cross flowrate per unit area | L/min/m² | 5 | 5 | 5 |

### Example 5: Reaction Parameter Process Development

### 5.1 Materials and Methods

The following process materials and equipment were used during this study: Keyhole limpet hemocyanin (KLH) purchased as Vacmune® (Biosyn Corporation lots 739690V1, R625258, 817873V1, and 817873V2); EGFRvIII peptide (Ambiopharm Inc. lot APi 080616); EGFRvIII peptide (Chinese Peptide Corporation (CPC) lot # CH-0600453); Sulfosuccinimidyl 4-[N-maleimidomethyl]cyclohexane-1-carboxylate (Sulfo-SMCC) (Thermo, various lots, catalog # 22322); Dimethyl sulfoxide (DMSO), anhydrous 99.9% (Sigma catalog # 276855 or equivalent); NaPh buffer = 100mM sodium phosphate pH 7.2; PBS = Gibco 1x PBS, pH 7.2 (Gibco, catalog # 20012), or Dulbeccos 1x PBS (Gibco, catalog # 14190), or OmniPur 10x phosphate buffered saline diluted to 1x (VWR catalog # EM6506); 2 mL glass HPLC vials for reactions < 2mL (Agilent cat. no 5182-0715 or equivalent); 15 mL corning polypropylene tubes for reactions ≥ 2 mL; Amicon 50K and 100K Centrifugal Filters (Millipore, cat no. UFC805906 and UFC810096) or equivalent; Zeba Desalt Spin Columns, 0.5 ml (Thermo, catalog # 89883); Benchtop centrifuge.

### 5.2 Methods

The optimized manufacturing process includes: 1) an activation reaction which covalently links the amine groups of the KLH protein with the NHS-ester group of the Sulfo-SMCC linker to form activated KLH; 2) an initial TFF step to remove excess linker and linker-related impurities; 3) a conjugation reaction which covalently attaches the C-terminal cysteine of the synthetic EGFRvIII peptide with the maleimide group of the Sulfo-SMCC linker which is part of the activated KLH molecule forming conjugated CDX-110, followed by; 4) a second TFF step to remove excess peptide and peptide-related impurities and to exchange the buffer. This report describes the optimization of reaction conditions using a scaled-down system called a one-pot reaction. An extinction coefficient of 1.30 for KLH and activated KLH and 1.38 for CDX-110 was used throughout this study.

### 5.2.1 One-Pot Reaction

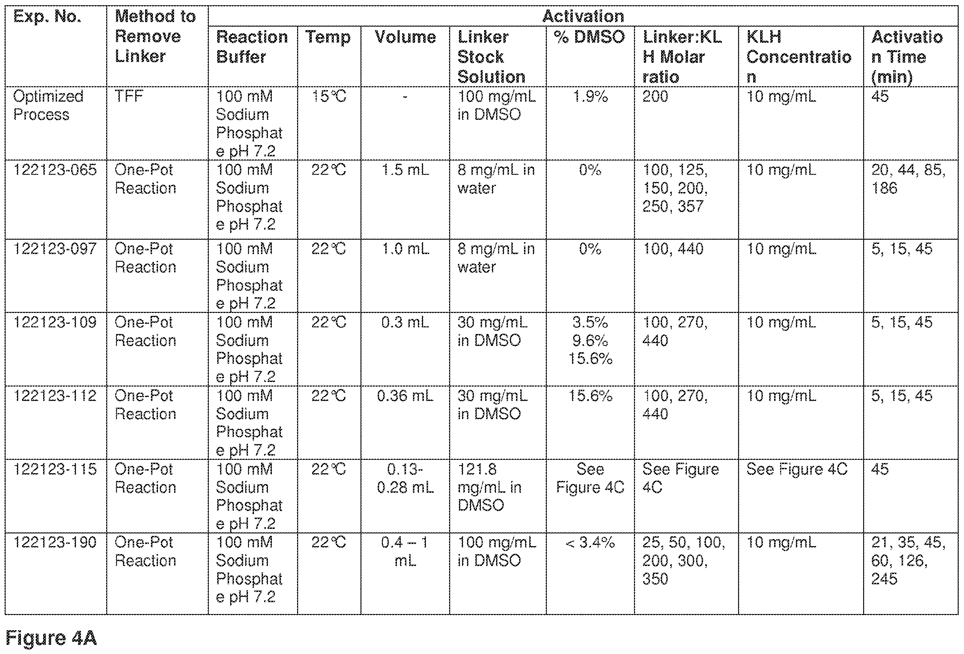

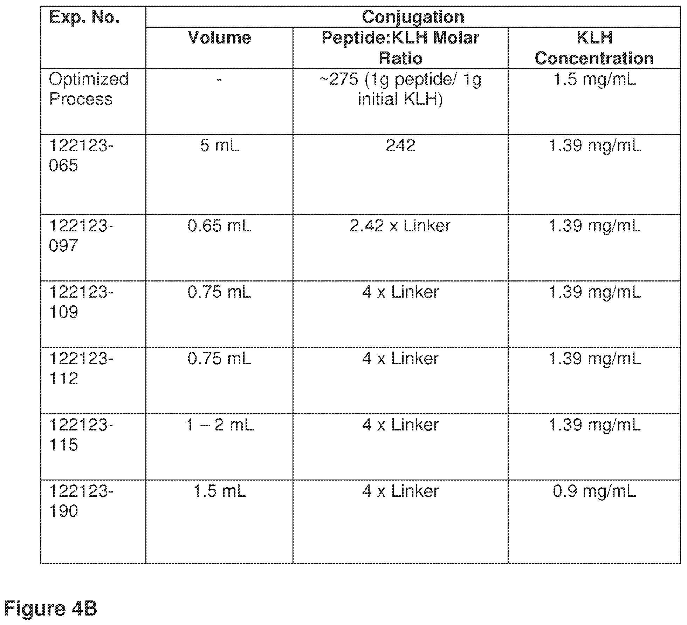

KLH was aliquoted into a 2 mL glass HPLC vial or a 15 mL polypropylene tube, diluted with NaPh buffer, and mixed gently by inversion. The linker solution (pre-dissolved either in water or DMSO) was added to the KLH mixture, vortexed briefly (15-30 sec), and then held for 45 ± 5 minutes on an orbital mixer. After 45 minutes, conjugation was started by quenching the reaction with peptide solution at a ratio of 4:1 peptide:linker and briefly vortexing (15-30 seconds). The conjugation reaction was allowed to react for ∼2 to 3 hours on an orbital mixer and then held overnight on the mixer or in a refrigerator prior to buffer exchange. Peptide and peptide-related impurities were removed by diafiltration with a Millipore Amicon centrifugal filter prior to epitope density analysis. Reaction parameters were adjusted as needed to investigate the desired variables such as linker:protein ratio, activation time, protein concentration, and percent DMSO. Each experiment is briefly described in the next section. Figures 4A-4C contain a summary of the reaction parameters that were used for each experiment.

### 5.2.2 Experiment No. 122123-065

KLH was activated with sulfo-SMCC at linker:KLH molar ratios varying between 100:1 and 357:1 for -45 minutes. Sulfo-SMCC linker was dissolved in DMSO at 8 mg/mL prior to addition. Peptide at a molar ratio of 242:1 (peptide:KLH) was immediately added to the reaction at the end of the activation. The peptide:KLH ratio was held at 242:1 and may not have been in an excess in the higher linker ratio experiments. An increased peptide:linker ratio was used in later experiments.

### 5.2.3 Experiment Nos.122123-097, 122123-109, 122123-112

KLH was activated with sulfo-SMCC at linker:protein molar ratios varying between 100:1 and 440:1 for -45 minutes. Sulfo-SMCC linker was dissolved in either water at 8 mg/mL or DMSO at 30 mg/mL prior to addition. DMSO was not present in experiment 122123-097. DMSO varied between 3.5% and 15.6% for experiment 122123-109, and was held constant at 15.6% for experiment 122123-112. Peptide at a molar ratio of 2.42:1 peptide:linker was immediately added to the reaction at the end of the activation for experiment 122123-097. Although the results were acceptable, the peptide:linker molar ratio was increased to 4:1 for later experiments to ensure peptide was in excess.

### 5.2.4 Experiment No. 122123-115

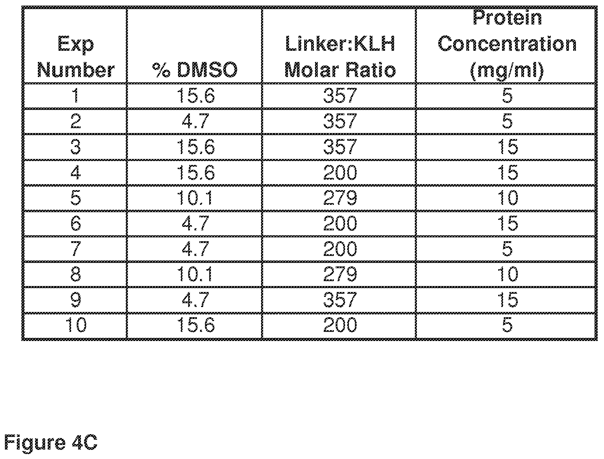

Experiment 122123-115 examined percent DMSO, Sulfo-SMCC:KLH molar ratio, and protein concentration using the full factorial Design of Experiments (DOE) shown in Figure 4C. Sulfo-SMCC linker was dissolved in DMSO at 121.8 mg/mL prior to addition. Peptide at a molar ratio of 4:1 peptide:linker was immediately added to the reaction at the end of the activation.

### 5.2.5 Experiment No. 122123-190

KLH was activated with sulfo-SMCC at linker:protein molar ratios varying between 25:1 and 350:1 for various activation times according to Figure 4A. Sulfo-SMCC linker was dissolved in DMSO at 100 mg/mL prior to addition. Peptide at a molar ratio of 4:1 peptide:linker was immediately added to the reaction at the end of the activation.

### Example 6: Results of Reaction Parameter Process Development

The focus of the reaction parameter process development was the optimization of reaction parameters using a scaled down system. The reaction volumes were less than 2 mL and clearance of peptide was achieved through the use of Amicon centrifugal filters. Clearance of peptide was needed in order to have an accurate analysis of epitope density. The trends generated using the scaled-down reaction can be used to understand the effects and interactions of the process parameters but the clearance of peptide is not equivalent to a continuous TFF operation. The analytical attributes significant for reaction optimization are epitope density and size distribution profile. Purity of conjugates by AEX and/or RP-HPLC was used to ensure peptide was removed prior to analysis by amino acid composition but is not included herein. The targets for the optimized process are listed below in Table 4.

| **Table 4 Quality Targets for CDX-110 Drug Substance** | | | |
|---|---|---|---|
| **Parameter** | **Method** | **Target** | |
| Epitope Density | Amino Acid Composition | 30-64 Peptides/KLH | |
| Size Distribution Profile | SEC HPLC | Peak 1 | < 2% |
| | | Peak 2 | 8-17% |
| | | Peak 3 | 50-60% |
| | | Peak 4 | 20-30% |
| | | Peak 5 | 1-5% |
| Purity | AEX | Peptide dimer ≤ 5% | |
| | RP-HPLC | Linker or linker-related impurities ≤ 1% | |
| | | Total peptide-linker impurities ≤ 10% | |

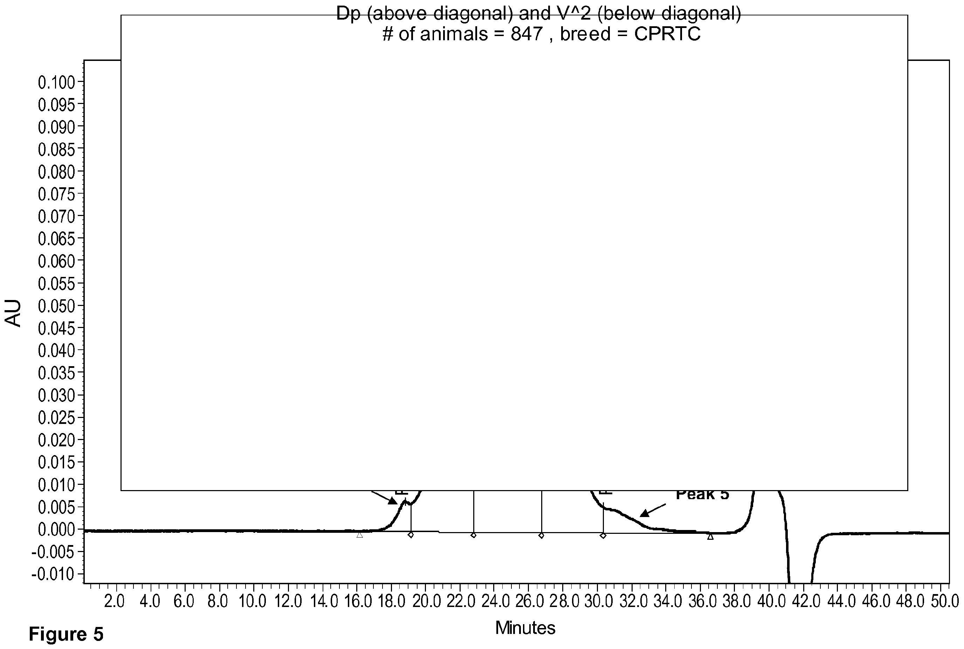

Figure 5 contains a typical SEC HPLC chromatogram for CDX-110 showing the approximate retention times of the five peaks. Peaks 1 and 2 are believed to be high molecular weight species, peak 3 is believed to be dimer, peak 4 is believed to be monomer, and peak 5 is believed to be low molecular weight species.

### 6.1 One-Pot Reaction

The scaled down reaction model included testing multiple parameters at once and used reaction volumes less than 2 mL. Although some experiments removed linker from the activation by slow diafiltration with Amicon filters, most experiments used a one-pot reaction model. The one-pot system quenches the activation reaction and any linker impurities with an increased molar ratio of peptide (4:1 peptide:linker molar ratio) instead of clearing the linker from the reaction. Techniques to remove linker at this small scale were either not effective at removing impurities or could change the SEC distribution during processing.

The one-pot reaction does not confound operation time with the variables tested and can show a true trend for the activation parameters. However the SEC distribution results from the one-pot reactions are generally not always in the target range for the drug substance process because of the absence of a TFF operation time. The SEC distribution and epitope density continue to change with time as shown for activated KLH after clearance of linker and for activation time (see 6.2). The effect of time on SEC distribution and epitope density was considered when selecting parameters for the optimized process.

### 6.2 Molar Ratio of Sulfo-SMCC:KLH and Activation Time

### Epitope Density

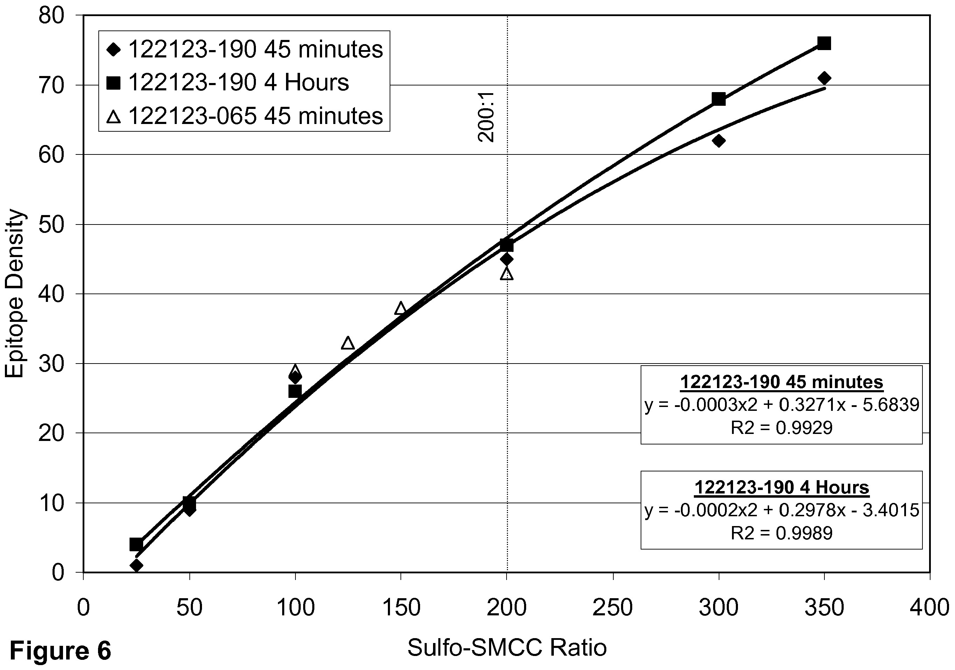

The increase in epitope density associated with an increase in linker ratio for experiment 122123-190 was fit to the second order polynomial shown in Figure 6. A second order curve has been used in preliminary kinetic modeling evaluations. This fitted curve can be used to predict epitope density at a given linker:protein ratio using the same activation conditions. The results from experiment 122123-065, also shown in Figure 6, are comparable to experiment 122123-190.

A linker:protein ratio of 200:1, the ratio chosen for the optimized process, results in an epitope density of -45 peptides/KLH using a one-pot reaction scheme. The 200:1 ratio provides a mid-range epitope density and the entire target range of 30-64 peptides/KLH can be achieved by linker molar ratios of 200 ± 125 with a 45 minute activation time. The epitope density expected for the manufacturing process will be less than this to account for the processing time to remove the linker by diafiltration.

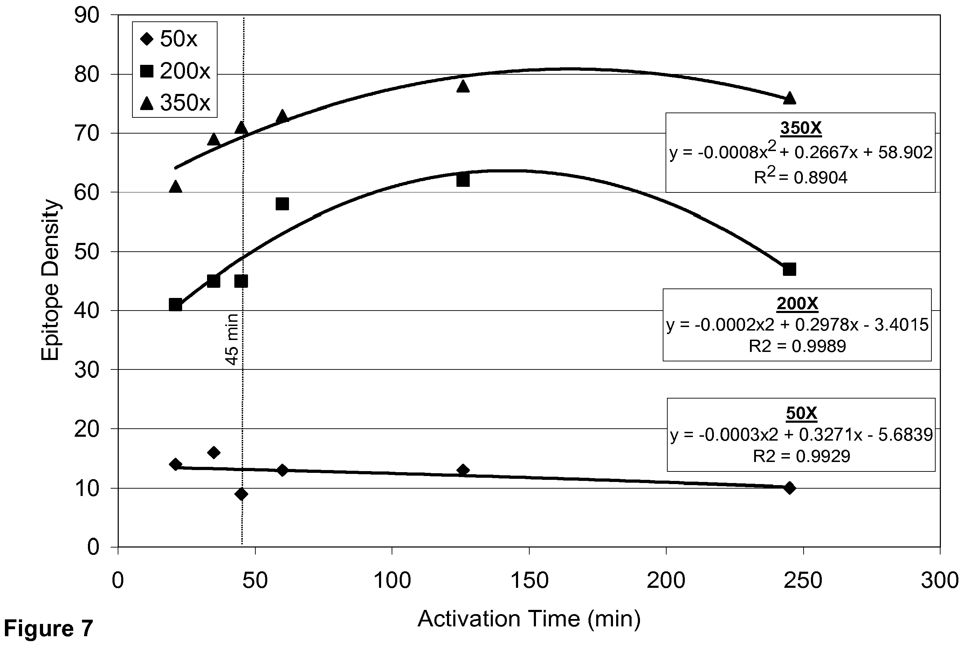

An activation time of -150 minutes resulted in a maximum epitope density of -80 peptides/KLH for a reaction using a molar ratio of 350:1 Sulfo-SMCC:KLH and a maximum epitope density of -60 peptides/KLH for a reaction using 200:1 Sulfo-SMCC:KLH (Figure 7). After 150 minutes, the epitope density achieved at each linker ratio begins to decline. The decline in epitope density with time could be related to hydrolysis of the active maleimide or linker degradation.

Similar epitope density curves result from both 45 minute and 4 hour activation times (see Figure 6). The similarity of the curves is related to the activation time results shown in Figure 7 where 45 minute and 250 minute (4 hour) have similar epitope densities. Higher epitope densities may be achieved at similar conditions if the same linker ratio curve was generated at the maximum (150 min).

### SEC Distribution

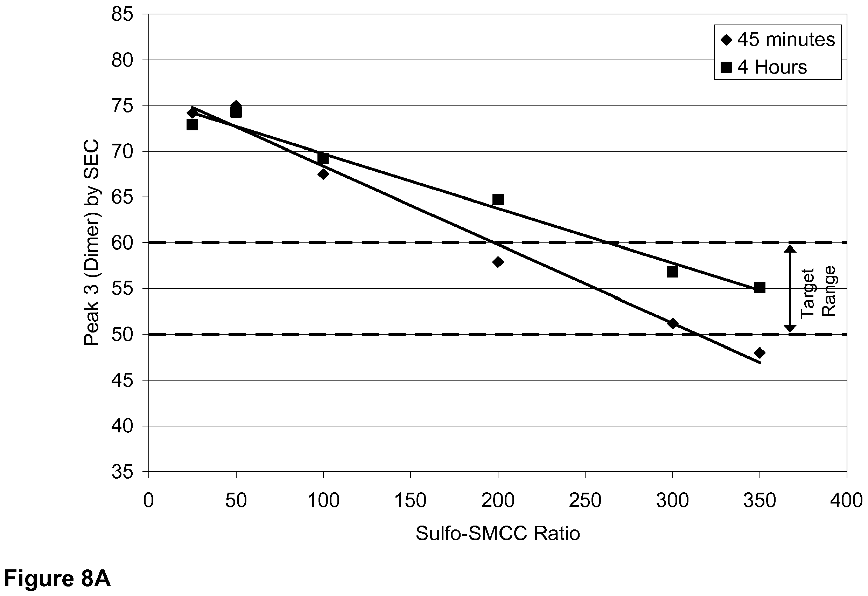

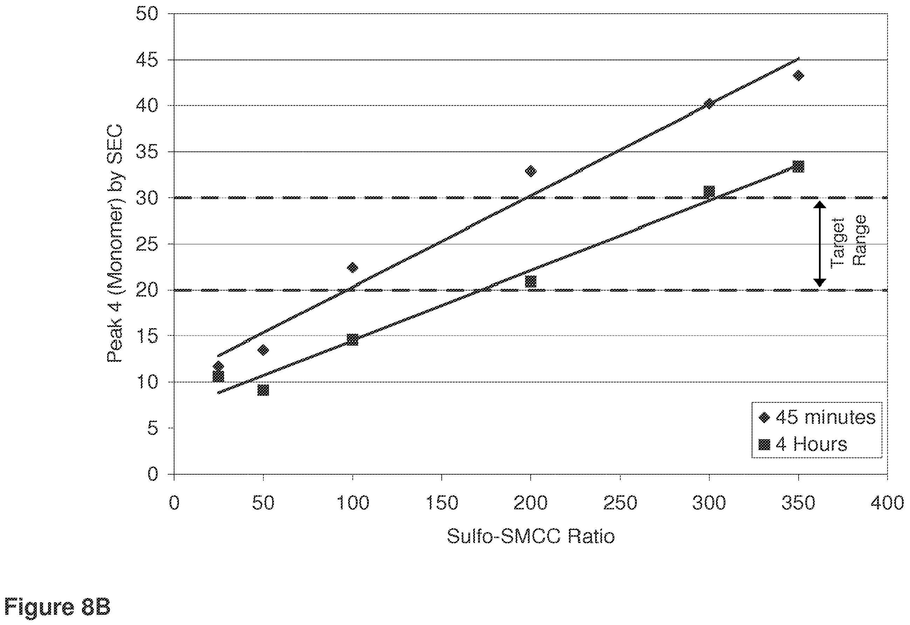

Although more data points are needed to determine the curve shape, the SEC results from experiment 122123-190 shown in Figures 8A and 8B were fit to a linear curve to show a general trend. Monomer species (peak 4) were shown to increase while high molecular weight (peak 2) and dimer species (peak 3) were shown to decrease with an increasing linker:protein ratio. At the optimized process conditions, a 200:1 linker:protein ratio and a 45 minute activation time, the SEC distribution does not meet the drug substance process targets because the reactions were performed using a one-pot reaction with no additional hold or processing time.

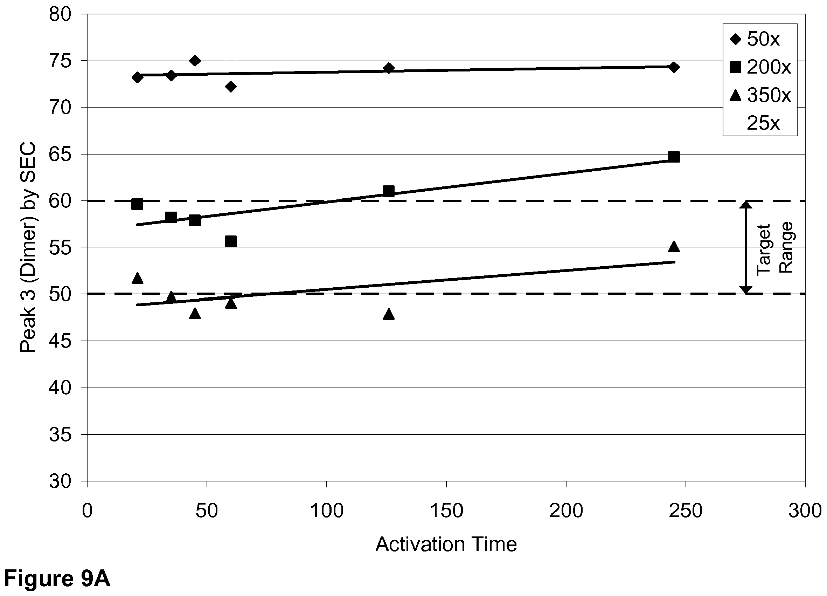

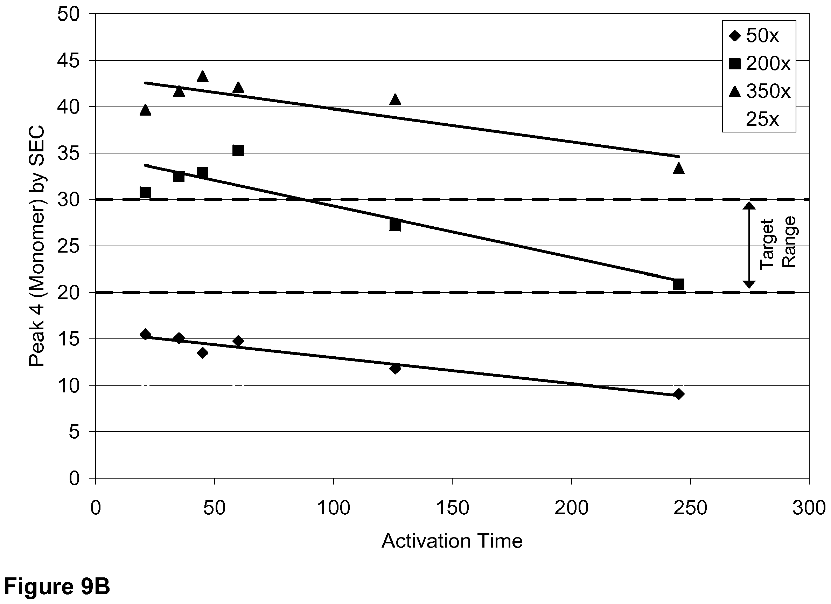

Higher molecular weight species (peaks 1, 2 and 3) form over time during the activation reactions. This observation is consistent regardless of linker:protein ratio. The SEC results for activation time (Figures 9A and 9B) were also fit to a linear curve to show a general trend although the actual relationships may not be linear.

Results for peaks 1 and 5 are not presented but generally follow similar trends of more higher molecular weight species with decreased linker:protein ratio and increased activation time; however, the percent of each species or the change in value is very small.

A straightforward relationship between epitope density and SEC heterogeneity has not been found. Each are affected by the same parameters but not necessarily in the same way. The parameters for the optimized process were chosen by considering how all the parameters interact. When the operation time for the TFF process is combined with the changes in the SEC distribution over time, all of the SEC peaks and epitope density will be within the target range.

### 6.3 Dissolution of Linker

Sulfo-SMCC linker in a previous process was suspended in phosphate buffer prior to starting the activation. The linker is not soluble in aqueous buffer at this concentration resulting in a linker slurry. The Sulfo-SMCC is fully soluble in DMSO at concentrations greater than 100 mg/mL and will offer several advantages that will make the overall process more robust. These advantages include: 1) Reduced linker hydrolysis - the linker will begin to degrade and hydrolyze as soon as it is in contact with aqueous solution but should remain stable in anhydrous DMSO, which will provide greater flexibility for manufacturing; and 2) Better control of linker:protein ratio - the transfer and dissolution of a slurry is more difficult to control than an aqueous solution. Small changes in the linker ratio could effect the epitope density and SEC distribution significantly.

### Linker Slurry vs Dissolution in DMSO

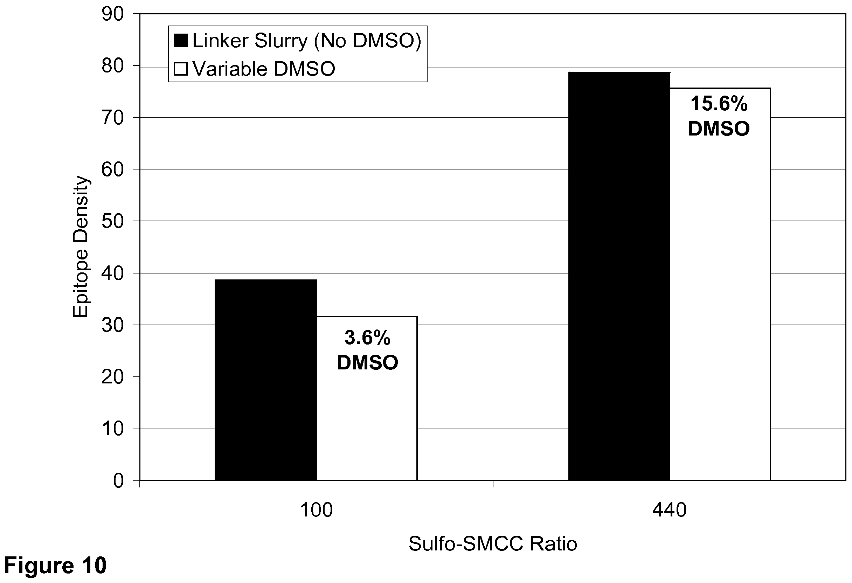

Epitope density was similar for linker:protein ratios of 100:1 and 440:1 when comparing linker added as a slurry (no DMSO) and linker dissolved in DMSO (Figure 10). This observation is consistent with the results shown in Figure 6 comparing experiments 122123-065 (no DMSO) and 122123-190 (DMSO < 3.4%) which also had similar epitope densities for reactions with and without DMSO.

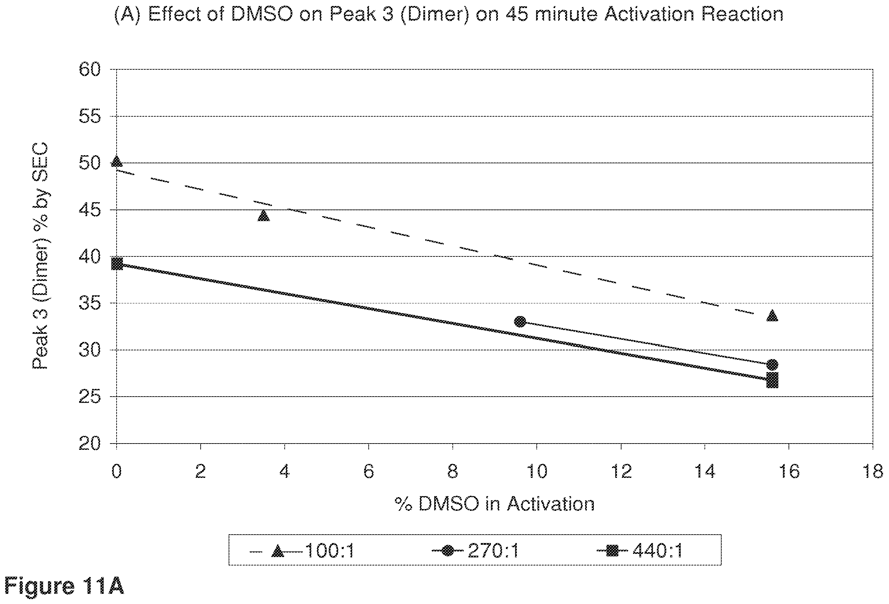

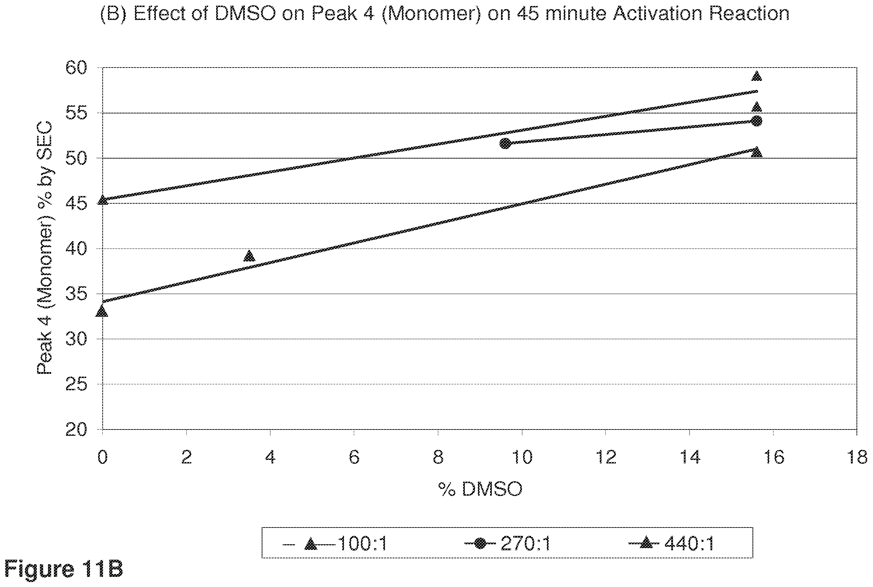

DMSO added to the activation reaction was shown to reduce the amount of higher molecular weight species (Figure 11A and 11B). Dimer (peak 3) and monomer (peak 4) changed by 10-15% as DMSO increased from 0% to ∼15.6%. Peaks 1, 2, and 5 generally follow the same trends although the percent of each species or the change in value is very small.

### 6.4 DOE to Determine Effect of DMSO, Protein Concentration, and Sulfo-SMCC Ratio

The initial experiments to look at linker slurry showed almost no change in epitope density and a significant effect of DMSO on the SEC distribution. A follow-up experiment, 122123-115, was designed to further examine the effect of DMSO using a full factorial design of experiments (DOE). The experiment was designed to test DMSO (4.7 - 15.6%), linker:protein molar ratio (200:1 - 357:1), and protein concentration (5-15 mg/mL).

### Epitope Density

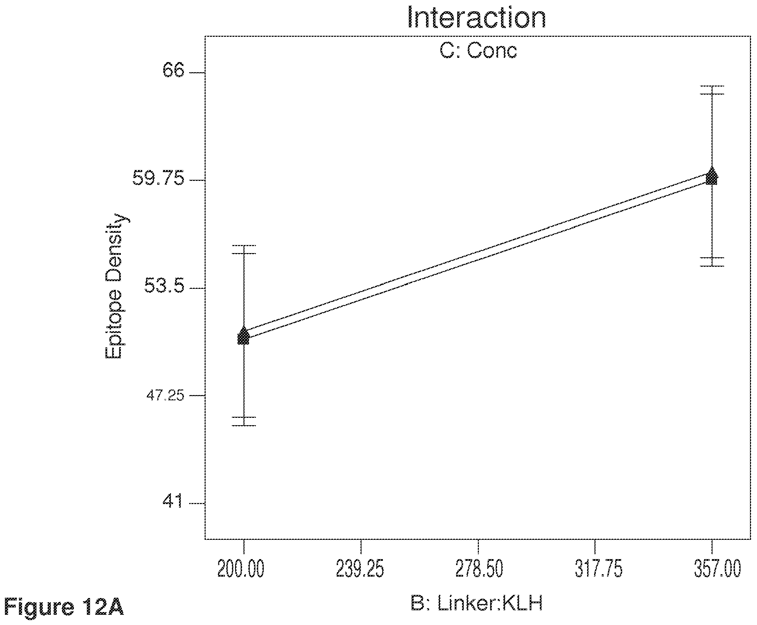

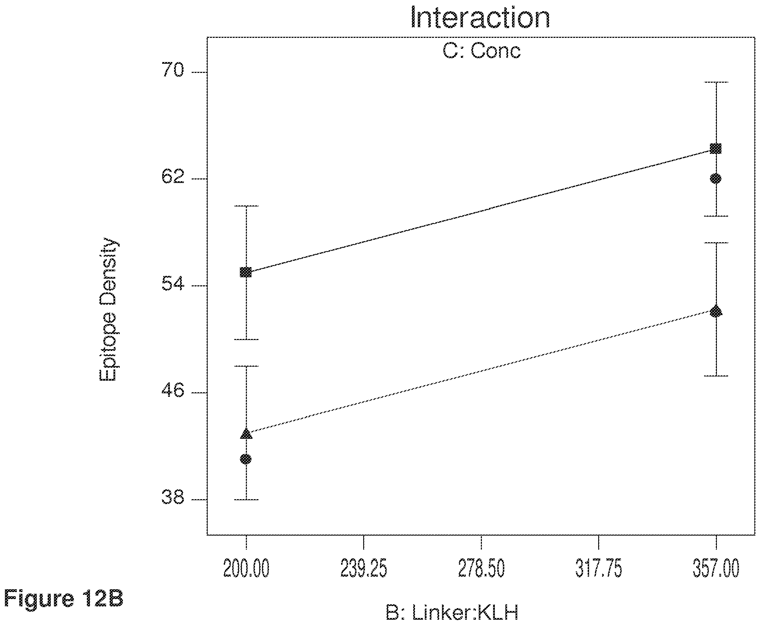

An analysis of variance (ANOVA) of the epitope density results with a 95% confidence interval indicate that linker:protein ratio is a statistically significant factor. Epitope density increases when more Sulfo-SMCC is present, which is consistent with the results discussed in section 6.2. Although not a statistical interaction, the final epitope density obtained for a given Sulfo-SMCC ratio is impacted by DMSO and protein concentration. At low DMSO concentrations (4.7%), there is little effect on epitope density (Figure 12A), while at high DMSO concentrations (15.6%) epitope density is offset by approximately 10 peptides/KLH depending on the protein concentration (Figure 12B).

If the confidence interval is reduced to 90%, protein concentration and the interaction between protein concentration and DMSO also become significant parameters effecting epitope density. At 4.7% DMSO, little change in epitope density is observed. At protein concentrations of 5 to 7.5 mg/mL, epitope density starts to increase as the percent of DMSO in the reaction is increased. At protein concentrations of 12 to 15 mg/mL epitope density starts to decrease as the percent of DMSO in the reaction is increased.

### SEC Distribution

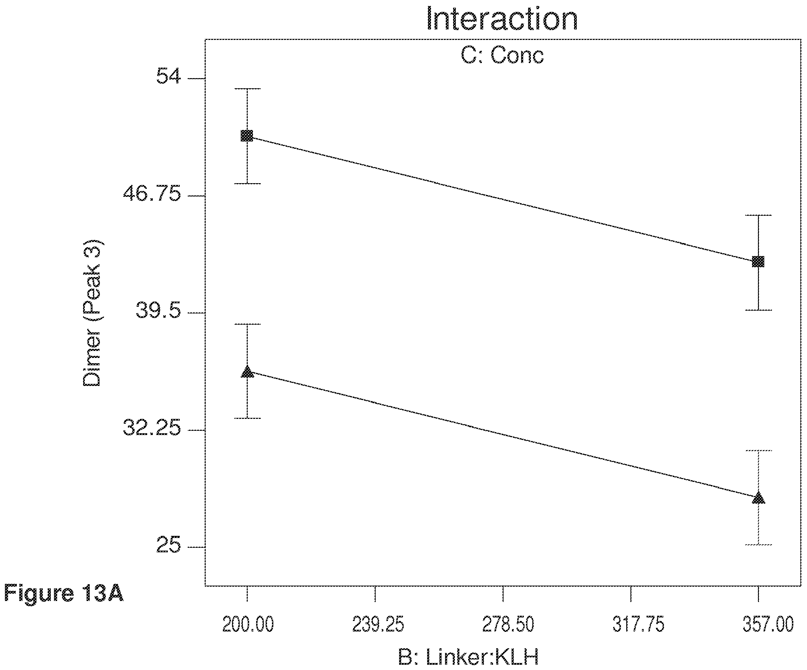

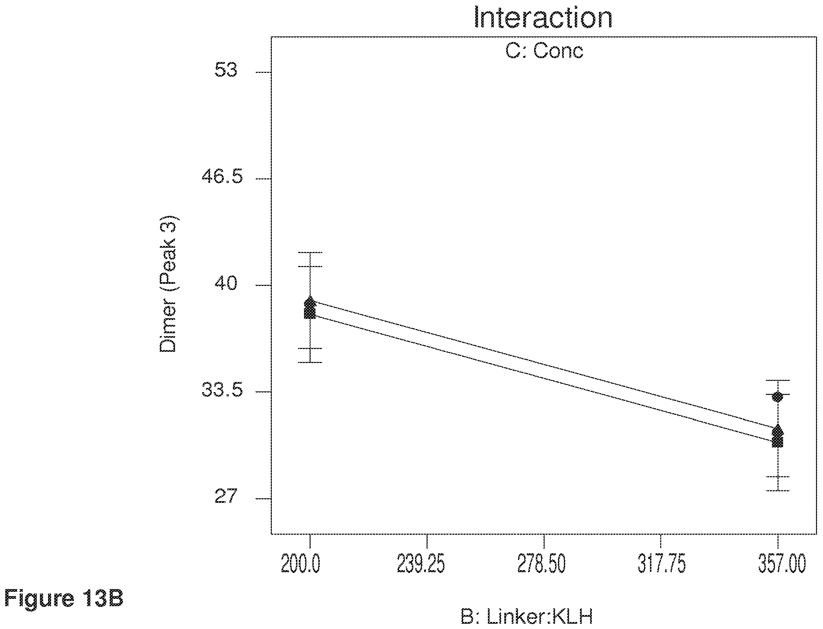

ANOVA of the dimer (peak 3) results with a 95% confidence interval indicate that linker:protein ratio, percent DMSO, protein concentration, and the interaction between percent DMSO and protein concentration are all statistically significant factors. Dimer (peak 3) decreases when more Sulfo-SMCC is present, which is consistent with the results discussed in section 6.2. Although not a statistical interaction, the percent dimer obtained for a given Sulfo-SMCC ratio is impacted by DMSO and protein concentration. At high DMSO concentrations (15.6%), there is little effect on epitope density (Figure 13B), while at low DMSO concentrations (4.7%) epitope density is offset by as much as 15% depending on the protein concentration (Figure 13A).

The interaction effect between protein concentration and DMSO on dimer (peak 3) can be summarized as follows. At 15.6% DMSO, little change in epitope density is observed. At protein concentrations of 5 to 11.5 mg/mL, the dimer (peak 3) starts to increase as the percent of DMSO in the reaction is lowered.

The protein concentration for the optimized process (10 mg/mL) did not change from the original process. At this concentration, the dimer (peak 3) changes slightly as the percent DMSO is increased from 4.7% to 15.6%. ANOVA of the monomer (peak 4) results with a 95% confidence interval indicate that Sulfo-SMCC ratio and percent DMSO are both statistically significant factors. Monomer increases when more Sulfo-SMCC and more DMSO are present, which is consistent with the results discussed in section 6.2.

### 6.5 Optimzed Process

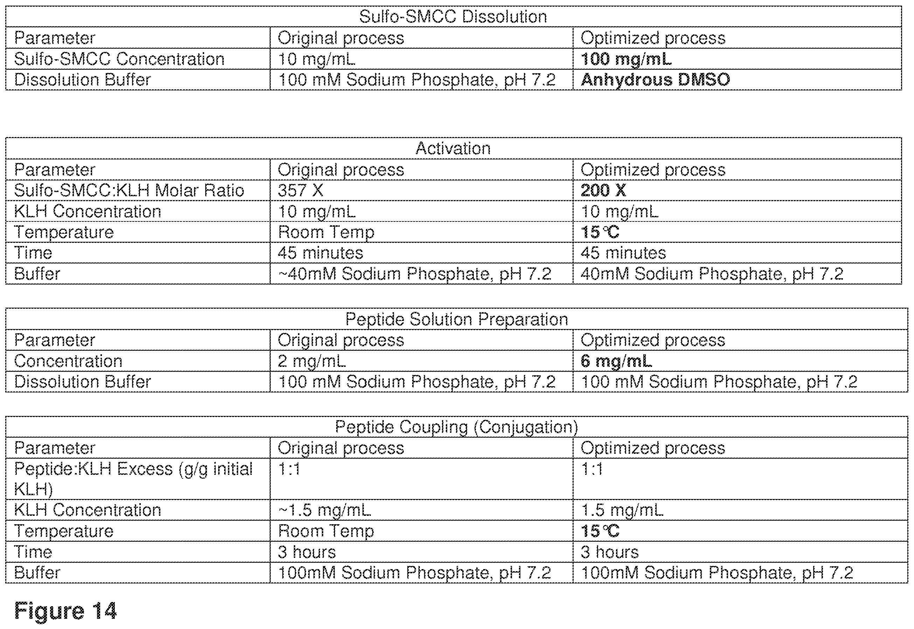

Figure 14 summarizes the reaction parameters used for both the original and optimized processes. The changes to the reaction conditions (highlighted in bold) are the following: 1) Dissolution of Sulfo-SMCC in anhydrous DMSO - reduced linker hydrolysis and provided better control of Sulfo-SMCC linker ratio; 2) Sulfo-SMCC molar ratio reduced to 200x - this change was to compensate for the dissolution of linker in DMSO; 3) Temperature - reduced to 15°C; 4) Peptide stock solution concentration - Increased to account for concentration of KLH after TFF operation. Since the peptide is soluble at >20 mg/ml and dimerization is slow, this should have little effect on the reaction.

### Example 7: Particulate Counts

The U.S. Pharmacopeia <788> (USP <788> Particulate Matter in Injections) sets forth certain criteria and protocols regarding the measurement of particulate counts per vial. As indicated in USP <788>, for the determination of particulate matter, two procedures can be used: the Light Obscuration Particle Count Test; and the Microscopic Particle Count Test. The criteria set forth in USP<788> are as follows: particle size ≥10um: not more than 6000/container; and particle size ≥ 25µm: not more than 600/container. For CDX-110, a study was performed comparing two formulations at time=0 and at the end of 26 weeks at 5°C to determine the effect of formulation components on particulate count using standard protocols as set forth in USP <788>. The first formulation (Formulation A) contained 1.0 mg/mL of the KLH-EGFRvIII conjugate CDX-110 1.0 mg/mL, 10 mM phosphate buffer (Na₂HPO₄KH₂PO₄), 137 mM NaCl, and 2.7 mM KCI. The second formulation (Formulation B) contained 1.0 mg/mL of the KLH-EGFRvIII conjugate CDX-110, 10 mM potassium phosphate buffer, 90mg/mL trehalose, and 0.2 mg/mL polysorbate 80.

Table 5 and Table 6 list the particulate count for Formulation A at time = 0 and after 26 weeks at 5°C, respectively. Table 7 and Table 8 list the particulate count for Formulation B at time = 0 and after 26 weeks at 5°C, respectively. The data demonstrate no significant increase in particulate content at the end of 26 weeks for Formulation B. A significant increase in sub-visible particles above 10 and 25 µm, however, was observed in 26 weeks for Formulation A when compared to Formulation B.

**Table 5: Formulation A particulate count at time = 0**

| **Particle Size (µm)** | **Avg cumulatitve particles/mL** | **Avg cumulative particles/container** |
|---|---|---|
| 1.5 | 926 | 555.6 |
| 2 | 366 | 219.6 |
| 5 | 56 | 33.6 |
| 8 | 13 | 7.8 |
| 10 | 6 | 3.6 |
| 15 | 1 | 0.6 |
| 20 | 0 | 0 |
| 25 | 0 | 0 |

**Table 6: Formulation A particulate count at time = 26 weeks, 5°C**

| **Particle Size (µm)** | **Avg cumulatitve particles/mL** | **Avg cumulative particles/container** |
|---|---|---|
| 1.5 | 2401 | 1441 |
| 2 | 821 | 493 |
| 5 | 206 | 123 |
| 8 | 79 | 48 |
| 10 | 54 | 32 |
| 15 | 27 | 16 |
| 20 | 11 | 7 |
| 25 | 5 | 3 |

**Table 7: Formulation A particulate count at time = 0**

| **Particle Size (µm)** | **Avg cumulatitve particles/mL** | **Avg cumulative particles/container** |
|---|---|---|
| 1.5 | 586 | 369 |
| 2 | 219 | 138 |
| 5 | 67 | 42 |
| 8 | 24 | 15 |
| 10 | 17 | 11 |
| 15 | 8 | 5 |
| 20 | 3 | 2 |
| 25 | 2 | 1 |

**Table 8: Formulation B particulate count at time = 26 weeks, 5°C**

| **Particle Size (µm)** | **Avg cumulatitve particles/mL** | **Avg cumulative particles/container** |
|---|---|---|
| 1.5 | 814 | 513 |
| 2 | 357 | 225 |
| 5 | 64 | 40 |
| 8 | 20 | 13 |
| 10 | 12 | 8 |
| 15 | 5 | 3 |
| 20 | 2 | 1 |
| 25 | 2 | 1 |

### SUMMARY OF SEQUENCE LISTING

| **SEQ ID NO:** | **DESCRIPTION** | **SEQUENCE** |
|---|---|---|
| 1 | EGFRvIII peptide | LEEKKGNYWTDH |
| 2 | EGFRvIII peptide with C-terminal Cys residue for linking | LEEKKGNYWTDHC |
| 3 | EGFRvIII peptide with N-terminal Cys residue for linking | CLEEKKGNYVVTDH |

### ASPECTS OF THE INVENTION

1. A composition comprising a KLH-peptide conjugate, a buffer, a saccharide, and a surfactant, wherein the peptide conjugated to KLH in the KLH-peptide conjugate comprises an EGFRvIII amino acid sequence.
2. The composition according to 1, wherein the buffer comprises a phosphate buffer.
3. The composition according to 2, wherein the buffer comprises a potassium phosphate buffer.
4. The composition according to any one of 1 to 3, wherein the buffer is present in a concentration ranging from 5 mM to 30 mM.
5. The composition according to any one of 1 to 3, wherein the buffer is present in a concentration such that after 12 weeks at 40°C, the concentration of the KLH-peptide conjugate in said composition, as measured in mg/mL, has changed by less than 15% when compared to the original concentration.
6. The composition according to 1, wherein the saccharide is a disaccharide.
7. The composition according to 6, wherein the disaccharide is trehalose, and is present in a concentration ranging from 45 to 150 mg/mL or is present in an amount ranging from 80 to 110 mg per mg of KLH-peptide conjugate.
8. The composition according to 6, wherein the disaccharide is trehalose and is present in a concentration such that after 12 weeks at 40°C, the concentration of the KLH-peptide conjugate in said composition, as measured in mg/mL, has changed by less than 15% when compared to the original concentration.
9. The composition according to 1, wherein the surfactant is polysorbate, and is present in a concentration ranging from 0.01 to 0.3 mg/mL or is present in an amount ranging from 0.01 to 0.3 mg per mg of KLH-peptide conjugate.
10. The composition according to 1, wherein the surfactant is polysorbate and is present in a concentration such that after 12 weeks at 40°C, the concentration of the KLH-peptide conjugate in said composition, as measured in mg/mL, has changed by less than 15% when compared to the original concentration.
11. The composition according to any of the preceding aspects, wherein the peptide conjugated to KLH in the KLH-peptide conjugate consists of SEQ ID NO:1.
12. The composition according to any of the preceding aspects, wherein the peptide conjugated to KLH in the KLH-peptide conjugate consists of SEQ ID NO:2.
13. The composition according to any of the preceding aspects, wherein the peptide is conjugated to KLH with a sulfo-SMCC linker.
14. The composition according to any of the preceding aspects, wherein the epitope density ranges from 20 to 80.
15. The composition according to any of the preceding aspects, wherein the amount of KLH-peptide conjugate present in dimer form ranges from 45% to 65% by mass of the total mass of the composition, as determined by size exclusion chromatography.
16. The composition according to any of the preceding aspects, wherein the amount of KLH-peptide conjugate present in monomer form ranges from 15% to 40% by mass of the total mass of the composition, as determined by size exclusion chromatography.
17. The composition according to any of the preceding aspects, wherein the amount of KLH-peptide conjugate present in monomer form ranges from 18% to 35% by mass of the total mass of the composition, and the amount of KLH-peptide conjugate present in dimer form ranges from 50% to 65% by mass of the total mass of the composition, as determined by size exclusion chromatography.
18. The composition according to any of the preceding aspects, wherein said composition is an aqueous pharmaceutical composition, and the pH of said composition ranges from 6 to 8.
19. The composition according to any of the preceding aspects, wherein said composition is an aqueous pharmaceutical composition, and the pH of said composition is an amount such that after 12 weeks at 40°C, the concentration of the KLH-peptide conjugate in said composition, as measured in mg/mL, has changed by less than 15% when compared to the original concentration.
20. A liquid composition comprising a KLH-EGFRvIII peptide conjugate, potassium phosphate buffer, trehalose, and polysorbate 80, wherein: the peptide conjugated to KLH comprises SEQ ID NO:1; the KLH-EGFRvIII peptide conjugate has an epitope density ranging from 30 to 65; the buffer is present in a concentration ranging from 9 mM to 11 mM; the pH of the composition ranges from 7.3 to 7.5; the trehalose is present in a concentration ranging from 85 mg/mL to 95 mg/mL; the polysorbate 80 is present in a concentration ranging from 0.1 mg/mL to 0.3 mg/mL; and further wherein the amount of KLH-EGFRvIII peptide conjugate present in monomer form ranges from 18% to 35% by mass of the total mass of the composition, and the amount of KLH-EGFRvIII peptide conjugate present in dimer form ranges from 50% to 65% by mass of the total mass of the composition, as determined by size exclusion chromatography.
21. The composition of 17, wherein the liquid composition is prepared by reconstituting a lyophilized composition with water.
22. The composition of 17 or 18, wherein the buffer is present in a concentration of 10 mM, the pH of the composition is 7.4, the trehalose is present in a concentration of 90 mg/mL, and the polysorbate 80 is present in a concentration of 0.2 mg/mL.
23. A method for preparing a KLH-EGFRvIII conjugate comprising: a) combining KLH with a linker and allowing the KLH and linker to interact for a time ranging from 30 to 60 minutes; and b) adding a peptide comprising SEQ ID NO: 1 to the activated KLH product resulting from step a) to provide the KLH-EGFRvIII conjugate.
24. The method of 20, wherein the linker is combined with KLH in a linker:KLH molar ratio that ranges from 75:1 to 325:1.
25. A method for preparing a KLH-EGFRvIII conjugate comprising: a) combining KLH with a linker and allowing the KLH and linker to interact; and b) adding a peptide comprising SEQ ID NO: 1 to the activated KLH product resulting from step a) to provide the KLH-EGFRvIII conjugate, wherein the linker is a sulfo-SMCC linker which is added in a non-aqueous solvent.
26. A method as claimed in 25 wherein the non-aqueous solvent comprises DMSO.
27. A composition comprising a KLH-EGFRvIII peptide conjugate wherein the amount of KLH-EGFRvIII peptide conjugate present in monomer form ranges from 18% to 35% by mass of the total mass of the composition, and the amount of KLH-EGFRvIII peptide conjugate present in dimer form ranges from 50% to 65% by mass of the total mass of the composition, as determined by size exclusion chromatography.
28. A composition comprising a KLH-EGFRvIII peptide conjugate wherein said composition is an aqueous pharmaceutical composition which has been stabilised such that after 12 weeks at 40°C, the concentration of the KLH-EGFRvIII peptide conjugate in said composition, as measured in mg/mL, has changes by less than 15% when compared to the original concentration.
29. A composition comprising a KLH-EGFRvIII peptide conjugate wherein the mean epitope density of said conjugate is between about 20 and 80.
30. A composition comprising a KLH-EGFRvIII peptide conjugate wherein said composition has the following properties:
i) epitope density as measured by amino acid composition of 30-70 peptides/KLH;
ii) size distribution profile as measured by SEC HPLC as follows:

| | |
|---|---|
| Peak 1 | < 2% |
| Peak 2 | 8-17% |
| Peak 3 | 50-60% |
| Peak 4 | 20-30% |
| Peak 5 | 1-5% |

iii) purity as measured by AEX RP-HPLC as follows:
Peptide dimer ≤ 5%
Linker or linker-related impurities ≤ 1%
Total peptide-linker impurities ≤ 10%.
31. The composition according to any one of 27 to 30, which comprises a buffer.
32. The composition according to 31, wherein the buffer comprises a phosphate buffer.
33. The composition according to 32, wherein the buffer comprises a potassium phosphate buffer.
34. The composition according to any one of 27 to 33 which comprises a croprotectant or lyoprotectant.
35. The composition according to any one of 27 to 33 which comprises a saccharide.
36. The composition according to 35 wherein the saccharide is a disaccharide.
37. The composition according to 36 wherein the diaccharide is trehalose.
38. The composition according to any one of 27 to 37 which comprises a surfactant.
39. The composition according to 38 wherein the surfactant is a polysorbate, poloxamer or triton surfactant.
40. The composition according to 39 wherein the surfactant is polysorbate 80.
41. The composition according to any one of 27 to 40, wherein after 26 weeks at 5°C, the number of particulates greater than or equal to 25 µm, as measured by USP <788>, increases by less than 1000%.

## Claims

1. A method for preparing a KLH-EGFRvIII conjugate comprising:
a) combining KLH with a linker and allowing the KLH and linker to interact, wherein the linker is combined with KLH in a linker:KLH molar ratio that ranges from 75:1 to 325:1 and allowed to interact for a time ranging from 30 to 60 minutes, and/or the linker is a sulfo-SMCC linker which is added in a non-aqueous solvent; and
b) adding a peptide comprising SEQ ID NO: 1 to the activated KLH product resulting from step a) to provide the KLH-EGFRvIII conjugate.

2. The method of claim 1, wherein the linker is combined with KLH in a linker:KLH molar ratio that ranges from 75:1 to 325:1.

3. The method of claim 1 or 2, wherein the linker:KLH molar ratio ranges from 150:1 to 250:1.

4. The method of any one of claims 1 to 3, wherein the linker:KLH molar ratio is 200:1.

5. The method of any one of the preceding claims, wherein the conjugate has an epitope density ranging from 20 to 80.

6. The method of any one of the preceding claims, wherein the linker is a sulfo-SMCC linker which is added in a non-aqueous solvent.

7. The method of any one of the preceding claims, wherein the non-aqueous solvent comprises DMSO.

8. The method of any one of the preceding claims, wherein the KLH and linker are allowed to interact for a time ranging from 30 to 60 minutes.

9. The method of any one of the preceding claims, further comprising formulating the conjugate as a pharmaceutical composition for parenteral administration.

10. The method of claim 9, wherein the composition is a lyophilized composition.

11. The method of claim 9, wherein the composition is a liquid composition.

12. A KLH-EGFRvIII conjugate produced by the method of any one of claims 1 to 9.
